(19) Europäisches Patentamt / European Patent Office / Office européen des brevets

(11) **EP 3 607 065 B1**

(12) **EUROPEAN PATENT SPECIFICATION**

(45) Date of publication and mention
of the grant of the patent:
**15.03.2023  Bulletin 2023/11**

(21) Application number: **18781272.2**

(22) Date of filing: **04.02.2018**

(51) International Patent Classification (IPC):
**C12N 15/10** (2006.01)     **C12N 15/66** (2006.01)

(52) Cooperative Patent Classification (CPC):
(C-Sets available)
**C12N 15/1093; C12N 15/1065; C12N 15/1096**

(Cont.)

(86) International application number:
**PCT/US2018/016778**

(87) International publication number:
**WO 2018/186930 (11.10.2018 Gazette 2018/41)**

(54) **METHOD AND KIT FOR CONSTRUCTING NUCLEIC ACID LIBRARY**

VERFAHREN UND KIT ZUM AUFBAU EINER NUKLEINSÄUREBIBLIOTHEK

PROCÉDÉ ET KIT DE CONSTRUCTION D'UNE BIBLIOTHÈQUE D'ACIDES NUCLÉIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **06.04.2017   US 201762482189 P**

(43) Date of publication of application:
**12.02.2020   Bulletin 2020/07**

(73) Proprietor: **Wang, Qing
Owings Mills, Maryland 21117 (US)**

(72) Inventors:
• **WANG, Qing
Owings Mills, Maryland 21117 (US)**
• **ZHANG, Ming
Owings Mills, Maryland 21117 (US)**

(74) Representative: **K&L Gates LLP
Markgrafenstraße 42
10117 Berlin (DE)**

(56) References cited:
**WO-A1-2016/195382      US-A1- 2004 185 484
US-A1- 2016 230 232**

• **CHANDRA SEKHAR REDDY CHILAMAKURI ET AL**: "Performance comparison of four exome capture systems for deep sequencing", BMC GENOMICS, BIOMED CENTRAL, vol. 15, no. 1, 9 June 2014 (2014-06-09), page 449, XP021189982, ISSN: 1471-2164, DOI: 10.1186/1471-2164-15-449
• **M. W. SCHMITT ET AL**: "Detection of ultra-rare mutations by next-generation sequencing /Supporting Information/", PROCEEDINGS OF THE NATIONAL ACADEMY OF SCIENCES, vol. 109, no. 36, 27 March 2013 (2013-03-27), pages 1-3, XP055646617, US ISSN: 0027-8424, DOI: 10.1073/pnas.1208715109
• **I. KINDE ET AL**: "Detection and quantification of rare mutations with massively parallel sequencing /Supporting Information/", PROCEEDINGS OF THE NATIONAL ACADEMY OF SCIENCES, vol. 108, no. 23, 17 May 2011 (2011-05-17), pages 9530-9535, XP055647660, US ISSN: 0027-8424, DOI: 10.1073/pnas.1105422108
• **A. NARAYAN ET AL**: "Ultrasensitive Measurement of Hotspot Mutations in Tumor DNA in Blood Using Error-Suppressed Multiplexed Deep Sequencing", CANCER RESEARCH, vol. 72, no. 14, 15 July 2012 (2012-07-15), pages 3492-3498, XP055407873, US ISSN: 0008-5472, DOI: 10.1158/0008-5472.CAN-11-4037

- **ENRIQUE RAMOS ET AL: "Population-based rare variant detection via pooled exome or custom hybridization capture with or without individual indexing", BMC GENOMICS, BIOMED CENTRAL, vol. 13, no. 1, 6 December 2012 (2012-12-06), page 683, XP021134772, ISSN: 1471-2164, DOI: 10.1186/1471-2164-13-683**
- **GANSAUGE et al.: "Single-stranded Dna library preparation for the sequencing of ancient or damaged DNA", Nature Protocols, vol. 8, no. 4, 14 March 2013 (2013-03-14), pages 737-748, XP055214715,**
- **KYUNG NAM et al.: "Oligo(dT) primer generates a high frequency of truncated cDNAs through internal poly(A) priming during reverse transcription", PNAS, vol. 99, no. 9, 30 April 2002 (2002-04-30) , pages 6152-6156, XP055119934,**
- **WANG et al.: "Targeted sequencing of both DNA strands barcoded and captured individually by RNA probes to indentify genome-wide ultra-rare mutations", Scientific Reports, vol. 7, no. 1, 13 June 2017 (2017-06-13), pages 1-14, XP055551336,**

Remarks:
   The complete document including Reference Table(s) and the Sequence Listing(s) can be downloaded from the EPO website

(52)   Cooperative Patent Classification (CPC): (Cont.)

   C-Sets
   **C12N 15/1065, C12Q 2525/191, C12Q 2531/113, C12Q 2535/122, C12Q 2563/179**

**Description**

TECHNICAL FIELD OF THE INVENTION

This present disclosure relates generally to the area of genetic analysis, and more specifically to a method and a kit for constructing a nucleic acid library.

BACKGROUND

Recent years have witness a rapid development and wide application of the next-generation sequencing technologies. Next-generation sequencing typically involves the construction of a nucleic acid library from a nucleic acid sample before sequencing.

Current method of constructing a DNA library typically involves chopping nucleic acid sequences to obtain double-stranded DNA fragments before ligation of adaptors at each of the 3' end and the 5' end of the fragments to thereby allow sequencing of each individual double-stranded DNA fragments. In this process, the presence of single-stranded segments in the DNA molecules, due to, for example, the damages to the DNA molecules accumulated during sample preparation, such as formalin-fixed paraffin-embedded (FFPE) samples, or over a long-time storage (e.g. fossil samples), imposes a huge issue, as these damaged DNA segments commonly lead to a great difficulty in DNA sequencing based on current technology for constructing the DNA library.

Nucleic acid samples are sometimes extremely limited, where only nanogram or picogram nucleic acids are available for further analysis. It is a challenging task to construct high quality library from such ultra-low amount of nucleic acid samples. However, this difficulty is frequently encountered in clinical applications of nucleic acid analysis, such as clinical NGS sequencing. In addition, rare mutations or ultra-rare mutations, as those that are commonly associated with cancers, have proven a challenging task for current sequencing platforms. This is primarily because normal tissues are typically collected together with the diseased tissues, which often significantly reduces the prevelance of diasese-related mutations in clinical samples, resulting in a great difficulty in looking for disease-related rare mutations using current sequencing technologies.

As such, genetic analysis of low-quality and/or low-quantity nucleic acid materials is particularly challenging for all current sequencing platforms and technologies.

SUMMARY OF THE INVENTION

In order to address the above-mentioned challenges for analyzing low-quality and/or low-quantity nucleic acid samples using current sequencing technologies, the present disclosure provides a method and a kit for constructing a nucleic acid library.

In a first aspect, the disclosure provides a method for constructing a DNA library from a biological sample containing a plurality of nucleic acid sequences. The method comprises:

preparing a DNA sample from the biological sample, wherein the DNA sample comprises a plurality of single-stranded DNA molecules, each having a dephosphorylated 5' end;

ligating a first strand of a first adaptor to a 3' end of each of the plurality of single-stranded DNA molecules, wherein the first strand of the first adaptor comprises a phosphate group, a barcode sequence and a first primer recognition sequence along a direction from a 5' end thereof to a 3' end thereof; and

synthesizing a complementary strand for each of the plurality of single-stranded DNA molecules ligated to the first strand of the first adaptor to obtain a barcoded double-stranded DNA molecule corresponding thereto,

the first strand of the first adaptor further include an immobilization portion at the 3' end thereof, which is configured to be able to form a stable coupling to a solid support, between the ligating a first strand of a first adaptor to a 3' end of each of the plurality of single-stranded DNA molecules and the synthesizing a complementary strand for each of the plurality of single-stranded DNA molecules ligated to the first strand of the first adaptor to obtain a barcoded double-stranded DNA molecule corresponding thereto, the method further comprises: immobilizing each of the plurality of single-stranded DNA molecules ligated to the first strand of the first adaptor to the solid support via the stable coupling between the immobilization portion and the solid support,

after the synthesizing a complementary strand for each of the plurality of single-stranded DNA molecules ligated to the first strand of the first adaptor to obtain a barcoded double-stranded DNA molecule corresponding thereto, the method further comprise: ligating a second adaptor to a free end of the double-stranded DNA molecule corresponding to the each of the plurality of single-stranded DNA molecules immobilized to the solid support at an immobilized end thereof,

the second adaptor comprises a third strand and a fourth strand, the fourth strand comprises a phosphate group, which is at a 5' end thereof and a second primer recognition sequence, which is configured to provide a priming site for amplification of the double-stranded DNA molecule corresponding to the each of the plurality of single-stranded DNA molecules, the third strand comprises a sequence complimentary to a 5'-end sequence of the fourth strand, and is configured to form a duplex with, and thereby to ensure a stability of, the 5'-end sequence of the fourth strand.

In the step of ligating a first strand of a first adaptor to a 3' end of each of the plurality of single-stranded DNA molecules in the method as described above, the barcode sequence can have any length, but preferably can have a length of 2-16 nt.

According to some embodiments, the plurality of nucleic acid sequences in the biological sample comprise a plurality of DNA sequences, and the preparing a DNA sample from the biological sample comprises: performing dephosphorylation reaction and dissociation reaction to obtain a plurality of single-stranded DNA molecules, each having a dephosphorylated 5' end.

Herein the performing dephosphorylation reaction and dissociation reaction can comprise at least one cycle of: performing a dephosphorylation reaction, and performing a dissociation reaction, or alternatively can comprise at least one cycle of: performing a dissociation reaction, and performing a dephosphorylation reaction.

Prior to the performing dephosphorylation reaction and dissociation reaction, the preparing a DNA sample from the biological sample can further comprise: shearing the plurality of DNA sequences into a plurality of DNA fragments. Herein each of the plurality of DNA fragments can have a size of about 100-300 bp, and preferably of about 150 bp.

According to some embodiments, the plurality of nucleic acid sequences in the biological sample comprise a plurality of RNA sequences, and the preparing a DNA sample from the biological sample comprises: treating the biological sample to thereby obtain a plurality of cDNA molecules, each corresponding to one of the plurality of RNA molecules.

The treating the biological sample to thereby obtain a plurality of cDNA molecules can comprise: performing a reverse transcription using an oligo(dT) as a primer to obtain a cDNA sequence corresponding to each of the plurality of RNA molecules.

According to some embodiments, prior to the performing a reverse transcription using an oligo(dT) as a primer to obtain a cDNA sequence corresponding to each of the plurality of RNA molecules, the treating the biological sample to thereby obtain a plurality of cDNA molecules further comprises: performing a polyadenylation at a 3' end of each of the plurality of RNA molecules.

According to some embodiments, the treating the biological sample to thereby obtain a plurality of cDNA molecules comprises: performing a reverse transcription using random primers or sequence-specific primers to obtain a cDNA sequence corresponding to each of the plurality of RNA molecules.

In the method disclosed herein, the first adaptor can comprise a single-stranded segment at the 5'end of the first strand thereof, and the ligating a first strand of a first adaptor to a 3' end of each of the plurality of single-stranded DNA molecules comprises: performing a ligation reaction through a single-stranded DNA ligase such that the 3' end of each of the plurality of single-stranded DNA molecules is ligated to the 5' end of the first strand of the first adaptor. Herein, the single-stranded DNA ligase can comprise at least one of CircLigase I or CircLigase II.

According to some embodiments of the method, the first adaptor further comprises a second strand, which comprises a first portion at a 5' end thereof and a second portion at a 3' end thereof. The first portion of the second strand has a length of at least 1 nt, and forms a double-stranded duplex with the 5' end of the first strand. The second portion has a length of at least 1 nt, and forms a single-stranded overhang in the first adaptor, and the ligating the 5' end of a first strand of a first adaptor to a 3' end of each of the plurality of single-stranded DNA molecules comprises: performing a ligation reaction through a bandage strand-facilitated DNA ligase such that the 3' end of each of the plurality of single-stranded DNA molecules is ligated with the 5' end of the first strand of the first adaptor.

Herein, the second portion can have a length of 4-10 nt. As such, the first adaptor can include a set of adaptors, each configured such that a second portion of a second strand thereof comprises a random sequence. The first adaptor can also include one or more adaptors, each configured such that a second portion of a second strand thereof comprises a specific sequence.

Herein the first portion can have a length of 8-18 nt. The bandage strand-facilitated DNA ligase can include at least one of T3 DNA ligase, T4 DNA ligase, T7 DNA ligase, or Taq Ligase.

In the method disclosed herein, the first strand of the first adaptor can further comprise an index sequence, which is disposed between the phosphate group and the barcode sequence, or between the barcode sequence and the first primer recognition sequence. The index sequence is configured to provide index information for each of the plurality of single-stranded DNA molecules ligated to the first strand of the first adaptor. Herein, the index sequence can have a length of 1-8 nt.

The first strand of the first adaptor can further comprise a separator sequence disposed between the phosphate group and the barcode sequence, which is configured to serve as a separation marker between the barcode sequence and each of the plurality of single-stranded DNA molecules ligated to the first strand of the first adaptor. Herein, the separator sequence can have a length of about 2-16 nt. According to some embodiments, the separator sequence can be further configured to provide index information for each of the plurality of single-stranded DNA molecules ligated to the first strand of the first adaptor.

In the method disclosed herein, the synthesizing a complementary strand for each of the plurality of single-stranded DNA molecules ligated to the first strand of the first adaptor to obtain a barcoded double-stranded DNA molecule corresponding thereto can comprise:

annealing a first primer with each of the plurality of single-stranded DNA molecules ligated to the first strand of the first adaptor, wherein the first primer comprises a sequence complementary to the first primer recognition sequence in the first strand of the first adaptor; and

performing a single-strand extension reaction to form a double-stranded DNA molecule for each of the plurality of single-stranded DNA molecules ligated to the first strand of the first adaptor.

Herein the annealing a first primer with each of the plurality of single-stranded DNA molecules ligated to the first strand of the first adaptor can include: slowly altering a temperature of a reaction from an original temperature to a working temperature for the single-stranded extension reaction. According to some embodiments, the first primer has a Tm of about 30-35°C, and the slowly altering a temperature of a reaction to a working temperature for the single-stranded extension reaction comprises: increasing the temperature from an original temperature of no more than ~15°C to the working temperature for the single-stranded extension reaction at a rate of no more than ~1°C per min. In one specific embodiment, the first primer recognition sequence has a sequence: CCTCAGCAAG (i.e. SEQ ID NO: 913), and correspondingly the first primer comprises a sequence: CTTGCTGAGG (i.e. SEQ ID NO.: 914), which is substantially a complimentary sequence of the first primer recognition sequence.

The synthesizing a complementary strand for each of the plurality of single-stranded DNA molecules ligated to the first strand of the first adaptor to obtain a barcoded double-stranded DNA molecule corresponding thereto can further include: performing a blunt-end repair to the double-stranded DNA molecule corresponding to the each of the plurality of single-stranded DNA molecules ligated to the first strand of the first adaptor. Herein, the blunt-end repair can be performed by at least one of T4 DNA polymerase, Klenow Fragment, or T4 polynucleotide kinase. Herein, the immobilization portion can include a first coupling partner, configured to be able to stably couple (i.e. covalently connect, or non-covalently but securely bind, etc.) to a second coupling partner attached to the solid support. According to some embodiments, the first coupling partner can comprise a biotin moiety, the second coupling partner can comprise at least one of a streptavidin moiety, an avidin moiety, or an anti-biotin antibody, and the solid support can comprise at least one of a magnetic bead, a filter, a resin bead, a nanosphere, a plastic surface, a microtiter plate, a glass surface, a slide, a membrane, or a matrix.

After the synthesizing a complementary strand for each of the plurality of single-stranded DNA molecules ligated to the first strand of the first adaptor to obtain a barcoded double-stranded DNA molecule corresponding thereto, the method can further comprise: ligating a second adaptor to a free end of the double-stranded DNA molecule corresponding to the each of the plurality of single-stranded DNA molecules immobilized to the solid support at an immobilized end thereof.

Herein the second adaptor can comprise a third strand and a fourth strand. The fourth strand comprises a phosphate group, which is at a 5' end thereof) and a second primer recognition sequence, which is configured to provide a priming site for amplification of the double-stranded DNA molecule corresponding to the each of the plurality of single-stranded DNA molecules. The third strand comprises a sequence complimentary to a 5'-end sequence of the fourth strand, and is configured to form a duplex with, and thereby to ensure a stability of, the 5'-end sequence of the fourth strand.

As such, the method further comprises: performing a PCR reaction to thereby amplify the double-stranded DNA molecule corresponding to the each of the plurality of single-stranded DNA molecules.

Herein the PCR reaction can be performed by means of a pair of primers respectively targeting the two end portions of the the double-stranded DNA molecule. In one specific embodiment, one of the pair of primers can comprise a sequence corresponding to at least a portion of a sequence of the first primer which has been used for the single-stranded extension reaction, and another of the pair of primers can comprise a sequence corresponding to at least a portion of a sequence in the fourth strand of the second adaptor. Herein "at least a portion" of a sequence can include part or all of the sequence.

Between the ligating a second adaptor to a free end of the double-stranded DNA molecule corresponding to the each of the plurality of single-stranded DNA molecules immobilized to the solid support at an immobilized end thereof and the performing a PCR amplification to each of the plurality of single-stranded DNA molecules, the method can further comprise: eluting the double-stranded DNA molecule corresponding to the each of the plurality of single-stranded DNA molecules from the solid support.

In a second aspect, the disclosure further provides a kit for constructing a DNA library from a biological sample containing a plurality of nucleic acid sequences, the kit includes a first adaptor, a DNA ligase, and a first prime,

the first adaptor includes a first strand, which comprises a phosphate group, a barcode sequence, and a first primer recognition sequence in a direction from a 5' end thereof to a 3' end thereof, the barcode sequence is configured to provide barcode information to each of the plurality of single-stranded DNA molecules ligated to the first strand of the first adaptor, the DNA ligase is configured to allow a ligation between the 5' end of the first strand of the first adaptor to a 3' end of each of a plurality of single-stranded DNA molecules, each of the plurality of single-stranded DNA molecules corresponds to one of the plurality of nucleic acid sequences in the biological sample, the first primer comprises a sequence complementary to the first primer recognition sequence of the first adaptor and is configured to allow for a single-strand extension reaction to thereby form a double-stranded DNA molecule corresponding to each of the plurality of single-stranded DNA molecules ligated to the first strand of the first adaptor, the kit disclosed herein further include a solid support, and the first strand of the first adaptor further include an immobilization portion at the 3' end thereof, which is configured to allow immobilization of each of the plurality of single-stranded DNA molecules ligated to the first strand of the first adaptor at the 5' end thereof to the solid support, the immobilization portion comprises a first coupling partner, configured to be able to form a stable coupling with a second coupling partner attached to the solid support, the stable coupling between the first coupling partner and the second coupling partner is a non-covalent binding,

the kit further includes a second adaptor, which is configured to ligate to a free end of the barcoded double-stranded DNA molecule corresponding to each of the plurality of single-stranded DNA molecules immobilized to the solid support at an immobilized end thereof, the second adaptor comprises a third strand and a fourth strand, the fourth strand include a phosphate group at a 5' end thereof, and futher include a second primer recognition sequence, which is configured to provide a priming site for amplification of the double-stranded DNA molecule corresponding to the each of the plurality of single-stranded DNA molecules, the third strand comprises a sequence complimentary to a 5'-end sequence of the fourth strand, and is configured to form a duplex with, and thereby to ensure a stability of, the 5'-end sequence of the fourth strand.

Herein, the fourth strand can further include at least one functional sequence at a 5' end of the second primer recognition sequence, which can comprise at least one of a second index sequence, or a second barcode sequence, or a sequencing primer sequence.

Herein the first primer can have a Tm of about 30-35°C, but can also have a Tm of about 55-65°C. In one specific embodiment, the first primer recognition sequence has a sequence: CCTCAGCAAG (i.e. SEQ ID NO: 913), and correspondingly the first primer comprises a sequence: CTTGCTGAGG (i.e. SEQ ID NO.: 914), which is substantially a complimentary sequence of the first primer recognition sequence. The barcode sequence has a length of about 2-16 nt.According to some embodiments, the stable coupling between the first coupling partner and the second coupling partner is a non-covalent binding, and the first coupling partner and the second coupling partner can respectively be one and another of a coupling pair, selected from one of a biotin-streptavidin pair, a biotin-avidin pair, a biotin-anti-biotin antibody pair, a carbohydrate-lectin pair, or an antigen-antibody pair. In one specific embodiment, the first coupling partner comprises a biotin moiety, and the second coupling partner comprises a streptavidin moiety attached to a magnetic bead.

According to some other embodiments, the stable coupling between the first coupling partner and the second coupling partner is a covalent connection, and the first coupling partner and the second coupling partner can respectively be one and another of a cross-linking pair. Examples of the cross-linking pair include an NHS ester-primary amine pair, a sulfhydryl-reactive chemical group pair (e.g. cysteines, or other sulfhydryls such as maleimides, haloacetyls, and pyridyl disulfides), an oxidized sugar-hydrazide pair, photoactivatable nitrophenyl azide's UV triggered addition reaction with double bonds leading to insertion into C-H and N-H sites or subsequent ring expansion to react with a nucleophile (e.g., primary amines), or carbodiimide activated carboxyl groups to amino groups (primary amines), etc.

The immobilization portion can further include a spacer between the first primer recognition sequence and the first coupling partner, and the spacer can include at least one C3 spacer unit.

According to some embodiments of the kit, the first strand of the first adaptor further includes an index sequence between the phosphate group and the barcode sequence or between the barcode sequence and the first primer recognition sequence, which is configured to provide index information for each of the plurality of single-stranded DNA molecules ligated to the first strand of the first adaptor. Herein, the index sequence can have a length of 1-8 nt.

According to some embodiments of the kit, the first strand of the first adaptor further comprises a separator sequence disposed between the phosphate group and the barcode sequence, which is configured to serve as a separation marker between the barcode sequence and each of the plurality of single-stranded DNA molecules ligated to the first strand of the first adaptor. Herein, the separator sequence can have a length of about 2-16 nt. According to some specific embodiments of the kit, the separator sequence can be further configured to provide index information for each of the plurality of single-stranded DNA molecules ligated to the first strand of the first adaptor.

In the kit, the first adaptor can be single-stranded, and the DNA ligase can be a single-stranded DNA ligase, which can comprises at least one of CircLigase I or CircLigase II.

Alternatively, the first adaptor can be partially double-stranded. In embodiments where the first adaptor comprises a single-stranded segment at the 5'end of the first strand, the DNA ligase can be a single-stranded DNA ligase, which can comprises at least one of CircLigase I or CircLigase II.

In some other embodiments, the first adaptor further comprises a second strand, which includes a first portion at a 5' end thereof and a second portion at a 3' end thereof. The first portion of the second strand forms a double-stranded duplex with the 5'end of the first strand, and the second portion forms a single-stranded overhang in the first adaptor. As such, the DNA ligase can be a bandage strand-facilitated DNA ligase, which can include at least one of T3 DNA ligase, T4 DNA ligase, T7 DNA ligase, or Taq DNA ligase. Herein, the first portion can have a length of 8-18 nt, and the second portion can have a length of 4-10 nt. According to some embodiments, the first adaptor can comprise a set of adaptors, each configured such that a second portion of a second strand thereof comprises a random sequence. According to some other embodiments, the first adaptor can comprise one or more adaptors, each configured such that a second portion of a second strand thereof comprises a specific sequence.

The kit disclosed herein can further include a second adaptor, which is configured to ligate to a free end of the barcoded double-stranded DNA molecule corresponding to each of the plurality of single-stranded DNA molecules immobilized to the solid support at an immobilized end thereof. The second adaptor can comprise a third strand and a fourth strand. The fourth strand include a phosphate group at a 5' end thereof, and futher include a second primer recognition sequence, which is configured to provide a priming site for amplification of the double-stranded DNA molecule corresponding to the each of the plurality of single-stranded DNA molecules. The third strand comprises a sequence complimentary to a 5'-end sequence of the fourth strand, and is configured to form a duplex with, and thereby to ensure a stability of, the 5'-end sequence of the fourth strand. Herein, the fourth strand can further include at least one functional sequence at a 5' end of the second primer recognition sequence, which can comprise at least one of a second index sequence, or a second barcode sequence, or a sequencing primer sequence.

In the kit as described above, the third strand can further include, at a 5' end of thereof, at least one of: a cap structure, an overhang sequence, or a functional moiety. The cap structure can include a sequence that does not match with a 3'-end sequence of the fourth strand, and is configured to avoid concatenation of the second adaptor in a ligation reaction. The overhang sequence can form a single-stranded segment for the second adaptor.

The kit can further include a pair of primers, which are configured to amplify the double-stranded DNA molecule corresponding to the each of the plurality of single-stranded DNA molecules therethrough.

Herein the pair of primers can be configured to respectively target the two end portions of the the double-stranded DNA molecule. In one specific embodiment, one of the pair of primers can comprise a sequence corresponding to at least a portion of a sequence of the first primer which has been used for the single-stranded extension reaction, and can, for example, comprise a sequence corresponding to the first primer recognition sequence in the first adaptor. Another of the pair of primers can comprise a sequence corresponding to at least a portion of a sequence in the fourth strand of the second adaptor. Herein "at least a portion" of a sequence can include part or all of the sequence.

The kit as disclosed herein may further include a third adaptor that can be ligated to the free ends of the first adaptor, and can be engineered to be compatible with commercial sequencing platforms to work together with the second adaptor to perform pair-end sequencing or to work along to perform sequencing starting from the first adapter sequence to the DNA molecule corresponding to each of the plurality of single-stranded DNA molecules.

These and other embodiments which will be apparent to those of skill in the art upon reading the specification provide the art with methods for assessing, characterizing, and detecting genetic markers, such as cancer markers, and genetic analysis, such as SNV identification. In particular, it provides methods for constructing single-stranded nucleic acids into libraries for desired analysis.

Throughout the disclosure, the term "about" or "around", and the sign "~" as well, generally refers to plus or minus 10% of the indicated number. For example, "about 20" may indicate a range of 18 to 22, and "about 1" may mean from 0.9-1.1. Other meanings of "about" may be apparent from the context, such as rounding off, so, for example "about 1" may also mean from 0.5 to 1.4.

As used herein, the term "double-stranded duplex", "hybridization" or "annealing" refers to the pairing of complementary (including partially complementary) polynucleotide strands. Hybridization and the strength of hybridization (e.g., the strength of the association between polynucleotide strands) is impacted by many factors well known in the art including the degree of complementarity between the polynucleotides, stringency of the conditions involved affected by such conditions as the concentration of salts, the melting temperature (Tm) of the formed hybrid, the temperature of the hybridization reaction, the presence of other components, the molarity of the hybridizing strands and the G:C content of the polynucleotide strands. When one polynucleotide is said to "hybridize" to another polynucleotide, it means that there is some complementarity between the two polynucleotides or that the two polynucleotides form a hybrid under high stringency conditions. When one polynucleotide is said to not hybridize to another polynucleotide, it means that there is no sequence complementarity between the two polynucleotides or that no hybrid forms between the two polynucleotides at a high stringency condition.

As used herein, the term "complementary" refers to the concept of sequence complementarity between regions of two polynucleotide strands (e.g. a double-stranded structure) or between two regions of the same polynucleotide strand (e.g. a "loop" or "hairpin" structure). It is known that an adenine base of a first polynucleotide region is capable of forming specific hydrogen bonds ("base pairing") with a base of a second polynucleotide region which is antiparallel to the first region if the base is thymine or uracil. Similarly, it is known that a cytosine base of a first polynucleotide strand is capable of base pairing with a base of a second polynucleotide strand which is antiparallel to the first strand if the base is guanine. A first region of a polynucleotide is complementary to a second region of the same or a different polynucleotide if, for example, when the two regions are arranged in an antiparallel fashion, at least one nucleotide of the first region is capable of base pairing with a base of the second region. Therefore, it is not required for two complementary polynucleotides to base pair at every nucleotide position. "Complementary" refers to a first polynucleotide that is 100% or "fully" complementary to a second polynucleotide and thus forms a base pair at every nucleotide position. "Complementary" also refers to a first polynucleotide that is not 100% complementary (e.g., 90%, or 80% or 70% complementary) contains mismatched nucleotides at one or more nucleotide positions. In one embodiment, two complementary polynucleotides are capable of hybridizing to each other under high stringency hybridization conditions.

Throughout the disclosure, the term "bandage strand-facilitated DNA ligase" is referred to as a DNA ligase that can catalyze a ligation between a 5' end of a first DNA strand and a 3' end of a second strand, facilitated by the presence of a third strand (i.e. "bandage strand") that has one segment complimentary to the 5' end of the first DNA strand and another segment complimentary to the 3' end of the second strand. Herein the bandage strand-facilitated DNA ligase includes, but is not limited to, T4 DNA ligase, T3 DNA ligase, T7 DNA ligase, and Taq DNA ligase, etc. The term "single-stranded DNA ligase" as in this disclosure is referred to as a DNA ligase that can catalyze the ligation between a 5' end of a first DNA strand and a 3' end of a second strand in an absence of the bandage strand.

Unless indicated otherwise, all sequences in the present disclosure have a direction from 5' end to 3' end.

## BRIEF DESCRIPTION OF THE DRAWINGS

FIG. 1 is a flow chart of a method for constructing a nucleic acid library from a biological sample;

FIGS. 2A, 2B, and 2C are respectively a flow chart of step S100 in the method as shown in FIG. 1 according to three different embodiments of the disclosure;

FIGS. 3A, 3B, and 3C are repectively a flow chart of a step S109 according to several different embodiments of the disclosure;

FIG. 4 illustrates a first adaptor having a single-stranded configuration;

FIG. 5A illustrates a first adaptor having an immobilization portion;

FIG. 5B illustrates a covalent connection by which the immobilization portion of the first adaptor can be coupled to a solid support;

FIG. 5C illustrates a noncovalent connection by which the immobilization portion of the first adaptor can be coupled to a solid support;

FIG. 5D illustrates a molecular structure of a spacer unit that forms the spacer in the immobilization portion of the first adaptor;

FIGS. 6A and 6B respectively illustrate a first adaptor having an index sequence according to two embodiments of the disclosure;

FIG. 6C illustrates the mechanism for the index sequence in the first adaptor to differentiate different biological sample (#1, #2, ..., #n) being analyzed simultaneously;

FIG. 7 shows a first adaptor having a separator sequence;

FIG. 8A shows a first adaptor according to yet another embodiment of the disclosure;

FIG. 8B shows one specific example of the first adaptor shown in FIG. 8A;

FIGS. 9A and 9B respectively illustrate a first adaptor having a partially double-stranded configuration according to two different embodiments of the disclosure;

FIG. 10 is a flow chart of step S300 in the method as shown in FIG. 1;

FIG. 11 is a flow chart of a method for constructing a nucleic acid library including steps for PCR amplification according to some embodiments of the disclosure;

FIGS. 12A, 12B, 12C, 12D and 12E each illustrate a second adaptor according to several different embodiments of the disclosure;

FIGS. 13A and 13B respectively illustrate two embodiments of the process in which each double-stranded DNA sequence in the DNA library is amplified;

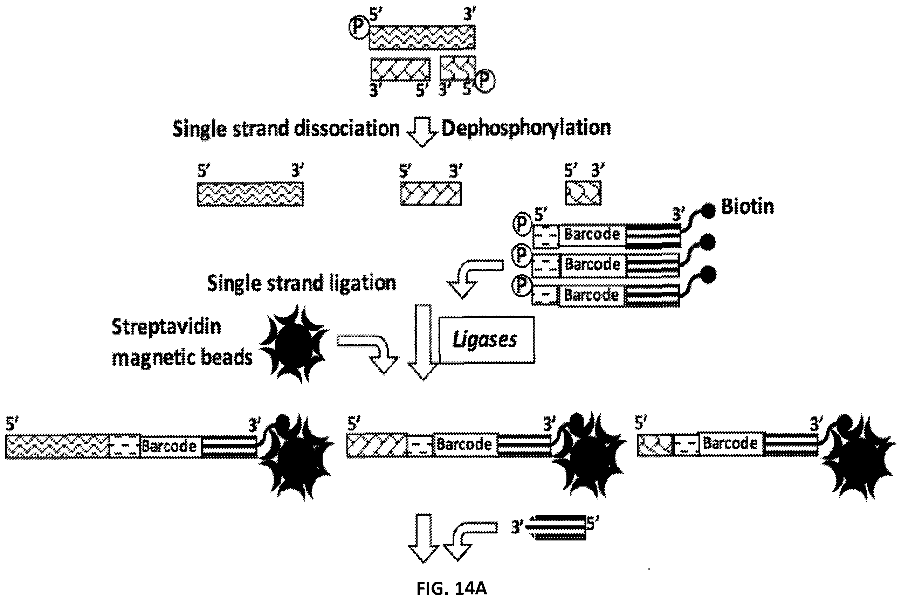

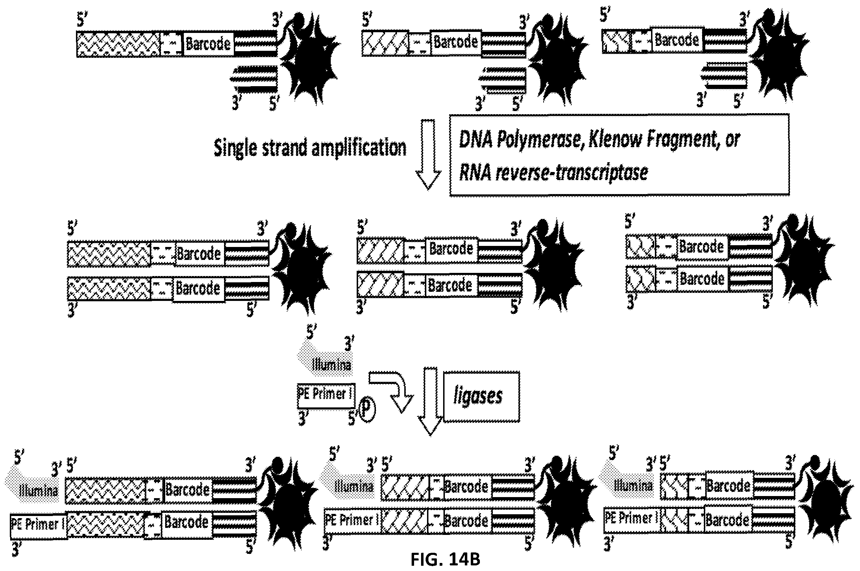

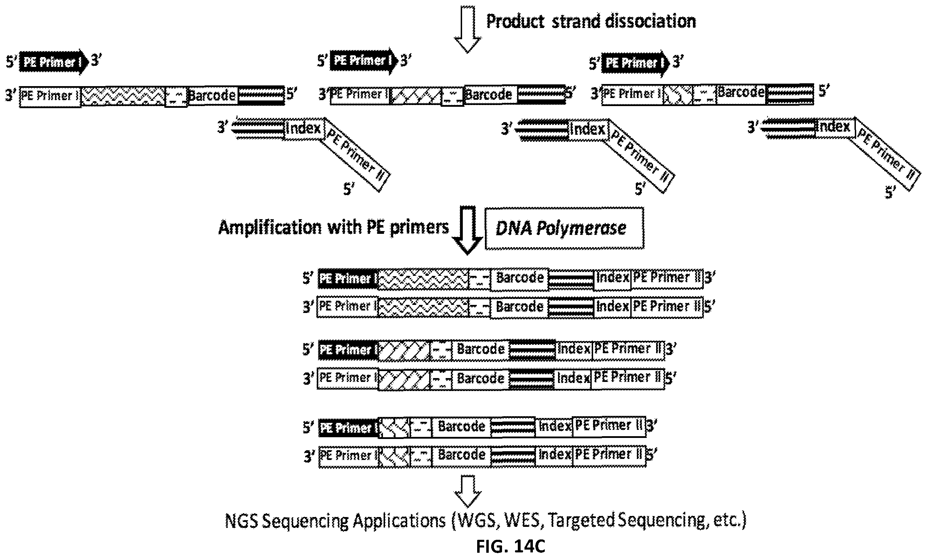

FIGS. 14A, 14B and 14C provide a schematic of a single strand DNA based library construction strategy. A double-stranded DNA molecule (bearing a damaged strand or not) is heated to dissociate complementary DNA single strands. A barcoded (12nt) single-stranded adapter is appended to the 3' end of a single-stranded DNA molecule and the entire molecule is immobilized on a streptavidin-bead. A PCR primer complementary to the 3' sequence on every adapter is added as a primer to synthesize the complementary sequences of the initial single-stranded DNA molecule and the barcode. Illumina PE sequencing adapter is appended to the 3' end of the newly synthesized complementary single-stranded DNA. PE primer I and a joined primer of single-stranded adapter - index - PE primer II are used to amplify the DNA fragments in the library. After amplification, the library is ready for direct NGS sequencing or subgenomic capture for targeted sequencing.

FIG. 15 shows incorporation ratios of single-stranded DNAs in barcoded single-stranded library pipeline. The fractions of DNA molecules that were incorporated into barcoded single-stranded library construction from different amounts of starting DNA (500ng, 20ng, 1ng, 100pg, 20pg and 10pg genomic DNA) were plotted. Ratios were measured by Qubit® ssDNA Assay Kit on a Qubit unit (ThermoFisher Scientific).

FIG. 16 shows the genomic locations of 298 cancer related genes on human chromosomes (indicated by arrows).

FIGS. 17A and 17B show the six $\Delta C_t$ values for each of the 298 genes calculated by real-time PCR assays detecting gene abundance difference between 500ng original genomic DNA input and 500ng barcoded single-stranded library final products created from six different amounts (500ng, 20ng, 1ng, 100pg, 20pg and 10pg) of input genomic DNA.

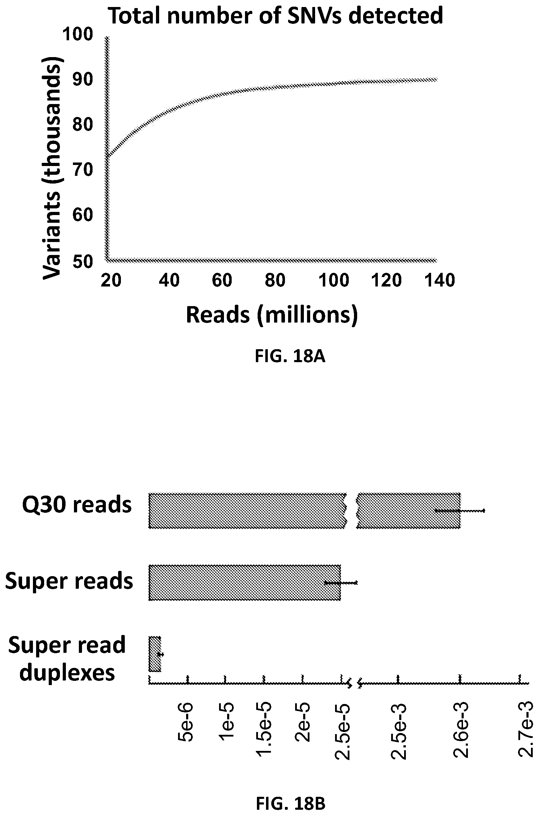

FIGS. 18A and 18B show SNV-calling trends and statistics of barcoded single-stranded library based WES (Whole Exome Sequencing) study. (FIG. 18A) Total number of SNVs detected at increasing read count thresholds. Sensitivity increases at higher read counts but quickly reaches a plateau with more than 80 million reads. (FIG. 18B) Average SNV frequencies of normal tissue DNA measured by three approaches: a standard NGS approach where barcodes were directly trimmed off, a super read based approach by barcoded single-stranded library based NGS without matching variants from both DNA strands (without the last step of the 4-step procedure), and a super read approach by barcoded single-stranded library based NGS matching the SNV on both strands (all steps in the 4-step procedure were performed).

FIGS. 19A, 19B, and 19C show read statistics. (FIG. 19A) Bar plot of percentage of initial reads, mapped reads and reads remained after filtering. Results were obtained from three technical replicates. Numbers of reads were shown under each bar with the unit of 1 million reads. (FIG. 19B) Stacked bar plot of subgroups of filtered reads in triple replicates. (FIG. 19C) Coverage efficiency correlation with read numbers. The percentage of target bases covered at $\geq$ 10X, $\geq$20X, $\geq$50X and $\geq$ 100X depths with 5 million to 50 million reads were shown;

FIGS. 20A and 20B show density plots of read depths to demonstrate the relationship between GC content against normalized mean read depth for (FIG. 20A) barcoded single-stranded library WES study with normal tissue DNA, and (FIG. 20B) barcoded single-stranded library WGS study with normal tissue DNA (without enrichment for whole exome);

FIG. 21 shows detection of ultra-rare SNVs in libraries created from normal DNA spiked with sequentially diluted tumor DNA samples. Reduced amounts of variants were re-detected from sequentially diluted samples. No variant was re-detected from 1:10,000 diluted group. Coverage of re-sequencing is ~5,000 X.

FIGS. 22A-22N show the 298-gene panel real-time PCR parameters and corresponding primer sequences.

FIG. 23 shows data yield from barcoded single-stranded library WES sequencing. Initial mapped reads represent raw reads that contain the 12nt barcode and mapped to the reference genome. Unique read family represents the number of URF. Each URF has a unique barcode and its sequence is obtained by consolidating read sequences arise from the same DNA molecule by PCR amplification. PCR errors are removed by requesting a sequence uniformity for over 95% of the reads within a URF. Super read duplexes represent the number of DNA duplex whose two strands are coming from two super reads.

FIGS. 24A-24E show results of mutation and ultra-rare mutation detection by barcoded single-stranded library based NGS. Sequence variants detected by barcoded single-stranded library based NGS, validation results by Sanger sequencing and ultra-rare mutation redetection results are shown and ranked by MAF (Mutant Allele Fraction).

DETAILED DESCRIPTION OF THE INVENTION

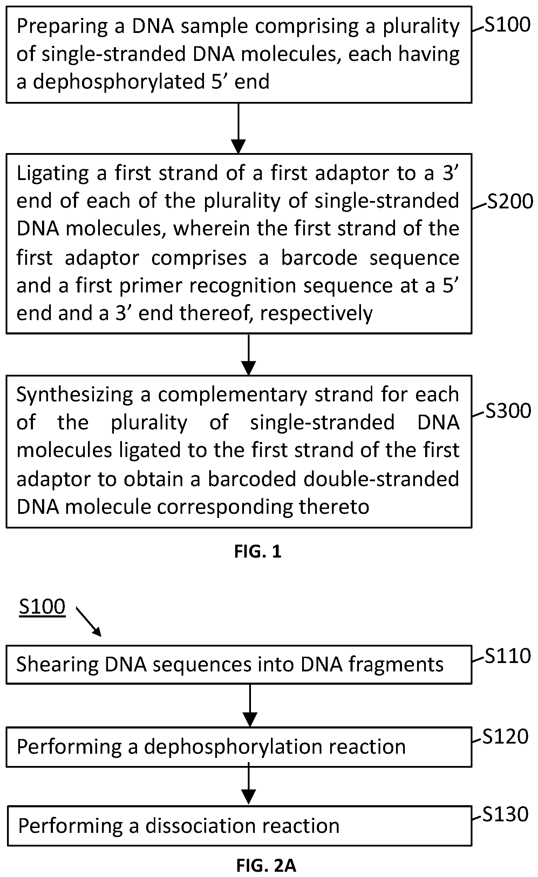

The present disclosure provides a method for constructing a nucleic acid library from a biological sample containing a plurality of nucleic acid sequences. As illustrated in FIG. 1, the method includes the following steps as set forth in S100-S300:

S100: preparing a DNA sample from the biological sample, wherein the DNA sample comprises a plurality of single-stranded DNA molecules, each having a dephosphorylated 5' end.

According to some embodiment of the method, the biological sample comprises a plurality of DNA sequences that are often double-stranded and commonly have phosphorylated 5' ends, and as such, step S100 can include the following sub-steps, as illustrated in FIG. 2A:

S110: Shearing the plurality of DNA sequences into DNA fragments;
S120: Performing a dephosphorylation reaction over the DNA fragments to thereby obtain dephosphorylated DNA fragments; and
S130: Performing a dissociation reaction over the dephosphorylated DNA fragments to thereby obtain the plurality of single-stranded DNA molecules.

Herein the biological sample can have a plurality of double-stranded DNA sequences, and can typically be a genomic DNA sample from a tissue, a mitochondrial DNA sample, or a cell-free DNA sample from blood or other body fluids, etc. These different types of DNA samples can be prepared based on different assays that are conventional in the field, whose description is skipped herein. Herein by means of step S100, the DNA sample comprising a plurality of single-stranded DNA molecules can be obtained from the biological sample.

In sub-step S110, the length of each DNA fragment can have a range of around 100-300 bp (preferably around 150 bp), but can vary depending on different needs. The DNA molecules in the biological sample can be sheared by a conventional shearing method. In one example, a DNA sample can be sheared into fragments of around 150 bp with Diagenode's Bioruptor at a program of 7 cycles of 30 seconds ON/90 seconds OFF using 0.65 ml Bioruptor® Microtubes. It is noted that sub-step S 110 may be optional and can vary depending on the source, nature, and composition of the biological sample. In one example, long nucleic acid sequences, such as genomic DNA obtained from a conventional preparation approach which typically have large double-stranded DNA fragments, can be sheared into small fragments. In another example, circulating cell free DNAs (cfDNAs) commonly purified from human plasma typically have a size of around 140 - 170 bp and may not need to be sheared, or only need minor shearing.

Sub-step S120 is configured to remove the phosphate group at the 5' end of any DNA fragment to thereby prevent the formation of concatemers between different nucleic acid fragments from the sample in the subsequent ligation reaction. Herein sub-step S120 can be performed at 37°C in the presence of a phosphatase (such as the FastAP Alkaline Phosphatase) for 5~10 min. Other reaction conditions are also possible.

In sub-step S130, the plurality of dephosphorylated DNA fragments can be dissociated from a double-stranded form to become a single-stranded form, to thereby obtain a plurality of single-stranded DNA molecules. As such, the sample can be heated at 95°C for 3-15 min and snap-frozen on ice. Other reaction conditions are also possible.

It is noted that there can be other embodiments of step S100 regarding the order and the cycles for the sub-steps S120 and S130.

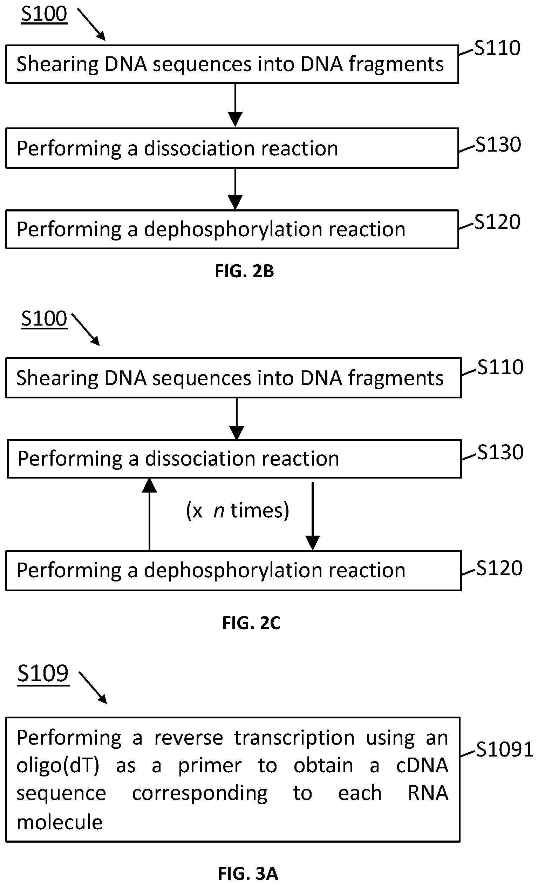

In one specific embodiment, after S110, the dissociation reaction (i.e. S130) can be performed prior to the dephosphorylation reaction (i.e. S120), as illustrated in FIG. 2B. This can be suitable for a DNA sample in which some double-stranded DNA molecules have nicks or gaps in one or both of the strands. Due to presence of a nick or a gap in a strand, the 5' end of the strand at the nick/gap commonly has a phosphate group, which is typically resistant to the dephosphorylation treatment. Yet if the DNA fragments are dissociated into single-stranded DNA molecules, the phosphate group can be presented at the 5' end of a sequence and can be removed by the dephosphorylation treatment.

To ensure that as many phosphate groups in the nicks/gaps or at the ends of DNA strand as possible are to be removed, in some embodiment of step S100, after S110, sub-steps S130 and S120 can be done in n cycles (n ≥ 2), as illustrated in FIG. 2C.

The actual selection of the various embodiments of step S100, as respectively illustrated in FIGS. 2A, 2B, or 2C, can depend on the nature and quality of the DNA molecules in the sample, and can also depend on actual needs.

According to some other embodiments of the method, the biological sample may contain a plurality of RNA sequences, and the method is employed to construct a DNA library from the plurality of RNA molecules in the biological sample. Correspondingly, prior to sub-step S110, step S100 comprises a sub-step of:

S109: preparing a cDNA sample comprising a plurality of cDNA molecules from the biological sample, wherein each cDNA molecule corresponds to one of the plurality of RNA molecules.

If mRNAs in the biological sample are included as the target nucleic acid sequences for the construction of the DNA library, because typically each mRNA contains a poly(A) tail at a 3' end thereof, specifically as shown in FIG. 3A, sub-step S109 includes:

S1091: Performing a reverse transcription using an oligo(dT) as a primer to thereby obtain a cDNA sequence corresponding to each of the plurality of RNA molecules.

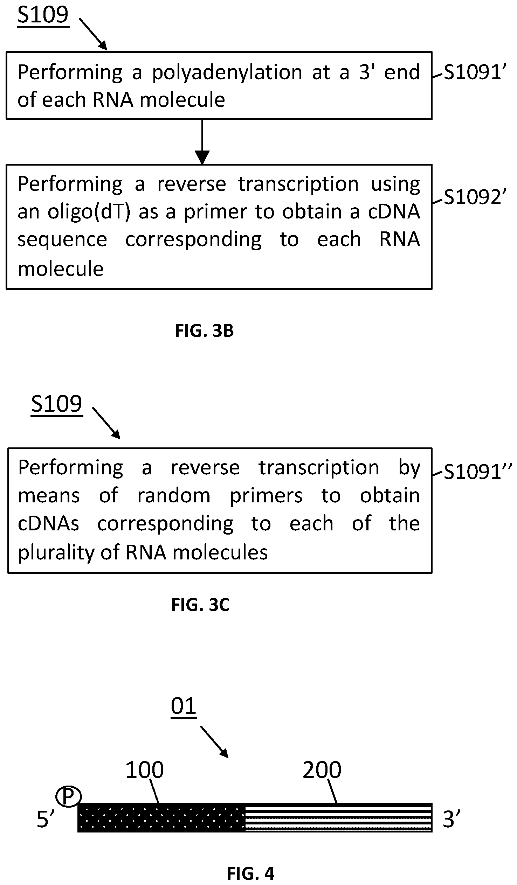

If RNAs in the plurality of RNA molecules other than the mRNAs are also included as the target nucleic acid sequences for the construction of the DNA library, because they typically do not have poly(A) tails at 3' ends, thus specifically, as illustrated in FIG. 3B, sub-step S109 includes the following sub-steps:

    S1091' : Performing a polyadenylation at a 3' end of each of the plurality of RNA molecules; and
    S1092' : Performing a reverse transcription using an oligo(dT) as a primer to thereby obtain a cDNA sequence corresponding to each of the plurality of RNA molecules.

Herein S1091' can be performed to each RNA molecule by means of a poly(A) polymerase to corresponding obtain a treated RNA molecule having a poly (A) tail. S1092' can include: annealing of the oligo(dT) primer with the poly (A) tail of each treated RNA molecule, and performing a reverse transcription in presence of a reverse transcriptase. The actual processes for S1091' and S1092' are well-known by people of ordinary skills in the field, and the description is skipped herein.

Alternatively, each RNA sequence in the biological sample can be reversely transcribed by means of random primers or sequence-specific primers. As shown in FIG. 3C, sub-step S109 can include:

S1091": Performing a reverse transcription initiated by a set of random primers or sequence-specific primers to obtain cDNAs corresponding to each of the plurality of RNA molecules.

The above-mentioned embodiments of the method can be applied to a biological sample containing only RNA molecules, which is prepared, for example, by a RNA purification protocol that is known to those of ordinary skills in the field. It can also be applied to a biological sample containing both DNA molecules and RNA molecules.

It is noted that every cDNA molecule obtained from reverse transcription of a RNA molecule by the two embodiments of sub-step S109 as shown in FIGS. 3A and 3B has an oligo(dT) sequence at its 5' end, which can serve as a specific marker for its original RNA source in the biological sample and can differentiate from any DNA molecule in the same biological sample, which is typically absent of the oligo(dT) sequence at its 5' end.

It is further noted that if only RNAs in the biological sample are targeted, during extraction of the RNAs, the genomic DNA can be removed by a RNA purification protocol that is known to people of ordinary skills in the field.

S200: ligating a first strand of a first adaptor to a 3' end of each of the plurality of single-stranded DNA molecules, wherein the first strand of the first adaptor comprises a barcode sequence and a first primer recognition sequence at a 5' end and a 3' end thereof respectively.

FIG. 4 illustrates a structural diagram of the first adaptor according to a first embodiment of the disclosure. As shown in FIG. 4, the first adaptor 01 is substantially a single-stranded adaptor (i.e. it comprises only the first strand) including a barcode sequence 100 and a first primer recognition sequence 200 at a 5' end and a 3' end thereof, respectively. Additionally, the first strand of the first adaptor 01 also has a phosphate group at the 5' end thereof, configured to allow the ligation of the first strand of the first adaptor 01 to a 3' end of each of the plurality of single-stranded DNA molecules obtained from step S100, which can be carried out, for example, by a single-stranded DNA ligase (e.g. CircLigase I, CircLigase II, etc.).

Herein in the first adaptor 01, the barcode sequence 100 substantially allows each single-stranded DNA molecule to be labelled uniquely. The barcode sequence can have any length, and can have preferably a length of 2-16 nt. According to some embodiments of the disclosure, the barcode sequence 100 has a length of 12 nt, which can uniquely apply a total of $4^{12}$ (or 16,777,216) different adaptors to a plurality of single-stranded DNA molecules. It should be noted that the length of the barcode sequence 100 can vary, depending on different needs in practice, for example, on the estimated complexity and abundance of different single-stranded DNA molecules in the DNA sample.

The first primer recognition sequence 200 in the first strand of the first adaptor 01 is substantially a universal primer recognition sequence across different DNA molecules, which allows each uniquely barcodedly labelled single-stranded DNA molecule to be conveniently amplified to obtain double-stranded DNA molecules in a subsequent single-cycle PCR reaction by means of a first primer 200' having a sequence complementary to the first primer recognition sequence 200 (as described below). Herein the first primer 200' can thus be regarded as a universal primer. It is noted that to avoid non-specific amplification of sequences in the above mentioned single-cycle PCR reaction, the first primer recognition sequence 200 can be configured to have a relatively unique sequence among different genes and across different species. Thus the first primer recognition sequence 200 may vary based on the nature and species of the target nucleic acid sample.

According to some embodiments, the first primer recognition sequence 200 is further configured to have a Tmthat allows efficient or specific amplification for the single-cycle PCR reaction, depending on different needs. The first primer recognition sequence 200 can optionally have a length of 5-30nt.

According to some preferred embodiments, the first primer recognition sequence 200 has a Tm of ~30-35°C, and a length of 8-12 nt. For example, the first primer recognition sequence 200 in one specific embodiment, which has a sequence of "CCTCAGCAAG" (i.e. SEQ ID NO: 913), has a length of 10 nt. In addition, to balance the length and Tm, the first primer recognition sequence 200 can be selected such that it has a GC content between 40%-70%, and is lack of any repetitive sequences. It is noted that this above configuration is especially suitable for constructing a DNA library directly from original DNA sequences in a DNA sample without any prior amplification. The use of a short first primer recognition sequence 200 in the first adaptor 01 allows a subsequent synthesis of a complementary strand of each single-strand DNA molecule (i.e. the amplification reaction for the single-cycle PCR reaction) to be efficiently performed in the presence of a short primer (i.e. the first primer 200' as described below, which has a sequence complementary to the first primer recognition sequence 200) having a relatively low Tm.

According to some other embodiments, the first primer recognition sequence 200 has a length of 13-30 nt and has a Tm of 55-65°C, just like a regular PCR primer sequence. This configuration allows a relative more specific amplification for the single-cycle PCR reaction to meet certain practical needs.

It is noted that besides the first adaptor 01 as shown in FIG. 4 which substantially takes a single-stranded form, the first adaptor 01 can also take a partially double-stranded form, which will be covered below in detail. Hereafter, unless mentioned explicitly, all descriptions involving the first adaptor 01 is based on the first embodiment of the first adaptor 01 (i.e. the single-stranded adaptor 01 as shown in FIG. 4).

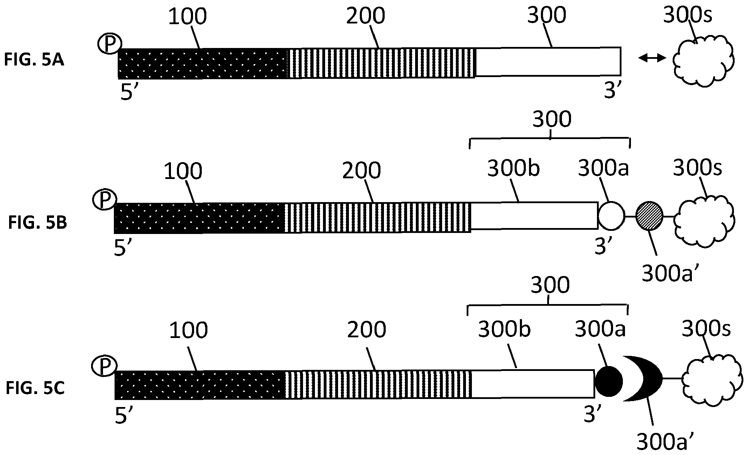

In addition to the barcode sequence 100 and the first primer recognition sequence 200 as described above, the first adaptor 01 can optionally include an immobilization portion 300, disposed at a 3' end of the first adaptor 01 (i.e. 3' end of the first primer recognition sequence 200) and configured to allow immobilization of the plurality of single-stranded DNA molecules attached therewith at a 5' end of the first adaptor 01 to a solid support 300s, as illustrated in FIG. 5A.

Herein the solid support 300s can be a filter, a bead (such as resin, or a magnetic bead, etc.), a nanosphere, a plastic surface, a microtiter plate, a glass surface, a slide, a membrane, a matrix (which can be packed into a cartridge or column structure), etc., and selection of specific solid support 300s can depend on the convenience, purpose, and situation. The solid support 300s can be treated and derivatized as is known in the art.

Immobilization of the plurality of single-stranded DNA molecules to the solid support 300s can be direct or indirect. According to some embodiments as illustrated in FIG. 5B, the immobilization portion 300 is directly linked, for example via a covalent connection, to a solid support 300s, which may rely on a pair of coupling partners capable of cross-linking therebetween. According to some other embodiments as illustrated in FIG. 5C, the immobilization portion 300 is indirectly attached to a solid support 300s, by means of, for example, the non-covalent and stable binding between a pair of coupling partners.

As such, in any of these above embodiments of the first adaptor 01, the immobilization portion 300 can include a first coupling partner 300a, which is covalently or non-covalently but stably attached to a 3' end of the first adaptor 01 (i.e. a 3' end of the first strand of the first adaptor 01). The first coupling partner 300a is configured to form a stable coupling or attachment with a second coupling partner 300a' immobilized (or covalently attached) to a solid support 300s, without interfering with other events.

Herein the stable attachment between the first coupling partner 300a and the second coupling partner 300a' can be a covalent connection, and as such the first coupling partner-second coupling partner pair can be, but is not limited to, the functional group pair of NHS esters-primary amines. Alternatively, the stable attachment between the first coupling partner 300a and the second coupling partner 300a' can be a non-covalent binding (or bonding), and as such the first coupling partner-second coupling partner pair can be, but is not limited to, a biotin-streptavidin/avidin pair, a biotin-anti-biotin antibody pair, a carbohydrate-lectin pair, and an antigen-antibody pair.

For example, the first coupling partner 300a can be a dye (e.g. a fluorescence dye), and the second coupling partner 300a' can be an antibody that specifically and stably binds with the first coupling partner 300a (i.e. the dye). The use of dye as the first coupling partner 300a allows the target sequence ligated to the first adaptor 01 to be visualized, and thus additionally providing a means for quality control or for other purposes.

As such, the stable attachment between the first coupling partner 300a and the second coupling partner 300a' allows the first adaptor 01, along with each single-stranded DNA molecule ligated thereby, to be immobilized to the solid support 300s, facilitating the capture, enrichment, isolation, and purification of the DNA molecules, which in turn brings convenience in subsequent reactions (e.g. PCR amplification, NGS sequencing, etc).

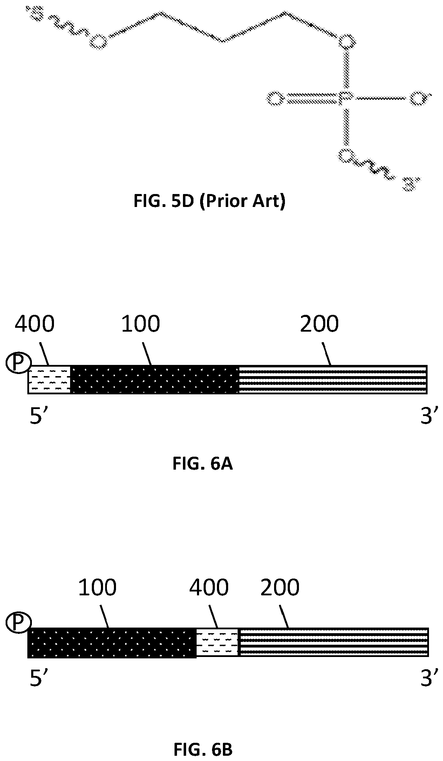

In order to increase the efficiency for the first primer 200' to bind with the first primer recognition sequence 200 in the first adaptor 01 to thereby facilitate the subsequent single-cycle PCR reaction, the immobilization portion 300 can be configured to further include a spacer 300b, disposed between the first primer recognition sequence 200 and the first coupling partner 300a. A length of the spacer 300b can rely on the nature and composition of the immobilization portion 300. In one illustrating example also illustrated in FIG. 5C, the first coupling partner 300a in the immobilization portion 300 is a biotin moiety, the second coupling partner 300a' is streptavidin/avidin/anti-biotin antibody, which is covalently attached to a magnetic bead (i.e. the solid support 300s), and the spacer 300b can be a C3 spacer (i.e. C3 Spacer phosphoramidite) having a length of 6-12 spacer units. The structure of a spacer unit is known in the field and is shown in FIG. 5D.

It should be noted that the biotin-streptavidin pair as described above and illustrated in FIG. 5C shall be construed only as one illustrating example, and thus shall not be interpreted as a limitation to the scope of the disclosure. Other first coupling partner-second coupling partner pairs may be used as well, as long as they can provide a strong coupling without an interference to the subsequent reactions.

It is further noted that the spacer 300b can include other spacer units, and can further include another moiety, such as triethyleneglycol (TEG). Herein the TEG spacer can be disposed to attach a biotin moiety, which can avoid hindrance issues and can be beneficial for attaching oligonucleotides to nanospheres or magnetic beads.

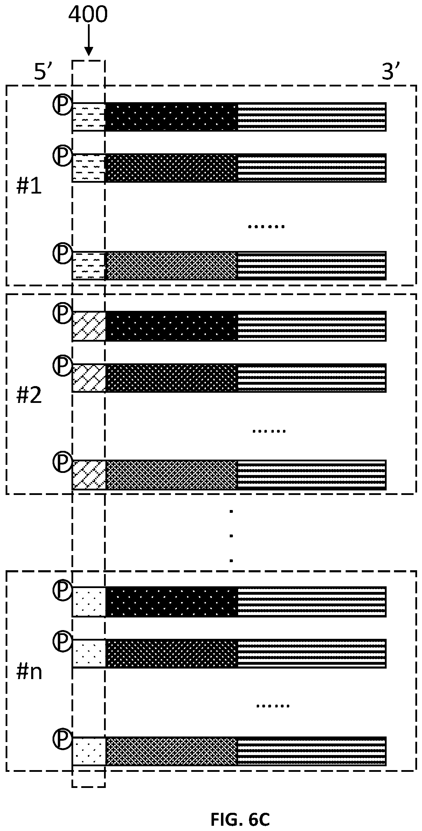

Additionally, the first adaptor 01 can optionally include an index sequence 400, disposed either at a 5' end of the first adaptor 01 (i.e. at a 5' end of the barcode sequence 100, as shown in FIG. 6A) or between the barcode sequence 100 and the first primer recognition sequence 200 (as shown in FIG. 6B). The index sequence 400 is configured to provide index information for each single-stranded DNA molecule. As illustrated in FIG. 6C, the index information provided by the index sequence 400 can, for example, indicate which biological sample (#1, #2, ..., #n) one particular single-stranded DNA molecule is from, thus allowing differentiation among two or more biological samples, in turn facilitating simultaneous analysis of the two or more biological samples. The index sequence 400 can have a length that depends on a total number of biological samples to be assayed. Preferably, the index sequence 400 can have a length of 1-8 nt. In one specific example, the index sequence can have a sequence of "CCCAA".

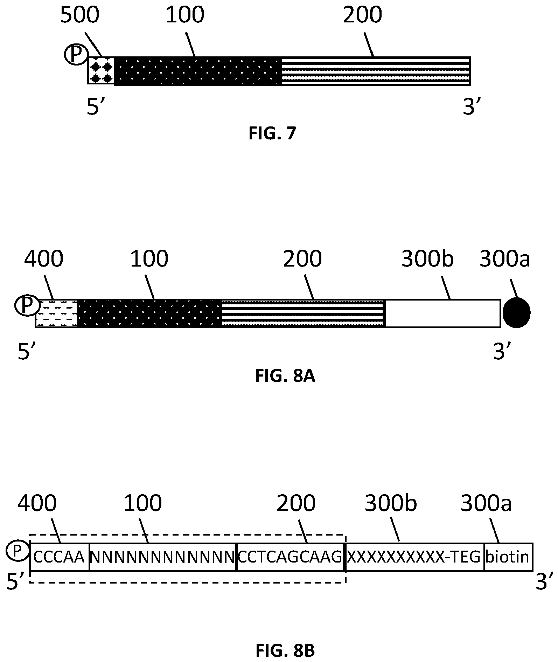

Furthermore, the first adaptor 01 can optionally include a separator sequence 500, disposed at a 5' end of the barcode sequence 100 (i.e. 5' end of the first adaptor 01 as illustrated in FIG. 7). The separator sequence 500 can have a length of 2-16 nt, and substantially serves as a separation marker between the barcode sequence 100 and the single-stranded sequence ligated thereto, which can be used to differentiate a ligated sequence and a barcode sequence in a subsequent sequencing effort. In addition, the separator sequence 500 can also provide a quality control information to the first adaptor 01 and to the single-stranded DNA molecule ligated thereto. For example, due to imperfect manufacturing of the first adaptor 01, the first adaptor 01 can have a loss of one or more nucleotides at the 5' end, possibly resulting in difficulties in differentiating barcode sequence 100 from the ligated DNA sequence, if the barcode sequence 100 is at the very 5' end of the first adaptor 01. However, the presence of the separator sequence 500 would allow a clear separation and distinguishing between the barcode sequence and the ligated nucleic acid sequence, and also this structure can provide a quality control means for the analysis if any defect existing in the barcode sequence during synthesis, and to provide a clear boarder line between ligated nucleic acid sequence and the adapter which is required by bioinformatics analysis. It is noted that in some preferred embodiments, the index sequence 400 can be integrated with the separator sequence 500, and in these embodiments, the separator sequence 500 at the 5' end of the first adaptor substantially comprises the index sequence 400.

According to some embodiments as illustrated in FIG. 8A, the first adaptor includes, in a 5' end-to-3' end direction, a phosphate group, an index sequence 400, a barcode sequence 100, a first primer recognition sequence 200, a spacer 300b, and a functional moiety 300a.

One specific example of the first adaptor as described above and shown in FIG. 8A is illustrated in FIG. 8B, which substantially comprises a polynucleotide sequence: CCCAANNNNNNNNNNNNCCTCAGCAAG as set forth in SEQ ID NO: 915 (shown in a box with dotted lines), a phosphate group and a modification (XXXXXXXXXX-TEG-biotin) connected repectively to the 5' end and the 3' end of the polynucleotide sequence. Herein, "CCCAA" (i.e. residues 1-5 of SEQ ID NO: 915) is substantially the index sequence 400 which could also serve the functionatlity of separator sequence 500 (not shown in the figure), "NNNNNNNNNNNN" (i.e. residues 6-17 of SEQ ID NO.:x, each "N" represents a nucleotide residue) is the barcode sequence 100, "CCTCAGCAAG" (i.e. residues 18-27 of SEQ ID NO: 915, which is also the sequence as set forth in SEQ ID NO: 913) is the first primer recognition sequence 200, "XXXXXXXXXX-TEG" (n=10, each "X" indicates a C3 spacer unit, and "TEG" the triethylene glycol) is the spacer 300b, and "biotin" is the first coupling partner 300a.

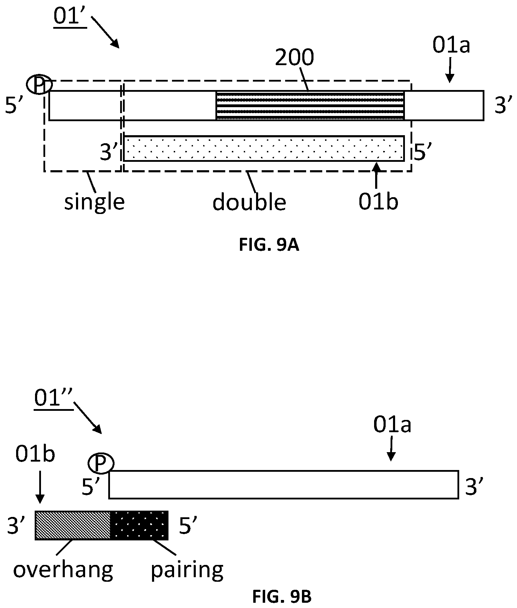

In addition to the aforementioned single-stranded first adaptor 01 (i.e. the first adaptor 01 includes only the first strand and all functional elements are substantially in the first strand of the first adaptor 01), which is described as a first embodiment of the first adaptor 01 and illustrated in FIGS. 4, 5A-5C, 6A-6C, 7, and 8, the first adaptor 01 can also be partially double-stranded, as illustrated in FIG. 9A and FIG. 9B. As such, the first adaptor 01 substantially includes a first strand 01a and a second strand 01b. The first strand 01a is substantially identical to, and thus comprises all elements of, the first strand of the first adaptor 01 in the first embodiment of (i.e. the single-stranded adaptor as shown in FIG. 4), yet the second strand 01b can vary in each different embodiment of the partially double-stranded first adaptor 01.

In a second embodiment of the first adaptor as shown in FIG. 9A, the first adaptor 01' consists of a first strand 01a and a second strand 01b. The first adaptor 01' comprises a single-stranded segment (labelled as "single") corresponding to the 5' end of the first strand 01a and a double-stranded segment (labelled as "double") comprising a sequence that corresponds to the whole or part of the first primer recognition sequence 200. The single-stranded segment has a length of at least 1 nt, configured to allow a subsequent ligation between the first strand 01a of the first adaptor 01' with each single-stranded DNA molecule under the action of a single-stranded DNA ligase (e.g. CircLigase I and CircLigase II). In the double-stranded segment of the first adaptor 01', the second strand 01b comprises a sequence that is at least complementary to, and thereby forms a double-strand duplex with, the whole or part of first primer recognition sequence 200, and as such, can be utilized in step S300 (as described below) as a primer to synthesize a complementary strand for each single-stranded DNA molecule ligated to the first adaptor 01' to obtain a barcoded double-stranded DNA molecule corresponding thereto.

In a third embodiment of the first adaptor as shown in FIG. 9B, the first adaptor 01" consists of a first strand 01a and a second strand 01b. The second strand 01b of the first adaptor 01" comprises a first portion at a 5' end and a second portion at a 3' end. The first portion of the second strand 01b forms a double-stranded duplex with the 5' end of the first strand 01a (i.e. the double-stranded segment, labelled as "pairing" in the figure) in the first adaptor 01". The first portion of the second strand 01b can have a length of at least 1 nt, and preferably of 8-18 nt, and can correspond to (i.e. have a sequence complimentary to) a sequence element at the 5' end of the first strand 01a, which can include the separator sequence 500, the index sequence 400, or a partial sequence in the barcode sequence 100, depending on different embodiments. The second portion substantially forms a single-stranded overhang (i.e. the single-stranded segment, labelled as "overhang" in the figure) in the first adaptor 01". The second portion in the second strand 01b can have a length of at least 1 nt, and preferably of 4-10 nt. This configuration allows the second strand 01b to substantially serve as a "bandage strand" to facilitate the ligation of the first strand 01a of the first adaptor 01" with a single-stranded DNA molecule whose 3' end sequence is complementary to the "overhang" sequence on the second strand 01b as illustrated in FIG. 9B under the action of a bandage strand-facilitated DNA ligase, such as a T4 DNA ligase, T3 DNA ligase, T7 DNA ligase, Taq DNA ligase, etc.

It is noted that due to the presence of the bandage strand (i.e. the second strand 01b in the first adaptor 01"), the ligation reaction by means of the bandage strand-facilitated DNA ligase (e.g. T4 DNA ligase) is demonstratably more efficient than a ligation reaction using a single-stranded DNA ligase. Additionally, the "overhang" sequence (i.e. the second portion) on the second strand 01b of the first adaptor 01" can add selection power to the ligation reaction by selectively annealing to target single-stranded DNA molecules whose 3' end sequences are complementary to the "overhang" sequences.

In order to ensure a sufficient coverage, according to some embodiments, the first adaptor 01" substantially includes a set of adaptors, where the second portion in the second strand of each adaptor comprises a random sequence, configured such that the random sequences in the second portion in the second strand of the plurality of adaptors together can cover all possible sequences of the 3' end of the plurality of single-stranded DNA molecules. As such, all possible single-stranded DNA sequences in the sample can be ligated to the first adaptor 01" to thus be incorporated in the library via the bandage strand-facilitated DNA ligase (e.g. T4 DNA ligase).

According to some other embodiments, the second portion of the first adaptor 01" can comprise one or more specific sequences, which allow a relatively specific ligation of the first adaptor 01" with certain target species in the single-stranded DNA molecules whose 3' end sequences are complementary to the second portion.

In step S200, the ligation of the 5' end of the first strand of the first adaptor to the 3' end of each of the plurality of single-stranded DNA molecules is carried out by a DNA ligase. In other words, under the action of the DNA ligase, a 5' end of the first strand of the first adaptor can be ligated to a 3' end of each of the plurality of single-stranded DNA molecules. Herein the DNA ligase can be any of CircLigase II, CircLigase I, T4 DNA ligase, etc.

The CircLigase II and CircLigase I can be the single-stranded DNA ligase used to perform a ligation between each of the plurality of single-stranded DNA molecules and the single-stranded first adaptor 01 (as shown in FIG. 4) or the first strand 01a of the partially double-stranded first adaptor 01' (the second embodiment as shown in FIG. 9A). The ligation reaction can be performed at 30-60°C. In one specific example, pre-dephosphorylated fragmented DNA samples can be mixed with the above mentioned first adaptor (final concentration 0.15uM), 20% PEG-8000, 100U CircLigase II, and can be incubated at 60°C for 1 hour. The ligation reaction can also be carried out at 60°C for 1.5 hour or at 30°C for 4 hours. The T4 DNA ligase can be used for the ligation between each of the plurality of single-stranded DNA molecules and the partially double-stranded first adaptor 01 (the third embodiment as shown in FIG. 9B). The ligation reaction can, for example, be carried out at 16°C for 1-3 hours, but can also be performed at 4-30°C.

Herein by ligating the the first strand of the first adaptor 01 to the 3'end of each single-stranded DNA molecule in step S200, each single-stranded DNA molecule is substantially labelled individually with a unique barcode (via the barcode sequence 100 in the first adaptor 01).

In embodiments where the first strand of the first adaptor 01 contains a first coupling partner 300a configured to be immobilized to a solid support attached to a second coupling partner 300b (via the stable coupling between the first coupling partner 300a and the second coupling partner pair 300b), after step S200 and before step S300 (mentioned below), the method includes the following step:

S250: immobilizing each of the plurality of single-stranded nucleic acid molecules ligated to the first strand of the first adaptor to a solid support.

Step S250 can be performed by incubating each single-stranded DNA molecule ligated to the first strand of the first adaptor 01 with the solid support at an appropriate temperature for an appropriate time period. In one specific example, the solid support is magnetic beads coupled with streptavidin, and the first adaptor is coupled with biotin. As such, the incubation can be performed at room temperature for 10-30 min. It is noted that this step S250 is optional and can be skipped in cases where no solid support is needed.

S300: synthesizing a complementary strand for each of the plurality of single-stranded DNA molecules ligated to the first strand of the first adaptor to obtain a barcoded double-stranded DNA molecule corresponding thereto.

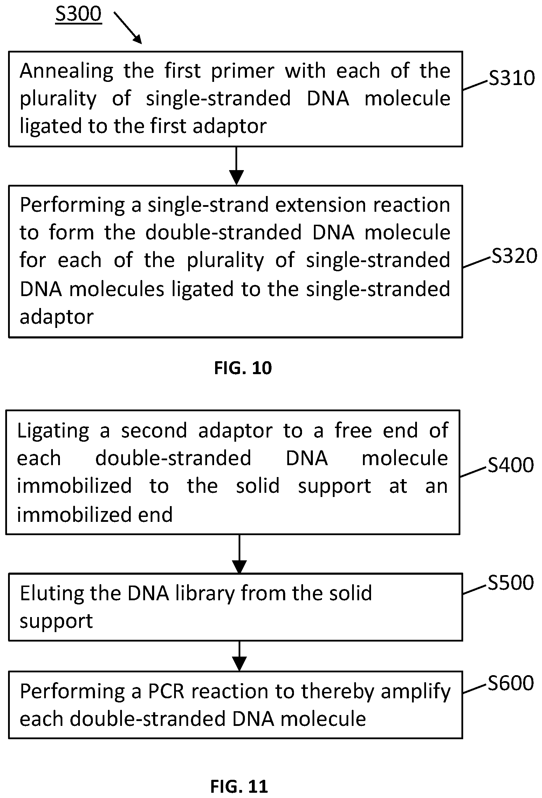

Herein S300 can be performed by a single-cycle PCR reaction via the aforementioned first primer 200', which comprises a sequence complementary to the first primer recognition sequence 200 in the first strand of the first adaptor 01. Specifically, if the first adaptor 01 takes a single-stranded form as shown in FIG. 4 or takes a partially double-stranded form as shown in FIG. 9B, step S300 can include the following sub-steps, as illustrated in FIG. 10:

S310: annealing the first primer with each of the plurality of single-stranded DNA molecules ligated to the first strand of the first adaptor; and
S320: performing a single-strand extension reaction to form the double-stranded DNA molecule for each of the plurality of single-stranded DNA molecules ligated to the first strand of the first adaptor.

Herein S310 is to ensure a sufficient binding of the first primer 200' with the first primer recognition sequence 200 in each single-stranded DNA molecule ligated to the first strand of the first adaptor, so that the single-stranded extension reaction (i.e. single-cycle PCR) can occur in sub-step S320. Specifically, sub-step S310 can include: slowly altering (increasing or decreasing) a temperature of the reaction to a working temperature (i.e. reaction temperature) of the single-stranded extension.

In one specific example where the first primer 200' has a Tm of 32°C, S310 specifically includes: (1) adding the first primer to the reaction, and incubating the reaction at 65°C for 2 min before quickly cooling on ice; (2) adding a BST DNA polymerase in the reaction, and incubating the reaction at 15°C; and (3) slowly increasing the temperature of the reaction at a rate of around 1°C per min, until the temperature reaches 37°C. Correspondingly, S320 includes: incubating the reaction at 37°C for 3-10 min. It is noted that in this specific example where the first primer 200' has a relatively low Tm (~30°C), the reaction temperature can only be slowly increased to give rise to satisfactory results, and based on an actual experiment, the manner of slowly decreasing the temperature of the reaction fails to obtain a satisfactory result.

In another specific example, the first primer 200' has a Tm of 60°C, S310 involves: (1) adding the first primer and a BST DNA polymerase to the reaction, and incubating the reaction at 70-80°C for 2 min; (2) slowly cooling the temperature of the reaction at a rate of around 1°C per min, until the temperature reaches about 60°C. Correspondingly, S320 includes: incubating the reaction at a temperature within the range of 50-72°C for 30 min. It is noted that in the above example where the first primer 200' has a relatively high Tm (~60°C), it is also possible to slowly increase the reaction temperature.

It is noted that in S320, besides the BST 3.0 polymerase, other DNA polymerases (such as Klenow fragment) or a RNA reverse transcriptase can also be used.

Optionally, after S320, the method can further include a sub-step:

S330: performing a blunt-end repair to each double-stranded DNA molecule obtained from the single-stranded extension reaction.

After the single-stranded extension reaction in S320, each double-stranded molecule may have a 3' overhang, which needs to be removed to ensure a high efficiency for any subsequent treatment, such as ligation with a second adaptor 02 as described below. Specifically, S330 can be performed in the presence of a T4 DNA polymerase (having a 3' end exonuclease activity) and incubated at 25°C for 15 minutes. Besides T4 DNA polymerase, other choices include Klenow Fragment or T4 polynucleotide kinase. It is possible to mixedly use these above enzymes.

It should be noted that if the first adaptor 01 takes a partially double-stranded form as shown in FIG. 9A, no additional first primer 200' is needed in the single-stranded extension reaction, because the second strand 01b of the first adaptor 01' comprises a sequence corresponding to the first primer 200'. As such, S310 is skipped, and step S300 only involves the aforementioned S320.

After the above steps S100 (i.e. preparing single-stranded DNA molecules), S200 (i.e. ligating a first strand of a first adaptor to each single-stranded DNA molecule), optionally S250 (i.e. immobilizing ligation product), and S300 (i.e. synthesizing complementary strand for each single-stranded DNA molecule), a DNA library comprising a plurality of barcode-labelled double-stranded DNA sequences is thus constructed. Each barcode-labelled double-stranded DNA sequence corresponds to one original single-stranded nucleic acid molecule.

The DNA library may subject to further treatment or analysis depending on different purposes. For example, the DNA library may be treated such that each barcode-labelled single-stranded DNA molecule can be inserted into a vector, allowing for subsequent amplification and/or expression in a model organism (such as in *E. Coli,* a yeast, or a phage). Alternatively, the DNA library may subject to amplification to thereby obtained amplified DNA library before a subsequent genetic analysis, such as sequencing analysis, a variant/mutation analysis, or a copy number analysis, can be performed.

In the following, a specific example is provided to illustrate steps implicated to amplify each barcode-labelled double-stranded DNA sequence in the DNA library, in order to facilitate a subsequent analysis of the single-stranded nucleic acid molecules corresponding thereto. Specifically, each single-stranded nucleic acid molecule is pre-treated in step S100, labelled with a first strand of a first adaptor attached with a biotin moiety at a 3'end thereof in step S200, immobilized to a solid support (more specifically, streptavidin-conjugated magnetic beads) in step S250 (via the biotin-streptavidin binding pair), and further treated to allow the synthesis of a complementary strand for each barcode-labelled single-stranded nucleic acid molecule in step S300. After these above steps, each original single-stranded nucleic acid molecule is converted into a corresponding barcode-labelled double-stranded DNA molecule immobilized onto a magnetic bead, which then undergoes further treatment to allow an amplification and a subsequent sequencing analysis using substantially an Illumina sequencing platform.

Specifically, as illustrated in FIG. 11, the following steps are carried out after step S300, in order to amplify each double-stranded DNA molecule ligated to the solid support obtained after step S300.

S400: ligating a second adaptor to a free end of each double-stranded DNA molecule immobilized to the solid support at an immobilized end.

Herein in the DNA library, each barcode-labelled double-stranded DNA sequence corresponding to one original single-stranded nucleic acid molecule is immobilized to the magnetic beads at the immobilized end via the aforementioned bonding between a biotin moiety attached to a 3' end of the first adaptor and a streptavidin moiety attached to the magnetic beads. The free end of each double-stranded DNA molecule is substantially the end opposing to the immobilized end.

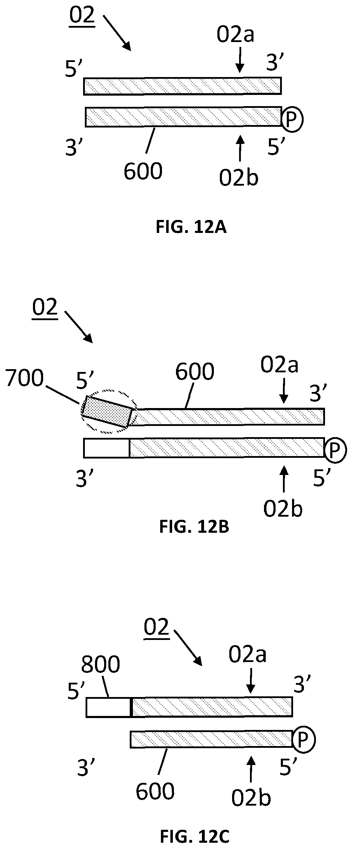

FIGS. 12A-12E illustrate several different embodiments of a second adaptor as mentioned in S400. In the embodiment as shown in FIG. 12A, the second adaptor 02 is substantially a universal double-stranded adaptor comprising a third strand 02a and a fourth strand 02b. The fourth strand 02b includes a second primer recognition sequence 600 and a phosphate group at a 5' end of the fourth strand 02b. The second primer recognition sequence 600 is configured to allow a subsequent PCR reaction to occur by means of a pair of primers, one of which (i.e. a second primer 600') has a sequence at a 3' end thereof that matches the second primer recognition sequence 600. The phosphate group is configured to allow the fourth strand 02b to ligate with the free 3' end of each double-stranded DNA molecule immobilized to the solid support at the immobilized end.

The third strand 02a comprises a sequence that is at least complimentary to a 5'-end sequence of the fourth strand 02b, and is configured to form a duplex with, and thereby ensures a stability of, the 5'-end sequence in the fourth strand 02b. In order to prevent the formation of concatemers or unwanted ligation products during subsequent ligation reaction, the third strand 02a is configured to have no phosphate group at its 5' end.

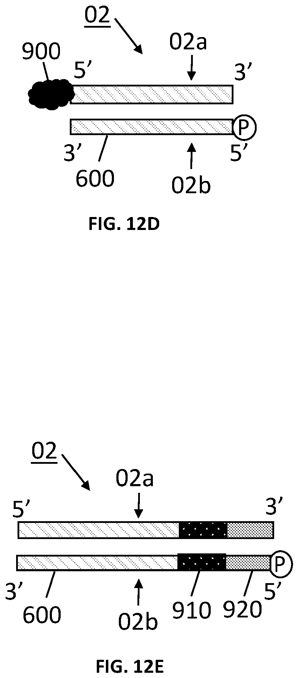

According to some other embodiment as shown in FIG. 12B, the third strand 02a further comprises a cap structure 700 at its 5' end, which can comprise a sequence or a moeity that does not match with the 3' end of the fourth strand 02b (which could be the 3' end second primer recognition sequence 600, or could be a sequence not in the second primer recognition sequence 600, as shown in FIG. 12B). Because of the non-matching between the 5' end of the third strand 02a and the 3' end of the fourth strand 02b, the second adaptor 02 substantially forms a Y-shaped adaptor, as shown in FIG. 12B. According to yet some other embodiment as shown in FIG. 12C, the third strand 02a further comprises an overhang sequence 800 at its 5' end, which substantially forms a single-stranded segment for the second adaptor 02. Other configurations for the second adaptor 02 are also possible. According to yet some other embodiment as shown in FIG. 12D, the third strand 02a further comprises a functional moiety 900 at its 5' end, configured to prevent the formation of concatemers yet also to provide a means for a subsequent treatment or analysis. For example, the functional moiety can be a binding partner, which can form a cross-link or a stable non-covalent binding with another binding partner, allowing for the further immobilization of the captured sequences. The functional moiety can also serve a marker (such as a dye). There are no limitations herein.

It is noted that besides the second primer recognition sequence 600, the fourth strand 02b of the second adaptor 02 can further comprise one or several other functional sequences, such as a second index sequence 910, a second barcode sequence 920, etc., as illustrated in FIG. 12E. Each of these functional sequences is disposed at the 5' end of the second primer recognition sequence 600 in order to allow the amplification of each captured sequence along with these functional sequences. It is further noted that in practical practice, the second adaptor utilized in S400 can substantially comprise a combination of these aforementioned embodiments as illustrated in FIGS. 12A-12E, to realize a mixed use.

Specifically, ligation of the second adaptor 02 to the free end of each double-stranded DNA molecule immobilized to the solid support at the immobilized end can be performed using a T4 DNA ligase with an incubation at 16°C for 1 hour, and the reaction can be performed using other enzymes and under other reaction conditions.

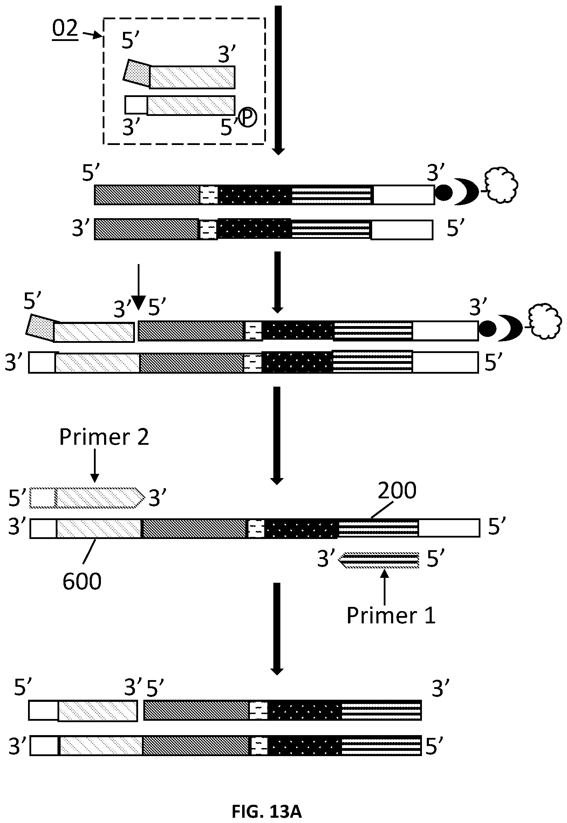

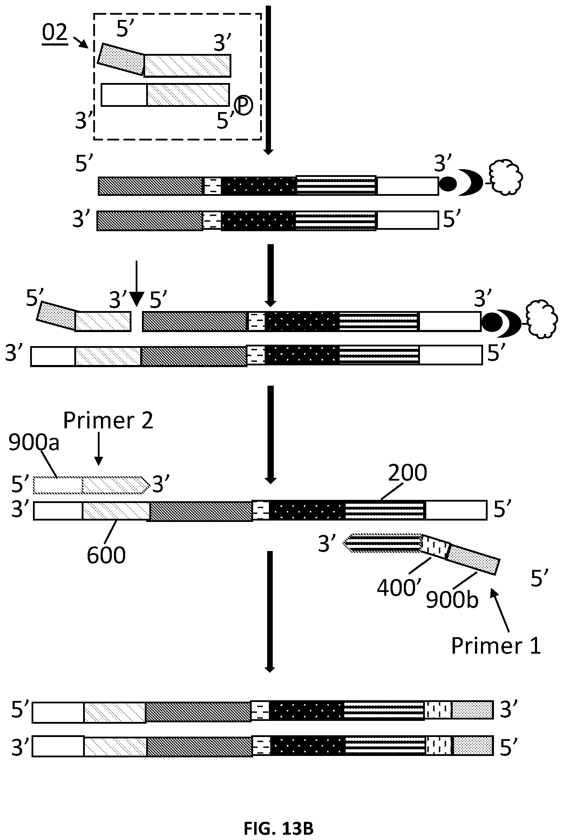

It is noted that because of the lack of a phosphate group in the free end (more specifically the 5' end) of each double-stranded DNA molecule immobilized to the solid support, only the 3' end at the free end of each double-stranded DNA molecule is ligated to the 5' end of the fourth strand 02b, and there is a gap/nick between the 3' end of the third strand 02a of the second adaptor 02 and the 5' dephosphorylated end (formed in step S100) on the original single-stranded DNA molecule in each double-stranded DNA molecule (as shown by the arrow in FIGS. 13A and 13B).

S500: eluting the DNA library from the solid support.

Herein in step S500, a strand complementary to the barcode-labelled and solid support-immobilized strand of each double-stranded DNA molecule in the DNA library can be eluted from the solid support, and the eluted strand substantially includes the second primer recognition sequence 600 in the second adaptor. In one specific example, step S500 can be performed by incubation at 95°C for 5 minute in the presence of an elution buffer (such as TET buffer composed of 10mM Tris-HCl, 1mM EDTA, 0.05% Tween-20). Under these conditions, the original single-stranded DNA molecule ligated to the first strand in first adaptor can also be eluted from the solid support due to the instable binding of single biotin-streptavidin coupling at high temperature, but this DNA strand can not serve as PCR template because of the 5' dephosphorylation gap on the original single-stranded DNA molecule which leads to no universal primer recognoization sequence on the newly formed 3' end after the first cycle of PCR amplification.

S600: performing a PCR reaction to thereby amplify each double-stranded DNA molecule.

Herein the PCR reaction can be performed by means of a pair of primers respectively targeting the two end portions of each double-stranded DNA molecule.

According to some preferred embodiments, one of the pair of primers (i.e. Primer 1) can comprise a sequence corresponding to at least a portion of a sequence of the first primer which has been used for the single-stranded extension reaction, and another of the pair of primers (i.e. Primer 2) can comprise a sequence corresponding to at least a portion of a sequence in the fourth strand of the second adaptor. Herein "at least a portion" of a sequence can include part or all of the sequence.

It is noted that there is no limitation regarding the pair of primers used in the PCR reaction as long as each double-stranded DNA molecule corresponding to the each of the plurality of single-stranded DNA molecules in the sample can be amplified. Therefore, the one of the pair of primers (i.e. Primer 1) used in S600 can include a 3' end portion that corresponds to a portion, or all, of the first primer recognition sequence 200, but can possibly include a sequence that does not correspond to the first primer recognition sequence 200 but corresponds to the sequence of the first primer at 5' end of the first primer recognition sequence 200 (such as a second index sequence 400' and a second sequencing primer sequence 900b in FIG. 13B described below). Similarly, the other of the pair of primers (i.e. Primer 2) can include a 3' end sequence that corresponds to at least a portion of the second primer recognition sequence 600 in the second adaptor 02, but can have other options.

In addition, the pair of primers can be enigineered as well. For example, Primer 1 can comprise a sequence corresponding to the first primer recognition sequence 200 as mentioned above, but can also include other functional elements, depending on practical needs. Similarly, the second primer can comprise a sequence corresponding to the second primer recognition sequence 600 as mentioned above, but can also include other functional elements.

FIG. 13A and FIG. 13B illustrate two embodiments of the method for amplifying a DNA library that has been constructed by the aforementioned steps S100, S200, and S300.

In the embodiment as shown in the FIG. 13A, Primer 1 only contains a sequence corresponding to the first primer recognition sequence 200 without other functional elements (thus Primer 1 is substantially the first primer 200' as mentioned above), and Primer 2 contains a sequence corresponding to the second primer recognition sequence 600.

In the embodiment as shown in FIG. 13B, Primer 2 also contains a first sequencing primer sequence 900a at a 5' end thereof in addition to a sequence corresponding to the second primer recognition sequence 600. In Primer 1, besides the sequence corresponding to the first primer recognition sequence 200, Primer 1 also includes a second index sequence 400' and a second sequencing primer sequence 900b.

In both of the embodiments as shown in FIGS. 13A and 13B, the sequence corresponding to the first primer recognition sequence 200 in Primer 1 and the sequence corresponding to the second primer recognition sequence 600 in Primer 2 allow for the amplification of the target sequence (i.e. each of the plurality of single-stranded DNA molecule, shown as dark solid bars in FIGS. 13A and 13B) along with other tags (i.e. the index sequence, the barcode sequence, etc.).

Furthermore, the presence of other functional sequences would allow the target sequence to be sequenced or for other purposes. For example, in some embodiments, Primer 1 and Primer 2 respectively include a pair of sequencing primers (e.g. Primer 2 includes a PE Primer I sequence, and Primer 1 includes a PE primer II sequence), thus the amplified target sequence can undergo direct sequencing using a current NGS sequencing platform (e.g. Illumina sequencing platform). Similarly, other functional elements such as the second index sequence 400' can allow for an additional differentiation among different samples, for a convenience for a subsequent analysis.

Thus through steps S400-S600 as described above, each double-stranded DNA molecule in the DNA library that corresponds the barcode-labelled single-stranded nucleic acid molecule can be amplified. As such, there are sufficient copies for each barcode-labelled single-stranded nucleic acid molecule in a subsequent analysis, such as next generation sequencing (NGS) analysis, which can improve the sensitivity.

In addition to the sequencing analysis as described above, the amplified DNA molecules in the DNA library that correspond to the originally single-stranded nucleic acid molecules in the biological sample allow for further nucleic acid assays. Any means of testing for a sequence variant or sequence copy number variant, including without limitation, a point mutation, a deletion, an amplification, a loss of heterozygosity, a rearrangement, a duplication, may be used. Sequence variants may be detected by sequencing, by hybridization assay, by ligation assay, etc. Non-targeted assays may be used, where the location of a sequence variant is unknown. If locations of the relevant sequence variants are defined, specific assays which focus on the identified locations may be used, such as targeted sequencing, point-mutation targeted sequencing analysis (e.g. SAFE-SeqS, Duplex Sequencing, etc.). Any assay that is performed on a test sample involves a transformation, for example, a chemical or physical change or act. Assays and determinations are not performed merely by a perceptual or cognitive process in the body of a person.

The following are further noted. Single-stranded nucleic acid library construction can make assays feasible that would otherwise fail to yield valid sequencing-ready materials. The biological sample can be from any appropriate sources in the patient's body that will have nucleic acids from a cancer or lesion that can be collected and tested. Test samples can be also from any appropriate sources derived from patient tissue, such as FFPE slides, FFPE tissue blocks, and test samples can be also from any appropriate sources derived from other biological specimens, such as fossils, body remains of ancient human species or animal species.

Suitable test samples may be obtained from body tissue, stool, and body fluids, such as blood, tear, saliva, sputum, bronchoalveolar lavage, urine and different organ secreted juices. The samples may be collected using any means conventional in the art, including from surgical samples, from biopsy samples, from endoscopic ultrasound, phlebotomy, etc.

Obtaining the samples may be performed by the same person or a different person that conducts the subsequent analysis. Samples may be stored and/or transferred after collection and before analysis. Samples may be fractionated, treated, purified, enriched, prior to assay. Any of the assay results may be recorded or communicated, as a positive act or step. Communication of an assay result, diagnosis, identification, or prognosis, may be, for example, orally between two people, in writing, whether on paper or digital media, by audio recording, into a medical chart or record, to a second health professional, or to a patient. The results and/or conclusions and/or recommendations based on the results may be in a natural language or in a machine or other code. Typically, such records are kept in a confidential manner to protect the private information of the patient or the project.

Collections of barcoded adaptors, primers, control samples, and reagents can be assembled into a kit for use in the methods. The reagents can be packaged with instructions, or directions to an address or phone number from which to obtain instructions. An electronic storage medium may be included in the kit, whether for instructional purposes or for recordation of results, or as means for controlling assays and data collection.

Control samples can be obtained from the same patient from a tissue that is not apparently diseased, or can be obtained from a healthy individual or a population of apparently healthy individuals. Control samples may be from the same type of tissue or from a different type of tissue than the test sample. Control samples may be provided together with the barcoded adaptors, primers, and reagents in a kit for use in the method, where the control samples may be a standard reference sample for the purpose of validating the performance of the kit and the operation performed by the user.

The data described below document the results for the identification of ultra-rare mutations from a whole exome sequencing study based on one specific embodiment of the method for constructing a nucleic acid library as described above.

Barcoded single-stranded library construction method (as described above) is used to generate barcoded single-strand DNA based library for NGS studies. The barcode on each individual single-stranded DNA mulecule is used as a marker to label each individual DNA sequence, only when a sequence variant (an SNV) is identified at the same corresponding sites on two complementary DNA strands labeled by different and non-complementary barcodes, can an SNV be called. Such barcoded single-stranded library is PCR error-proof and facilitates the identification of ultra-rare mutations (SNVs).

SNVs can be detected with confidence only when the sequencing system's error rate is significantly lower than the frequency of identified SNVs. Therefore, baseline error rate of an NGS pipeline is critical for its performance of detecting ultra-rare SNVs. To further assess the baseline mutation frequency of this method, an updated normal exome reference database was created for the patient. With the updated reference exome, the error rate for barcoded single-stranded based NGS method was calculated to be $2.25 \times 10^{-10}$. This error rate is very close to the theoretical error frequency of $2.08 \times 10^{-10}$ and the method is sufficiently accurate to identify most ultra-rare mutations.

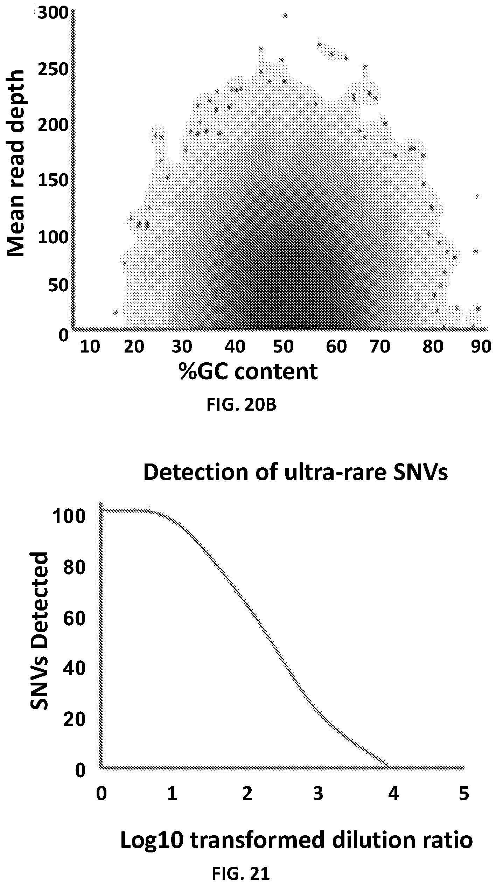

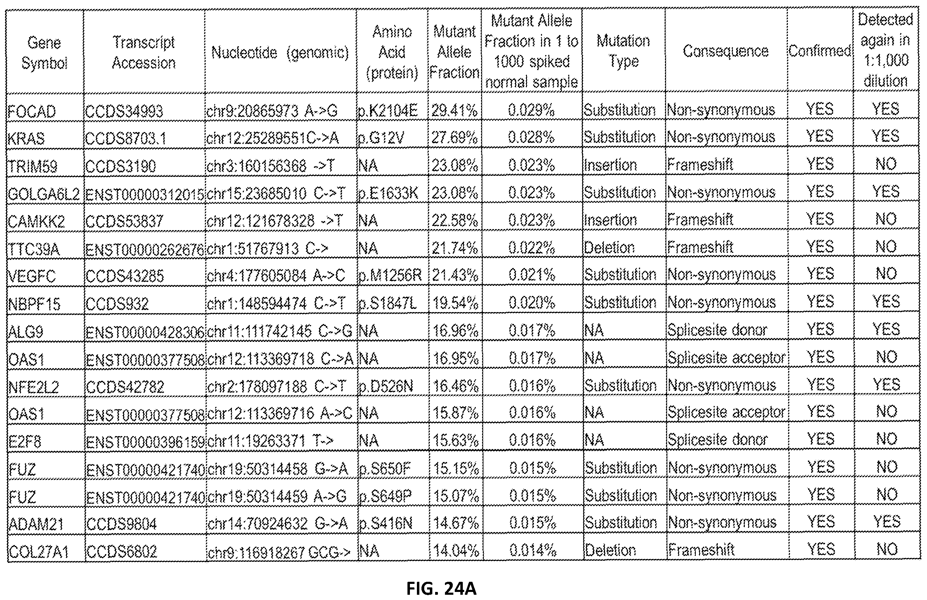

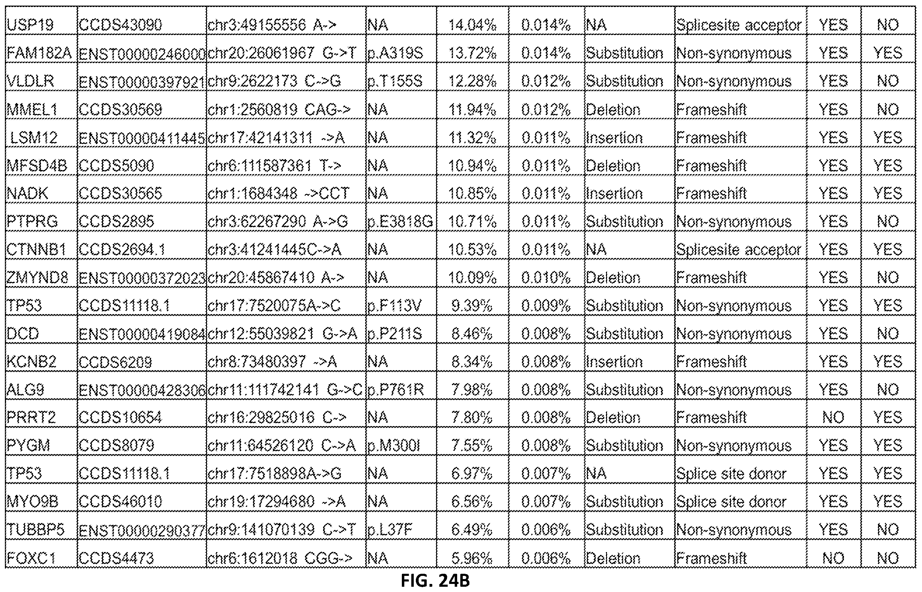

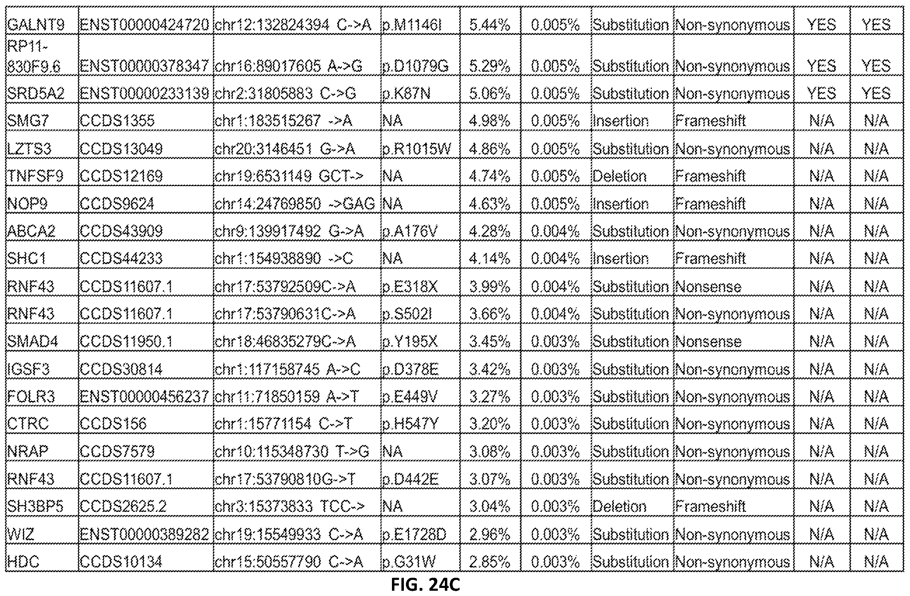

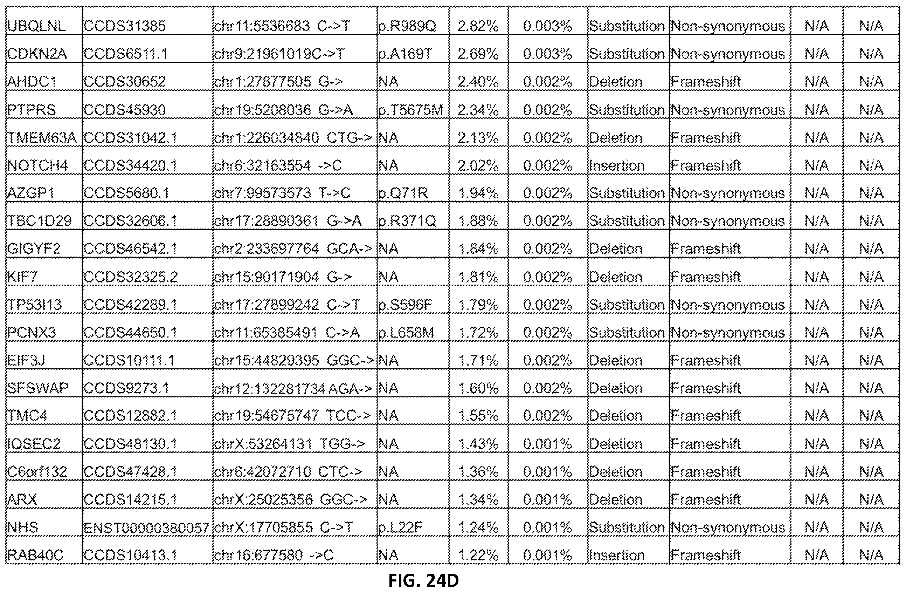

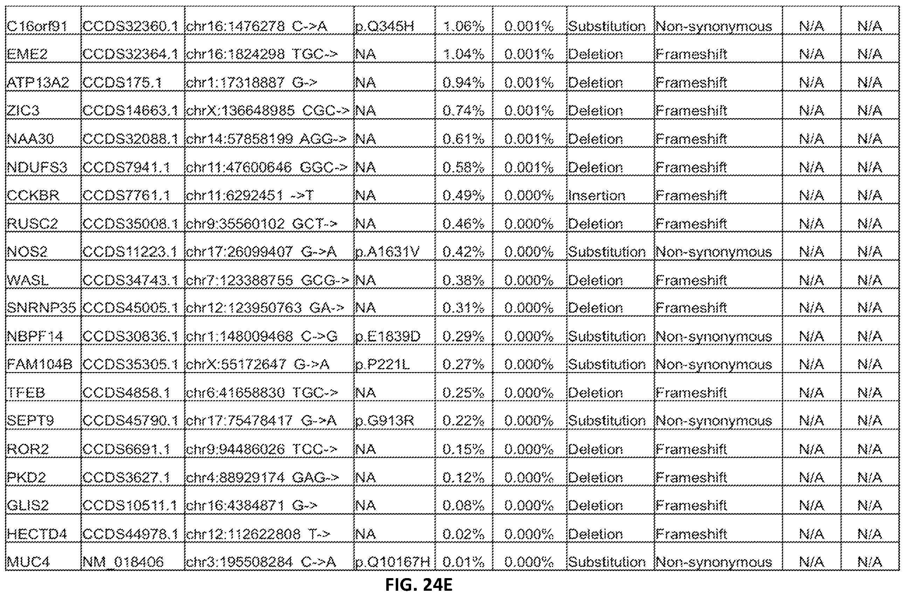

The ultra-rare mutation detection performance of this method was then evaluated by the success rate of re-detecting the 38 Sanger sequencing validated sequence variants in the libraries created from normal DNA samples which were spiked with sequential dilutions of tumor DNA. As the dilution folds increased, as expected, less and less variants were detected (FIG. 21), and when the tumor DNA sample was diluted 1,000 folds (the diluted sample containing 0.1ng tumor DNA and 100ng normal DNA), only 21 out of the 38 validated variants could be detected (FIGS. 24A-24E). The allelic fractions of these 21 SNVs in the 1:1000 diluted sample range from 0.03% to 0.005% with an average of 0.013% (FIGS. 24A-24E). None of the sequence variants in 1: 10,000 diluted sample was detected which may presumably due to the limitation of sequencing depth achieved. For each sample, a targeted sequencing was performed with an average depth of 5,000X, which theoretically only allows us to see SNVs down to the frequency of 1/5000 (0.02%). To observe ultra-rare SNVs with even lower frequency, a greater than 5000X coverage is needed. It is also helpfµl to design capturing probes targeting only a small number of genes. With a smaller number of sequencing targets, a standard barcoded single-stranded library based NGS can achieve a much greater sequencing depth with a significantly improved accuracy of ultra-rare SNV calling. The extremely low baseline error rate of the method allows ultra-rare SNV calling at the whole exome level with high accuracy, and the depth of NGS sequencing becomes the only limiting factor for such applications.

Barcoded single-stranded library construction can be used as an improved pipeline to perform NGS, particularly targeted NGS. Improved performance in a human genome WES study has been demonstrated. Aside from WES, another very important application of barcoded single-stranded library would be the targeted resequencing of a gene panel. Targeted re-sequencing is one of the most popular NGS applications and it allows people to sequence a small cohort of gene targets to extreme depths, usually thousands of folds of coverage. And such sequencing depth can facilitate the detection of ultra-rare mutations with great sensitivity. In a barcoded single-stranded library based WES study, the entire exome of all human genes was attempted to be captured, where an over 98% coverage with the depth of over 200X was achieved on a standard NGS platform. More importantly, this method's detection limit of rare-mutation detection on whole exome scale is as low as 0.03%. For an even smaller cohort of target genes, the depth and coverage of barcoded single-stranded library NGS can be further increased, and the performance of ultra-rare mutation detection can be subsequently improved over additional several orders of magnitude.

Other than identifying ultra-rare SNVs with high sensitivity and accuracy, barcoded single-stranded library construction method can also be adopted for gene copy number variant (CNV) assays. Barcoded single-stranded library construction links a unique barcode to every single-stranded DNA molecules. Such barcode information can not only be used to label the molecules and create super reads for the purpose of reducing PCR errors, but also be used as a location marker for DNA fragments. After mapping the super reads back to human genome, the barcode on each super read can be assigned to the position where the super read sequence is mapped. Therefore, a human genome can be reconstructed by unique barcodes. Copy number information can be represented by the diversity of barcodes at subgenomic loci. More importantly, in this method, unique barcodes are specific to DNA single strands. Such information can allow further normalization of the CNV data by taking into the consideration that genomic DNA exists as duplex molecules and the density of unique barcodes for both DNA strands should match. Such calculation can massively improve the accuracy of CNV calling.

Aside from CNV analysis, large structural variants frequently observed in cancer genomes can also be analyzed in our pipeline. NGS sequencing improved by high sensitivity and deep coverage of library construction will provide reads covering the breakpoints with higher confidence than standard pipeline, and targeted capturing probes can be designed to specifically enrich subgenomic regions flanking popular genome breakpoints. A highly sensitive pipeline for translocation and large indel identification could be built based on barcoded single-stranded library construction pipeline.

In addition to applications in basic research, barcoded single-stranded library construction has a great potential in clinical NGS fields. This method can highly efficiently construct NGS DNA libraries with very low amount of DNA materials ($\leq$ 20pg), meanwhile it can detect ultra-rare mutations with high confidence. Such features are critical for NGS based clinical diagnostics where the samples are often limited and highly heterogeneous. A typical example would be the NGS sequencing of FFPE samples. FFPE has been a standard sample preparation method for many decades. Historically archived FFPE sample is a very valuable resource for retrospective studies in biomedical research. However, due to chemical modifications during specimen preparation and chronic damages to the tissue blocks or slides over long-term storage, it has been a challenging task to conduct NGS studies with FFPE samples. Poor DNA quality and artificial sequence changes are two major issues coming along with FFPE based NGS studies. WES data have been reported to be discordant between FFPE and fresh frozen samples at lower coverage levels (~20X), however, this discrepancy can be reduced when higher coverages are achieved (Kerick, Isau et al. 2011). To ensure a high coverage in NGS sequencing, a sufficient number of original DNA molecules need to be incorporated into the library construction, and barcoded single-stranded library construction is a method meeting such a need.

This method has a great potential to discover novel low-frequency disease-causing variants in biomedical and clinical applications, and can identify more actionable therapeutic targets for patients. This method can fulfill an unprecedented level of personalized precision medicine by revealing the most complete patient genomic profile to date including highfrequency, low-frequency and particularly ultra-low-frequency mutations. This method can also be applied in other clinical applications, like circulating DNA sequencing from body fluid samples, where only limited amount of DNA materials is available. In clinical NGS applications, it is critical to construct NGS libraries from very limited amount of highly heterogeneous samples thus being less- or non-invasive; to highly efficiently enrich target sequences thereby reaching a great sequencing depth with limited cost and improved diagnostic sensitivity; and to remove artificial sequencing errors as completely as possible for the best diagnostic specificity. This method has been demonstrated to meet these needs with great potentials in numerous NGS applications.

EXAMPLE 1

MATERIALS AND METHODS

The paired tumor and normal tissue samples from a pancreatic cancer patient of Asian race were obtained in accordance with guidelines and regulations from Tianjin Medical University Cancer Institute & Hospital, P.R. China after Institutional Review Board (IRB) approval at Tianjin Medical University, and under full compliance with HIPAA guidelines. An informed consent for conducting this study was obtained from the patient. The tumor tissue sample has an estimated neoplastic content of 43.4%.

Library preparation: Genomic DNA from patient normal and tumor fresh frozen tissues were extracted using DNeasy Blood & Tissue Kit (Qiagen) and sheared into 150bp fragments with Diagenode's Bioruptor at a program of 7 cycles of 30 seconds ON/90 seconds OFF using 0.65 ml Bioruptor® Microtubes. Barcoded single-stranded library preparation starts from a complete dissociation of DNA duplex to form single-stranded DNA and tagging the 3' end of each DNA single strand individually with a unique digital barcode. Barcoded first adaptors were synthesized with a sequence as described above and illustrated in FIG. 8B. Pre-dephosphorylated fragmented DNA samples were mixed with barcoded first adaptor (final concentration 0.15uM), 20% PEG-8000, 100U CircLigase II, and incubated at 60°C for 1 hour. After immobilizing the ligation product on Streptavidin-coupled Dynabeads (ThermoFisher Scientific), each barcoded single-stranded DNA molecule is subject to an individual single-cycle PCR reaction to form its complementary strand. A DNA primer complimentary to the first adaptor was annealed and extended using Bst 3.0 polymerase at 50°C for 30 minutes. Blunt-end repair using T4 DNA polymerase was performed at 25°C for 15 minutes. A double-stranded adaptor was then ligated to the 5' end of the DNA duplex using T4 DNA ligase with an incubation at 16°C for 1 hour. The library is eluted from the beads by an incubation at 95°C for 1 minute. High fidelity PCR amplification is performed to amplify the DNA sequence as well as the unique barcode. Adaptor sequences are designed to be compatible with Illumina sequencing platforms. Barcoded single-stranded library construction procedure can be outlined in FIGS. 14A, 14B and 14C.

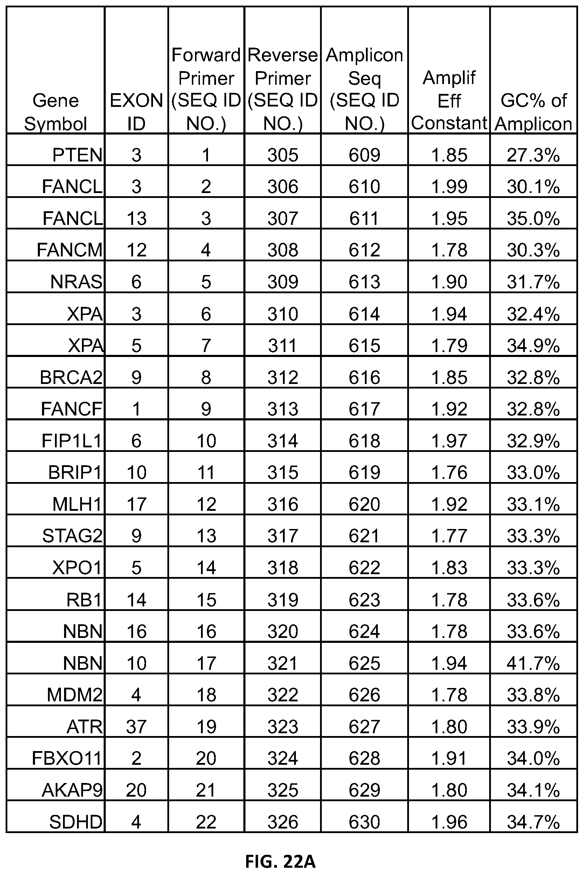

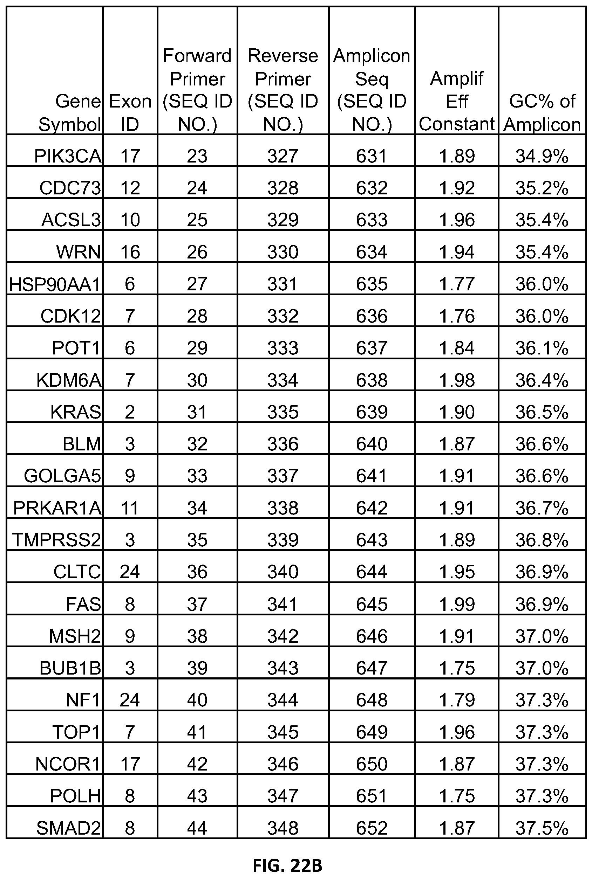

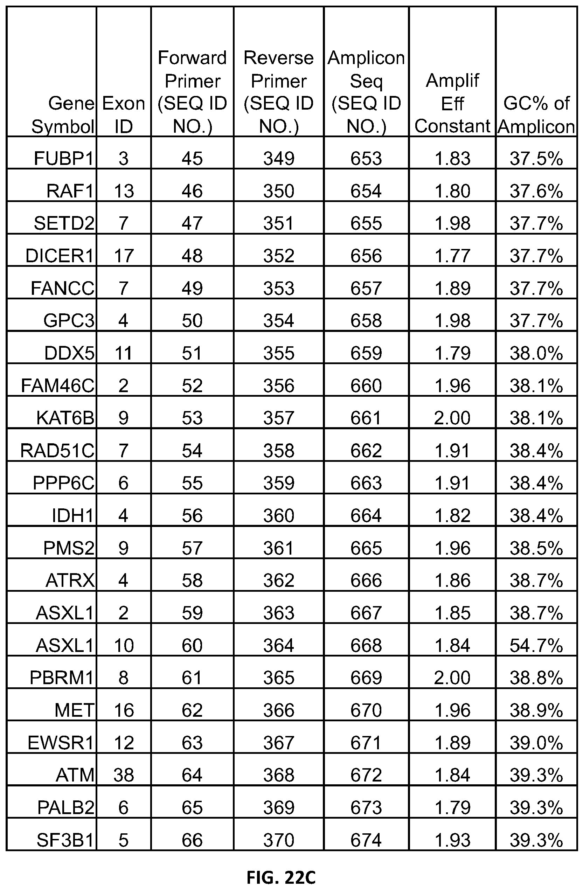

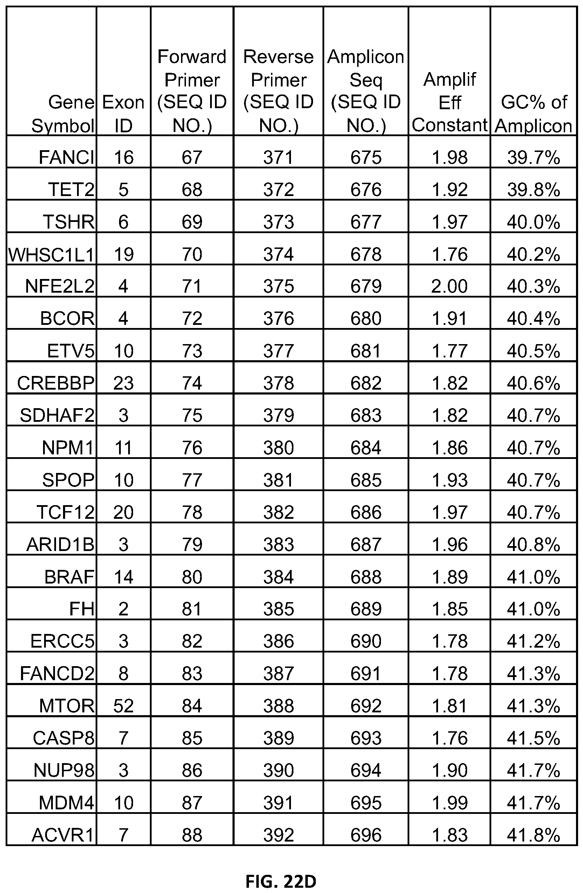

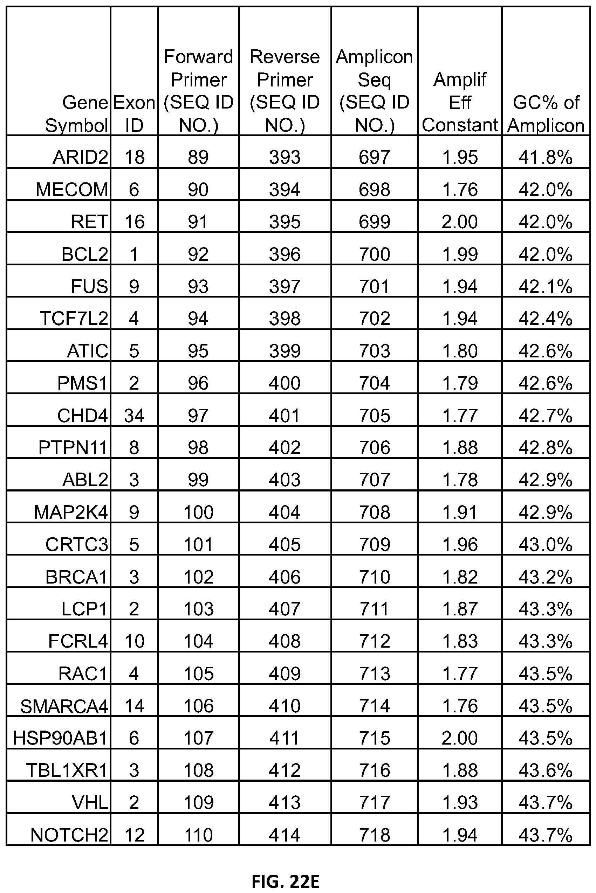

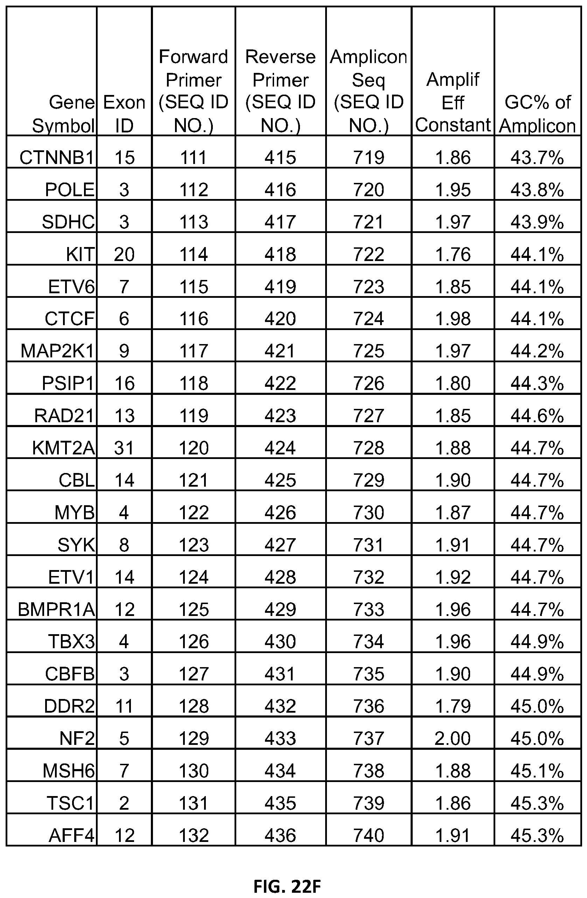

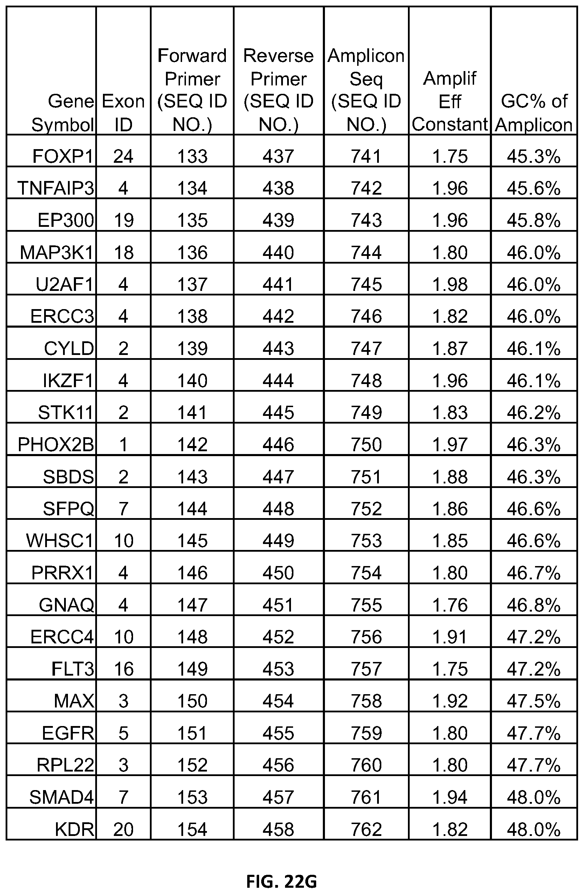

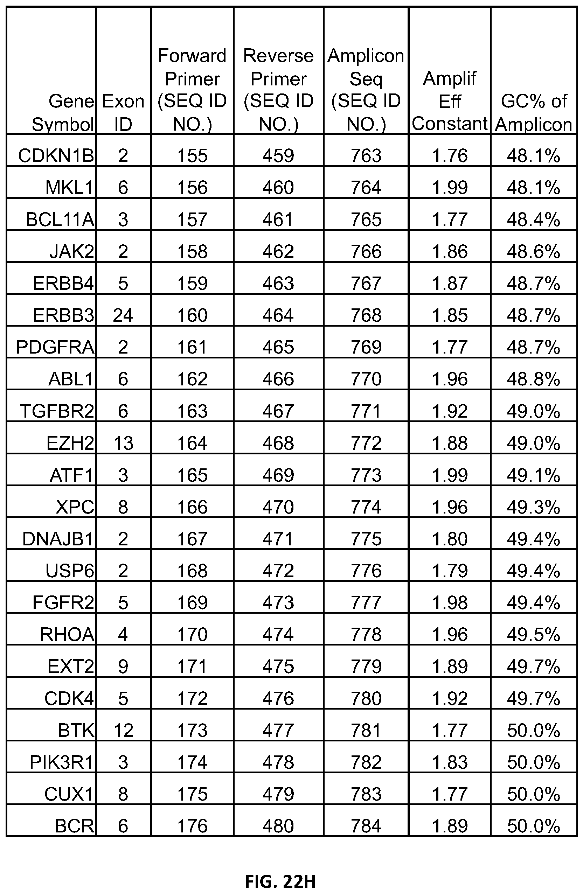

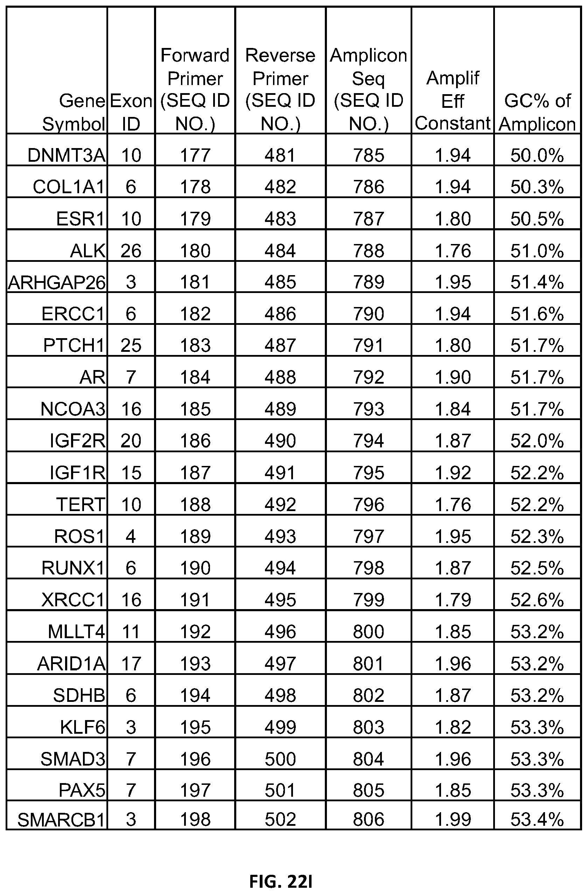

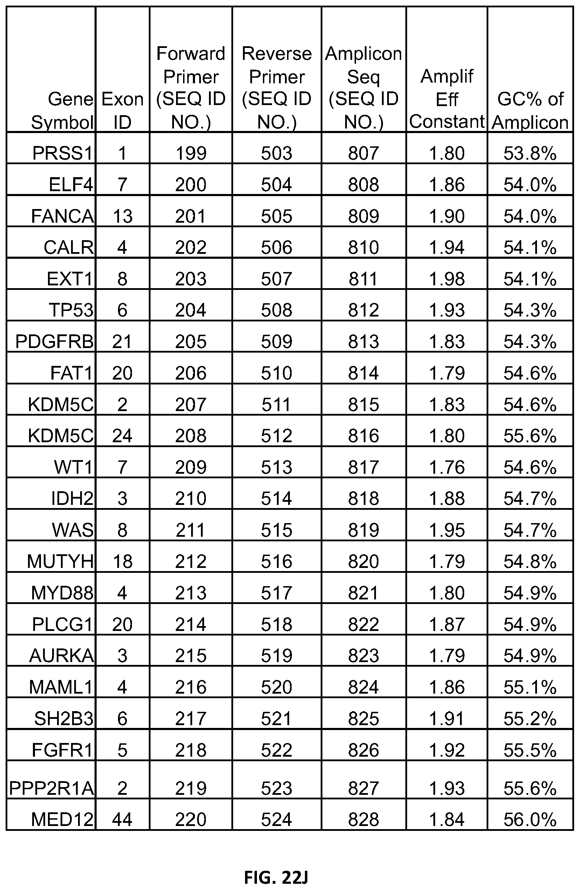

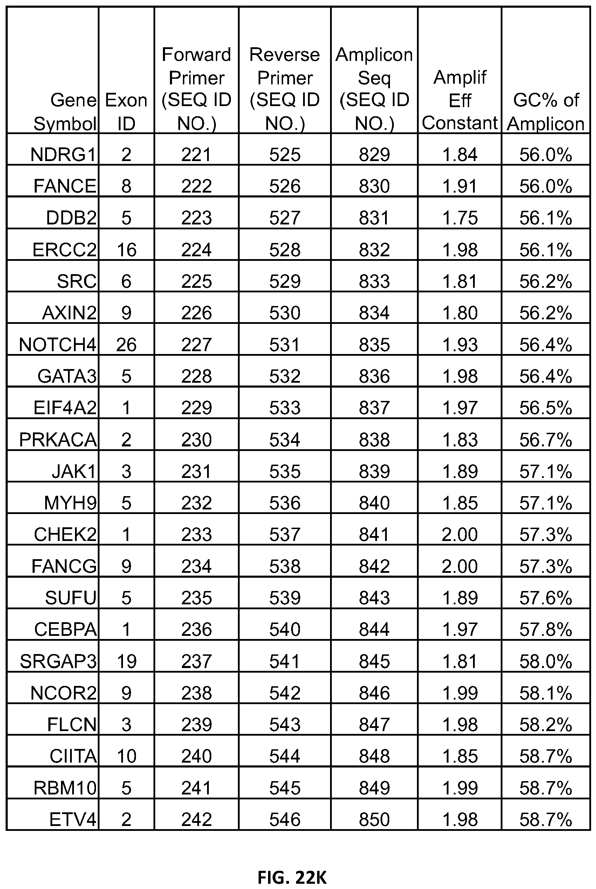

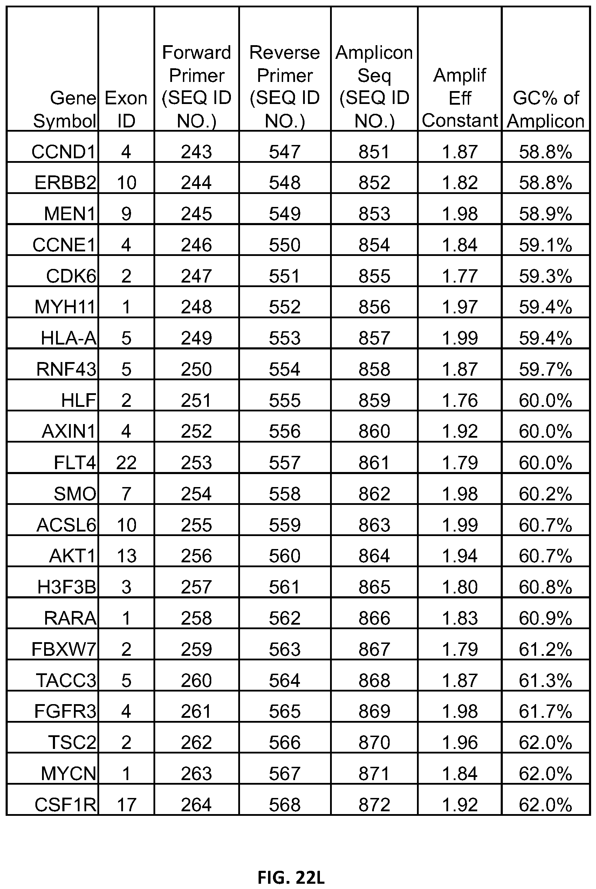

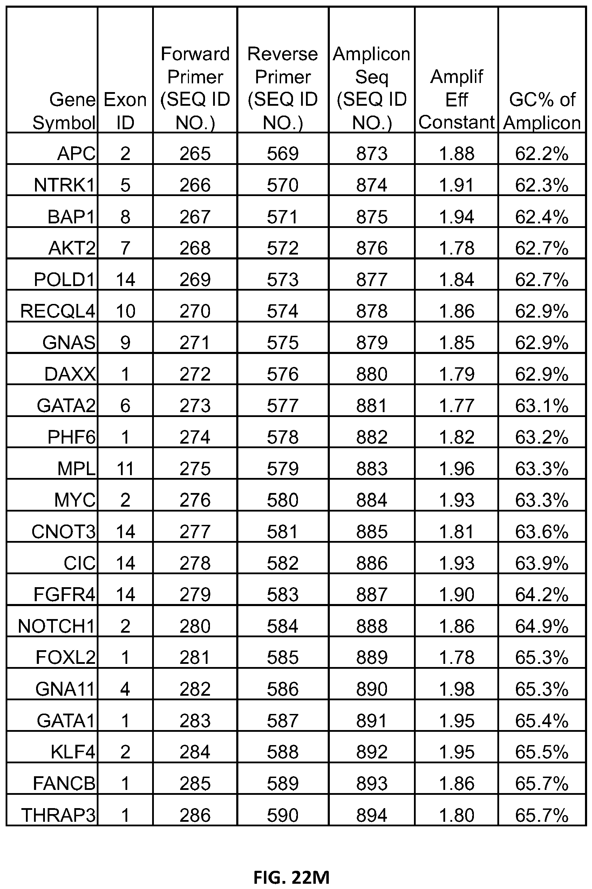

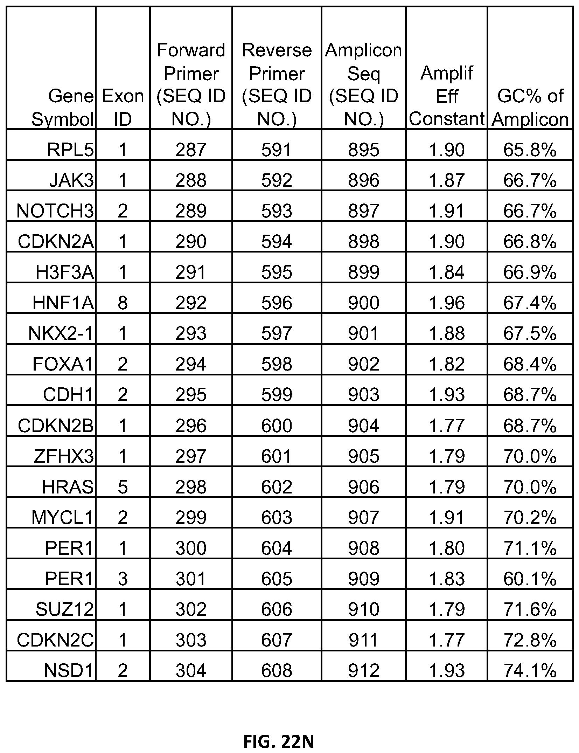

Real-time PCR assays with SYBR green detection was carried out using an ABI PRISM 7500 Sequence Detection System (Applied Biosystems). Briefly, the reaction conditions consisted of 500ng of genomic DNA or DNA library products, 0.2 $\mu$M primers, and SYBR Green Real-Time PCR Master Mix (ThermoFisher Scientific) in a final volume of 20$\mu$l. Each cycle consisted of denaturation at 95°C for 15 seconds, annealing at 58.5°C for 5 seconds and extension at 72°C for 20 seconds, respectively. Gene specific primers were designed using Primer 3 (Untergasser, Cutcutache et al. 2012) and their sequences are provided in FIGS. 22A-22N. Reactions were run in triplicate in three independent experiments. The primer pair's standard amplification curve for each gene was established through using sequential dilutions of the "+" clone constructs containing the amplicon sequence. Amplification efficiencies for 298 target amplicons were established and listed in FIGS. 22A-22N. Gene abundance ratios between different samples were calculated by the raising the gene specific amplification efficiency (AE) to the power of $\Delta C_t$ value between different samples. For example, the ratio (r) of gene abundance in sample A vs sample B can be calculated through real-time PCR assay by:

$$r_{(A/B)} = AE^{\Delta Ct},$$

where $\Delta C_t = C_{t\,(sample\,B)} - C_{t\,(sample\,A)}$

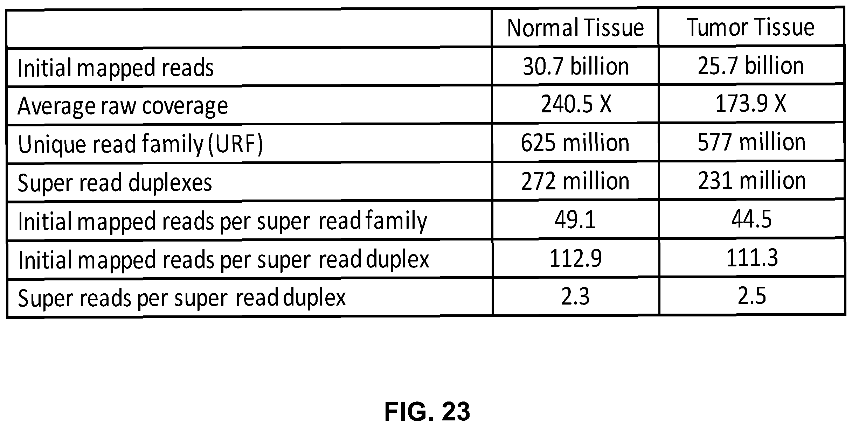

Whole exome sequencing was performed on an Illumina HiSeq 2500 platform according to manufacturer's manual. Total number of on-target reads from randomly chosen 5 million to 50 million reads were calculated. After trimming and barcoded super read grouping, SNVs were called with GATK (version 3.6) in a default mode as recommended by the GATK documentation with reference genome of Hg19 (McKenna, Hanna et al. 2010). In brief, for every sample (tumor or normal DNA), sequencing result was preprocessed by mapping to reference genome with BWA (version 0.7.10), and duplicates were marked with Picard (version 2.0.1). Base Recalibration was performed to generate the reads ready for SNV analysis. For individually processed T/N pair reads, Indel Realignment was performed to generate pairwise-processed T/N pair reads. HaplotypeCaller was used for raw SNV calling. Output from variant calling was directly used for SNV detection by MuTect (version 1) (Cibulskis, Lawrence et al. 2013). Mutations were filtered through a 4-step approach introduced in the section "Mutation and ultra-rare mutation detection". Low-quality variant with a Phred score <30.0 was abandoned. Paired SNVs from complementary reads bearing different barcodes were identified as true mutations and subject to further validation through Sanger sequencing. The data yields after each step of data analysis for a barcoded single-stranded library NGS study were shown in FIG. 23. SNVs identified and Sanger sequencing validation results were provided in FIGS. 24A-24E.

Mutation and ultra-rare mutation detection

The significantly increased number of unique reads obtained through barcoded single-stranded library approach enabled us to apply our stringent filters with the following 4-step procedure.

Step 1) group reads with the same barcode that are representing PCR duplicates of an original barcoded single-stranded DNA molecule, and call it a unique read family (URF);
Step 2) combine reads within each URF obtained from Step 1) by requesting >95% sequence identity among the reads;
Step 3) extract the unique DNA sequence and the barcode sequence for each URF, and call it a "super read";
Step 4) for all the super reads identified in Step 3), find their paired complementary super reads, and only score sequence variants with matched complementary sequences from paired super reads. To accommodate damaged DNA molecules in the sample, complementary super reads may not be at the same length (FIGS. 14A, 14B and 14C).

To evaluate the performance of barcoded single-stranded library in detecting low frequency (ultra-rare) mutations, 100ng tumor DNA sample was sequentially diluted by 10, 100, 1,000 and 10,000 folds, and spiked each of them into the same amount (100ng) of genomic DNA extracted from the paired normal tissue of the aforementioned cancer patient. This design can simulate early stages of cancer occurrence. The major obstacles in early cancer diagnostics using NGS include the very low allelic fractions of tumor specific mutations in the sample.

Build a highly accurate reference exome for ultra-rare mutation identification: To highly accurately assess the baseline mutation frequency of barcoded single-stranded library pipeline, six replicates of standard NGS DNA libraries were constructed in parallel, each using 100ng normal DNA input. These six replicates of exome datasets were used to re-build our own reference exome database for this particular patient by requesting that if the same SNV was observed in $\geq 5$ out of 6 independent datasets, the SNVs were considered as germline variants and updated our reference exome sequence database. For a standard NGS pipeline, the error rate is 1%, and the chance to see exactly the same random error at a fixed position for 5 times is $(\frac{1}{3} * 1\%)^5 = 4.12 \times 10^{-13}$. This number means that if this approach is used to sequence the whole human genome once, there is presumably going to be only one artificial error, because $3 \times 10^{12}$ human genome bases X $(4.12 \times 10^{-13}) = 1.24$. However, the human exome is being enriched and sequenced, which is occupying only 1.5% of human genome, therefore the chance to see a single artificial error within the entire human exome is only 1.86% (=1.5% $\times$ 1.24). An updated highly accurate normal exome reference database of the patient was built accordingly.

EXAMPLE 2

Barcoded Single-stranded Library Construction Creates Errorproof Libraries with ultra-low Quality and Quantity of DNA

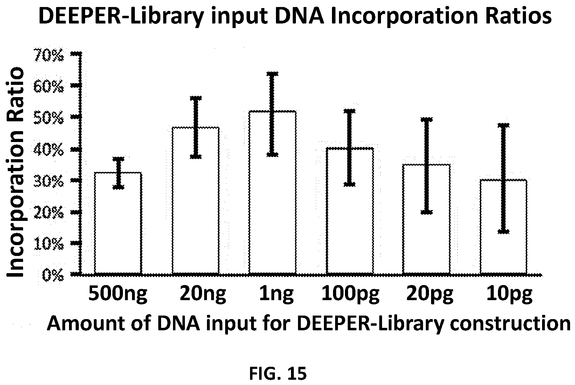

The library is prepared by a barcoded single strand library construction method. To assess the performance of such method in creating valid NGS libraries from limited amounts of DNA materials, 6 barcoded single-stranded libraries were constructed from sequentially diluted genomic DNA extracted (500ng, 20ng, 1ng, 100pg, 20pg and 10pg) from the normal pancreas tissue of a cancer patient. The first step of library construction is to ligate barcoded first adaptors to single strand DNA molecules, and this step is critical, since it provides the initial pool of DNA molecules for all downstream procedures. The average ligation efficiency for this step measured for 6 libraries were 32.3%, 46.5%, 52.1%, 40.3%, 35.1% and 30.5% (FIG. 15). These values indicated the incorporation ratios of different amounts of genomic DNA molecules into the library construction workflow. This ratio is essential for successf$\mu$l NGS applications with very limited starting materials and very heterogeneous samples. The ligation proved very efficient that it utilized over 50% of 1ng genomic DNA molecules, and this ratio remained above 30% with as low as 10pg genomic DNA input. Six library constructions were performed and 500ng library products from each of the 6 libraries were used for further performance evaluation.

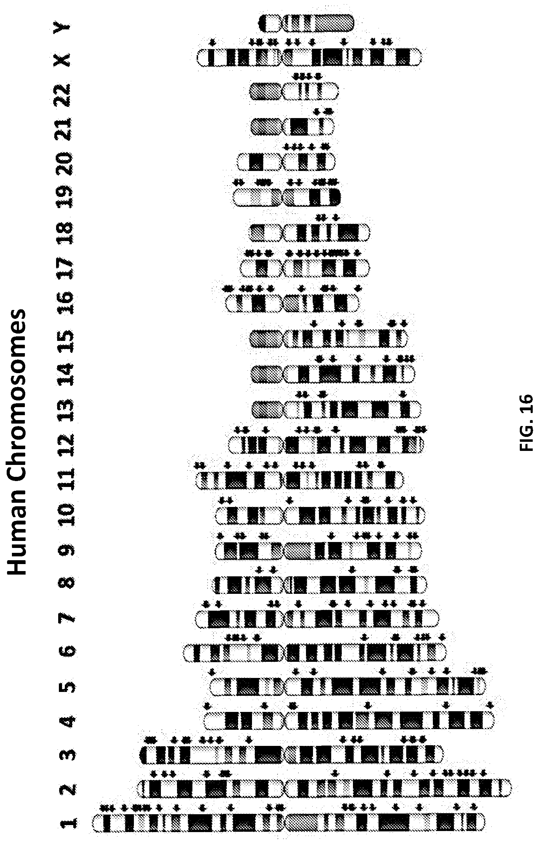

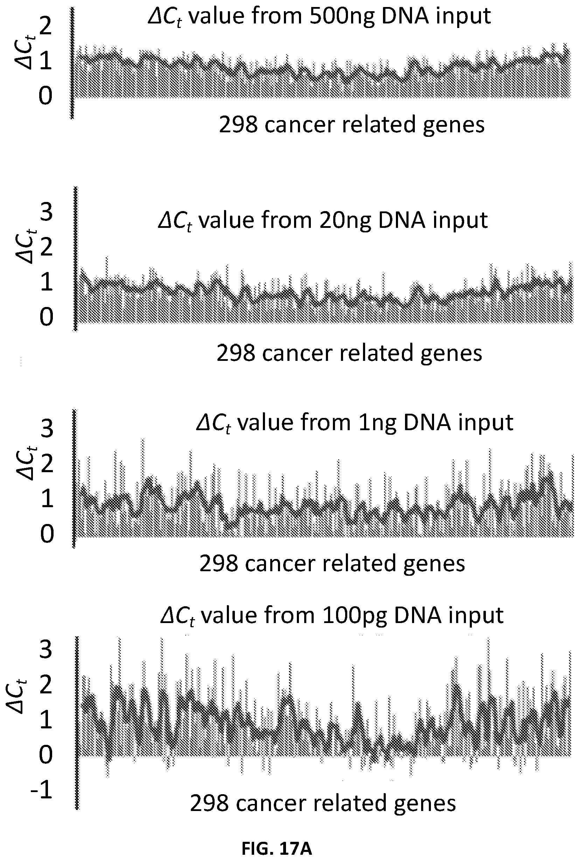

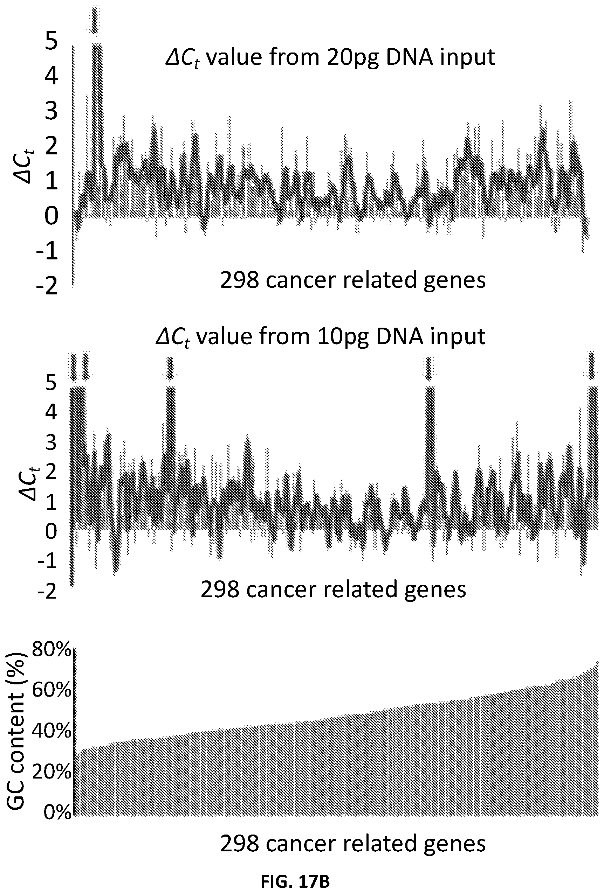

298 human cancer related genes located on chromosome 1 through 22 and chromosome X (FIG. 16) were selected as genome landmarks to indicate the broadness and depth of coverage of library as well as the enrichment efficiency and evenness of subgenomic regions by targeted capture, measured by real-time PCR assays. Gene-specific primer pairs were designed and used to amplify the 298-gene panel (FIGS. 22A-22N). Seven real-time PCR reactions, with three replicates for each reaction, were performed for each gene using 500ng genomic DNA and 500ng library product from each of the six libraries created from different amounts of input DNA. After taking the average of triplicates, six $\Delta C_t$ values between initial DNA input and six library products were calculated for each gene, and were subsequently plotted to compare across 298 genes for their abundances before and after the library constructions with different amount of starting materials (FIGS. 17A and 17B). Amplification efficiencies for 298 target amplicons were established and listed in FIGS. 22A-22N with an average value of 1.88. The average size of sheared DNA single strands is 150bp, and the total length of adaptor sequences added to the sequence during library preparation is 135nt (FIGS. 14A, 14B and 14C). Therefore, the distribution of $\Delta C_t$ between 500ng final library products and 500ng initial DNA input fragments should be presumably centered at $\log_{1.88}[(135+150)/150] = 1.017$, which was in consistency with the data observed (FIGS. 17A and 17B). All target genes were detected in 500ng original genomics DNA input, and in four (500ng, 20ng, 1ng and 100pg DNA inputs) out of six libraries. Only one and five genes were not detected from the libraries constructed with 20pg or 10pg DNA, respectively (FIGS. 17A and 17B). There is no significant GC% dependent abundance bias observed from $\Delta Ct$ values for all genes. More importantly, despite the different amounts of DNA materials to start with, the library constructed using barcoded single-stranded library construction method evenly amplified the entire human genome landmarked by the panel of 298 genes. PCR primers were re-designed to target a different genomic region for each of the six genes that were not detected in the two most diluted DNA samples (20pg and 10pg), and re-performed the same set of seven real-time PCR assays for each gene. Positive results were observed using new primers (FIGS. 22A-22N).

Our results demonstrate that barcoded single-stranded library construction method is able to create DNA library from very low DNA material amount (10~20pg) and generate NGS feasible library products (>1ug) with high broadness of coverage. The library has no obvious GC content bias and library molecules are evenly amplified to represent original input DNA's genome sequence abundance. These results also indicate that it becomes less efficient to amplify certain subgenomic regions when DNA input amount is extremely limited, i.e. around or lower than 20pg. To construct DNA libraries with extremely low amount of DNA, a whole genome pre-amplification may be necessary. However, such procedure may generate artificial errors before the initial barcoding step in library construction, and can hinder its rare mutation detectability. Therefore, no further test with any lower amount of DNA materials for library construction, and the minimal input limit for a successfµl library construction was noted as 20pg DNA. This amount (20pg) contains the total DNA materials from less than 3 human somatic cells. The vast majority of biological samples will be more than enough to offer such abundance level of DNA materials, and our library construction method has demonstrated an excellent performance in creating NGS libraries with this low amount of DNA.

EXAMPLE 3

Whole Exome Sequencing

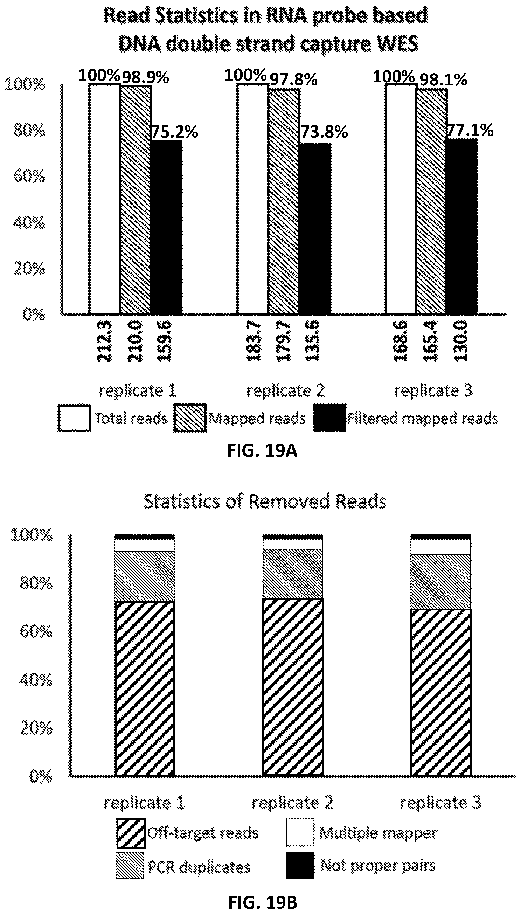

To evaluate the performance of barcoded single-stranded library construction in NGS, WES assays were performed using this method and compared the data to what obtained through standard NGS library preparation with a standard exome enrichment procedure. All libraries were constructed with 100ng genomic DNA derived from the normal tissue of the cancer patient and 3 technical replicates were performed for each sample. All NGS runs were carried out on the same Illumina HiSeq 2500 platform with the same technical specifications of the runs. As shown in FIG. 19A, an average of 188 million reads were obtained from barcoded single-stranded library construction derived WES, where 98.3% were aligned to human genome, and the total read counts were significantly more (1.6 folds) than that from the standard sequencing pipeline. The higher numbers of reads for barcoded single-stranded libraries presumably came from the ultra-sensitive single-stranded DNA library construction, and the much more efficient enrichment designed to capture both DNA strands (including DNA molecules that have damages ranging from minor single strand breaks to major damages on both strands).

All NGS data were analyzed on the same software pipeline with the same settings. Raw reads were filtered to remove duplicates, multiple mappers, improper pairs, and off-target reads. On average 75.4% reads were retained after filtering (FIG. 19A). For the reads that were removed, 71.8% were off-target reads, which were mapped to the human genome but outside of the target regions; 21.6% were PCR duplicates; and the remaining reads were mapped to multiple sites of the genome or not mapped at all (FIG. 19B). No statistically significant difference was observed in all the specifications measures for the three technical replicates in this experiment, which indicates that barcoded single-stranded library construction pipeline is technically highly reproducible (FIGS. 19A and 19B).

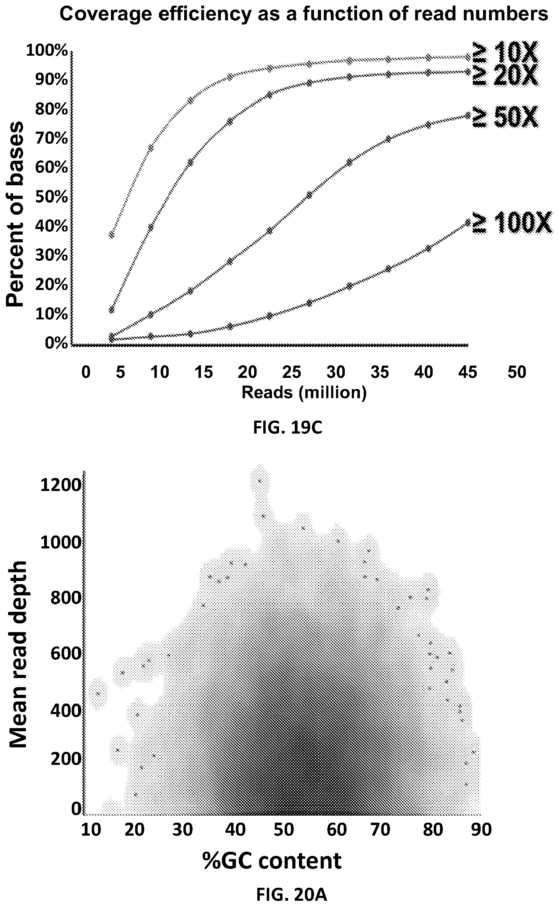

Next, the correlation between coverage efficiency and sequencing depth in barcoded single-stranded library was evaluated. Filtered reads were randomly selected in 5 million read increments from 5 million to 50 million. The fractions of the retained on-target reads covering the depths of at least 10X, 20X, 50X, and 100X were plotted using randomly selected 5 to 50 million reads (FIG. 19C). 20 million reads could cover close to 90% of the target bases with no less than 10X depth. With 50million reads, over 90% target bases were covered by at least 20X. The efficiency of coverage is not only dependent on the efficiency of barcoded single-stranded library construction, but also dependent on the length of the sheared molecules that were initially incorporated into the pipeline. For the current study, the average length of sheared DNA molecule is 150bp. Our real-time PCR results for the 298-gene panel indicated that enrichment efficiency of the barcoded single-stranded library construction approach is not significantly biased by GC content (FIGS. 17A and 17B).

To assess the impact of GC content on barcoded single-stranded library WES result, normalized mean read depth was plotted against GC content. There is a correlation between GC content and read depth in the barcoded single-stranded library WES experiment (FIG. 20A), and this bias is reduced in a WGS study using the same barcoded single-stranded library (FIG. 20B). In barcoded single-stranded library sequencing, the mean read depth ratios of GC50%/GC20% =1.55, which demonstrates a low GC bias in this method.

EXAMPLE 4

Detection of SNVs

One of the most important goals of exome sequencing is to identify sequence variants that are disease-causing or of clinical significance. To evaluate the sensitivity and specificity of sequence variant identification performance of barcoded single-stranded library construction, a WES study was conducted with 100ng genomic DNA from a pair of normal and tumor tissue samples obtained from the same cancer patient. The same SNV calling pipeline was used for all data analysis in this study. Briefly, the normal DNA libraries created by barcoded single-stranded library construction method was sequenced and the data was analyzed using a standard data analysis pipeline, where the single-stranded barcodes were directly trimmed off, and 78,721 SNVs were detected from the exonic sequences of normal DNA sample at a read count of 30 million (error frequency 2.6 × $10^{-3}$, FIG. 18A). Next, we investigated if there is any bias in SNVs identified in barcoded single-stranded library using the standard NGS data analysis workflow. Transition-transversion (ts/tv) ratio is routinely used to evaluate the specificity of new SNP calls. The ts/tv ratio on the target regions of barcoded single-stranded library-based WES was calculated to be 2.766. Then the ts/tv ratio was determined in CCDS exonic regions as 3.225, which falls into the range of 3.0-3.3 for exonic variations.

The accuracy of mutations identified by barcoded single-stranded library based mutation calling was then examined. Following the 4-step data analysis procedure introduced in Materials and Methods, super reads were generated after Step 3). Steps 1~3 helped to reduce the mutation frequency by over 2 orders of magnitude from $2.6 \times 10^{-3}$ down to $2.5 \times 10^{-5}$ by removing most PCR related errors (FIG. 18B). This result indicates that PCR related artificial mutations dramatically reduce NGS sequencing accuracy. To detect rare mutations, or even ultra-rare mutations using NGS, a correction for PCR errors is mandatory. As outlined in Step 4), we then tried to further reduce artificial errors of mutation calling by using the redundant sequence information offered by complementary DNA strands that were originally from the same DNA duplex molecule. Our results indicated that such procedure resulted in a single base mutation frequency of $1.6 \times 10^{-6}$ (FIG. 18B). For any single base in the DNA sequences, the possibility of having exactly the same artificial error on a paired position is $\frac{1}{3} \times (2.5 \times 10^{-5})^2 = 2.08 \times 10^{-10}$, which is equivalent to one artificial error per $4.8 \times 10^9$ nucleotides. This is the theoretical error rate for barcoded single-stranded library NGS. The total amount of DNA sequence data and the remaining amount of data after each step can be found in FIG. 23, where a stepwise drop of data amount is correlated to the increase of mutation calling stringency.

To determine the accuracy of variant detection by barcoded single-stranded library construction for clinically relevant mutations, the WES data generated from the normal and tumor tissue pair were analyzed side-by-side. For all assessed heterozygous exonic positions, the result was filtered through a 4-step procedure. The filtered result showed that for barcoded single-stranded library based WES study identified 97 sequence variants that were exclusively detected in tumor tissue DNA sample with $\geq$ 100X coverage at different fractions. 40 moderate-to high-abundance (> 5%) variants were subject to Sanger sequencing validation, and 38 were confirmed (FIGS. 24A-24E). Two variants failed to be validated where both allelic fractions were low and beyond the detection limit of Sanger sequencing. 57 sequence variants (with mutant allele fractions < 5%) were not subject to Sanger sequencing validation at all, due to the limited sensitivity of Sanger sequencing (Tsiatis, Norris-Kirby et al. 2010).

EXAMPLE 5

A protocol for barcoded single-stranded library preparation
Fragmentation of genomic DNA into 250bp by BioRuptor

Turn on BioRuptor and water bath (set to 3°C) at least 45 minutes before starting.
Place up to 1 µg of DNA adjusted to 57 µl with 1 ×TE buffer in a BioRuptor microtube.
Shear with below setting for a target size range of 175 bp:

| Setting | value |
| --- | --- |
| Intensity | H |

| On:Off | 30 seconds:90 seconds |
| --- | --- |
| Cycles | 7 |

Remove the large genomic DNA fragments through binding with 0.6×AMPure beads.
Transfer the supernatant into a new tube and then purify with 0.8×AMPure beads. Elute into 30µl 1×TE buffer.
Heat denaturation and first adaptor ligation.
DNA in ddH2O to a volume of 33µL in lo-bind tube.
Add 8 µl CircLigase II 10x reaction buffer.
Add 4 µl 50 mM MnCl2.
Add 1 µl (1 U) FastAP.

Incubate at 37°C for 10 minutes then 95°C for 2 minutes in Eppendorf thermomixer (thermal cycler with a heated lid in paper)

Place reaction tube into an ice-water bath.

Add 32 µl 50% PEG-8000
Add 1 µl 10 µM of the first adaptor as illustrated in FIG. 8B
Vortex intensely to mix
Add 1 µl CircLigase II (Epicentre)
Vortex intensely to mix
Incubate at 60°C for 3 hour in a thermal cycler then hold at 4°C.
Add 2 µl stop solution (98µl 0.5M EDTA (PH8.0), 2µl Tween-20)
Freeze overnight
Immobilization of ligation products on streptavidin beads
Wash 20 µl of MyOne C1 beads twice with 500µl bead-binding buffer (1 M NaCl, 10 mM Tris-HCl pH 8.0, 1 mM EDTA, 0.05% Tween-20, 0.5% SDS).

Re-suspended in 250 µl bead-binding buffer and transfer to a 1.5ml-siliconized tube (Sigma-Aldrich).

Thaw reaction mix.
Incubate reaction mix at 95°C for 2 minutes

Chill reaction mix in ice water bath.
Add reaction mix to beads and pipette up and down 10 times.
Rotate tube at room temp for 20 minutes.
Remove supernatant.

Wash beads with 200µl of wash buffer A (100 mM NaCl, 10 mM Tris-HCl pH 8.0, 1 mM EDTA, 0.05% Tween-20, 0.5% SDS) and once with 200 µl wash buffer B (100 mM NaCl, 10 mM Tris-HCl pH 8.0, 1 mM EDTA, 0.05% Tween).
Primer annealing and extension
Remove supernatant.
Re-suspend beads in 47 µl reaction mixture:

>40.5 µl water
>5 µl 10x Thermopol buffer (New England Biolabs)
>0.5 µl 25 mM each dNTP (Fermentas)
>1 µl 100 µM extension primer GTGACTGGAGTTCAGACGTGTGCTCTTGCTGAGG (i.e. SEQ ID NO: 916)

Incubate at 65 °C for 2 min.

>Immediately chill in ice-water bath
>Transfer to thermocycler pre-cooled to 15°C
>While in thermocycler add 3 µl (24 U) Bst 3.0 DNA polymerase (New England Biolabs)

Incubate reaction at 15°C for 5mins, then slowly increase the reaction temperature to 37°C at a rate of no more than 1°C per min, and then hold the reaction at 37°C for 3 min.
Mix gently every five minutes to keep beads in suspension.
Discard supernatant.
Wash beads with wash buffer A.
Beads were resuspended in 200 µl stringency wash buffer (0.1x SSC buffer (Sigma-Aldrich), 0.1% SDS).
Incubate at 45°C for 3 min in thermal mixer.
Wash beads with 200 µl wash buffer B.
Removal of 3'-overhangs
Re-suspend beads in 99 µl of a reaction mix containing:

>86.1 µl water
>10 µl 10x Tango buffer (Fermentas)
>2.5 µl 1% Tween-20
>0.4 µl 25 mM each dNTP

Add 1 µl (5 U) T4 DNA polymerase (Fermentas).
Incubate for 15 min at 25°C in a thermal cycler.
Gently mix every five minutes to keep beads suspended.
Add 10µl of EDTA (0.5M) to reaction mixture and vortex.
Wash beads with wash buffer A, stringency wash buffer with 45°C incubation for 3mins and then wash buffer B as described above.

Prepare double-stranded adaptor for ligation

A 100 µM solution of double-stranded DNA adaptor was generated by hybridizing two oligonucleotides (double-stranded adaptor oligo 1 and double-stranded adaptor oligo 2, sequence shown below) as follows: In a PCR reaction tube, 20 µl 500 µM DEEPER DS adaptor oligo 1, 20 µl 500 µM DEEPER DS adaptor oligo 1, 9.5 µl TE buffer and 0.5 µl 5 M NaCl were combined.

>Double-stranded adaptor oligo 1
>CGACCCTCAGCC-ddC (SEQ ID NO: 917, where ddC = dideoxy cytidine)
>Double-stranded adaptor oligo 2
>Phosphate-GGCTGAGGGTCGTGTAGGGAAAGAG*T*G*T*A (SEQ ID NO: 918, where* = PTO bonds)

This mixture was incubated for 10 seconds at 95°C in a thermal cycler and cooled to 14°C at a speed of 0.1°C/s. Final concentration of 100 µM was reached by dilution with 50 µl TE.
Blunt-end ligation of second adaptor and library elution
Re-suspend beads in 98 µl of a reaction mix containing:

>73.5 µl water
>10 µl 10x T4 DNA ligase buffer (Fermentas)
>10 µl 50% PEG-4000 (Fermentas)
>2.5 µl 1% Tween-20
>2 µl 100 µM adaptor CL53/73

Mix thoroughly and add 2 µl (10 U) T4 DNA ligase (Fermentas).
Incubate for 1 hour at 25°C in a thermal mixer.
Gently mix every twenty minutes to keep beads suspended.
Wash beads with 0.1×BWT+SDS (wash buffer A), stringency wash and 0.1xBWT (wash buffer B) as described above.
Re-suspend beads in 25 µl elution buffer (10 mM Tris-HCl pH 8.0, 0.05% Tween-20) and transferred to single-cap PCR tubes.
Incubate for 5 min at 95°C in a thermal cycler with heated lid.
Collect supernatant in fresh tube.
Library amplification
Take 1 µl ligated DNA for test PCR reaction: Prepare a master mix by multiplying the amount in column "per reaction" by the number of reactions plus one. Add in order the following:

| component | volume per reaction (µl) |
|---|---|
| Water | 34 |

(continued)

| component | volume per reaction (μl) |
|---|---|
| DMSO | 2.5 |
| 5X Phusion Buffer | 10 |
| 10mM dNTPs | 1 |
| Index PE primer II | 0.25 |
| PE primer I | 0.25 |
| HotStart Phusion | 1 |
| Total | 49 |

MIX well

add 1μl DNA

MIX well

Amplification conditions:

　　1 minute at 98 °C

　　10~14 cycles of:

　　　　20 seconds at 98°C

　　　　30 seconds at 60 °C

　　　　30 seconds at 72°C

　　5 minutes at 72 °C

　　Hold at 4 °C

PCR primer sequences:

　　PE primer I: AATGATACGGCGACCACCGAGATCTACACTCTTTCCCTACACGACGC TCTT (SEQ ID NO: 919)

　　Index PE primer II: CAAGCAGAAGACGGCATACGAGAT-7 mer Index-GTGACTGGAGTT CAGACGTGT (SEQ ID NO: 920)

The PCR is performed in two wells for each sample, 50 μl each. Then the amplified PCR product was purified using AMPure beads with ratio 1:1 (beads:sample), elute in 30μl 1×TE buffer.

Use Qubit to quantify yield. You will have ~150ng /μl in general.

REFERENCE

Cibulskis, K., et al. (2013). "Sensitive detection of somatic point mutations in impure and heterogeneous cancer samples." Nat Biotechnol 31(3): 213-219.

Kerick, M., et al. (2011). "Targeted high throughput sequencing in clinical cancer settings: formaldehyde fixed-paraffin embedded (FFPE) tumor tissues, input amount and tumor heterogeneity." BMC Med Genomics 4: 68.

McKenna, A., et al. (2010). "The Genome Analysis Toolkit: a MapReduce framework for analyzing next-generation DNA sequencing data." Genome Res 20(9): 1297-1303.

Tsiatis, A. C., et al. (2010). "Comparison of Sanger sequencing, pyrosequencing, and melting curve analysis for the detection of KRAS mutations: diagnostic and clinical implications." J Mol Diagn 12(4): 425-432.

Untergasser, A., et al. (2012). "Primer3--new capabilities and interfaces." Nucleic Acids Res 40(15): e115.

**Claims**

1. A method for constructing a DNA library from a biological sample containing a plurality of nucleic acid sequences, comprising:

　　preparing a DNA sample from the biological sample, wherein the DNA sample comprises a plurality of single-stranded DNA molecules, each having a dephosphorylated 5' end;
　　ligating a first strand of a first adaptor to a 3' end of each of the plurality of single-stranded DNA molecules, wherein the first strand of the first adaptor comprises a phosphate group, a barcode sequence and a first primer recognition sequence along a direction from a 5' end thereof to a 3' end thereof; and
　　synthesizing a complementary strand for each of the plurality of single-stranded DNA molecules ligated to the first strand of the first adaptor to obtain a barcoded double-stranded DNA molecule corresponding thereto,
　　wherein the first strand of the first adaptor further comprises an immobilization portion at the 3' end thereof, configured to be able to form a stable coupling to a solid support, wherein:

　　　　the method further comprises, between the ligating a first strand of a first adaptor to a 3' end of each of the plurality of single-stranded DNA molecules and the synthesizing a complementary strand for each of the plurality of single-stranded DNA molecules ligated to the first strand of the first adaptor to obtain a barcoded double-stranded DNA molecule corresponding thereto:

immobilizing each of the plurality of single-stranded DNA molecules ligated to the first strand of the first adaptor to the solid support via the stable coupling between the immobilization portion and the solid support;

the method further comprises, after the synthesizing a complementary strand for each of the plurality of single-stranded DNA molecules ligated to the first strand of the first adaptor to obtain a barcoded double-stranded DNA molecule corresponding thereto:

ligating a second adaptor to a free end of the double-stranded DNA molecule corresponding to the each of the plurality of single-stranded DNA molecules immobilized to the solid support at an immobilized end thereof, wherein the second adaptor comprises a third strand and a fourth strand, wherein:

the fourth strand comprises:

a second primer recognition sequence, configured to provide a priming site for amplification of the double-stranded DNA molecule corresponding to the each of the plurality of single-stranded DNA molecules; and
a phosphate group at a 5' end thereof;
and

the third strand comprises a sequence complementary to a 5'-end sequence of the fourth strand, and is configured to form a duplex with, and thereby to ensure a stability of, the 5'-end sequence of the fourth strand.

2. The method according to Claim 1, wherein the plurality of nucleic acid sequences in the biological sample comprise a plurality of DNA sequences, and the preparing a DNA sample from the biological sample comprises:

performing dephosphorylation reaction and dissociation reaction to obtain a plurality of single-stranded DNA molecules, each having a dephosphorylated 5' end.

3. The method according to Claim 1, wherein the first adaptor comprises a single-stranded segment at the 5' end of the first strand thereof, and the ligating a first strand of a first adaptor to a 3' end of each of the plurality of single-stranded DNA molecules comprises:

performing a ligation reaction through a single-stranded DNA ligase such that the 3' end of each of the plurality of single-stranded DNA molecules is ligated to the 5' end of the first strand of the first adaptor.

4. The method according to Claim 1, wherein the synthesizing a complementary strand for each of the plurality of single-stranded DNA molecules ligated to the first strand of the first adaptor to obtain a barcoded double-stranded DNA molecule corresponding thereto comprises:

annealing a first primer with each of the plurality of single-stranded DNA molecules ligated to the first strand of the first adaptor, wherein the first primer comprises a sequence complementary to the first primer recognition sequence in the first strand of the first adaptor; and
performing a single-strand extension reaction to form a double-stranded DNA molecule for each of the plurality of single-stranded DNA molecules ligated to the first strand of the first adaptor.

5. A kit for constructing a DNA library from a biological sample containing a plurality of nucleic acid sequences, comprising:

a first adaptor, having a first strand comprising, in a direction from a 5' end thereof to a 3' end thereof, a phosphate group, a barcode sequence, and a first primer recognition sequence, wherein the barcode sequence is configured to provide barcode information to each of the plurality of single-stranded DNA molecules ligated to the first strand of the first adaptor;
a DNA ligase, configured to allow a ligation between the 5' end of the first strand of the first adaptor to a 3' end of each of a plurality of single-stranded DNA molecules, wherein each of the plurality of single-stranded DNA molecules corresponds to one of the plurality of nucleic acid sequences in the biological sample; and
a first primer, comprising a sequence complementary to the first primer recognition sequence of the first adaptor and configured to allow for a single-strand extension reaction to thereby form a double-stranded DNA molecule corresponding to each of the plurality of single-stranded DNA molecules ligated to the first strand of the first adaptor,
a solid support, wherein the first strand of the first adaptor further comprises an immobilization portion at the 3' end thereof, configured to allow immobilization of each of the plurality of single-stranded DNA molecules ligated to the first strand of the first adaptor at the 5' end thereof to the solid support, wherein the immobilization portion comprises a first coupling partner, configured to be able to form a stable coupling with a second coupling partner attached to the solid support, wherein the stable coupling between the first coupling partner and the second coupling partner is a non-covalent binding, a second adaptor, configured to ligate to a free end of the double-stranded DNA molecule corresponding to each of the plurality of single-stranded DNA molecules immobilized to the solid support at an immobilized end thereof, wherein the second adaptor comprises a third strand and a fourth strand, wherein:

the fourth strand comprises:

a second primer recognition sequence, configured to provide a priming site for amplification of the double-stranded DNA molecule corresponding to the each of the plurality of single-stranded DNA molecules; and a phosphate group at a 5' end thereof;

and the third strand comprises a sequence complementary to a 5'-end sequence of the fourth strand, and is configured to form a duplex with, and thereby to ensure a stability of, the 5'-end sequence of the fourth strand.

6. The kit of Claim 5, wherein the first strand of the first adaptor further comprises at least one of an index sequence or a separator sequence, wherein:

the index sequence is between the phosphate group and the barcode sequence, or between the barcode sequence and the first primer recognition sequence, and is configured to provide index information for each of the plurality of single-stranded DNA molecules ligated to the first strand of the first adaptor; and the separator sequence is disposed between the phosphate group and the barcode sequence and is configured to serve as a separation marker between the barcode sequence and each of the plurality of single-stranded DNA molecules ligated to the first strand of the first adaptor.

7. The kit of Claim 5, wherein the first adaptor is single-stranded, and the DNA ligase comprises a single-stranded DNA ligase.

8. The kit of Claim 5, wherein the first adaptor is partially double-stranded, wherein:

the first adaptor comprises a single-stranded segment at the 5'end of the first strand; and the DNA ligase comprises a single-stranded DNA ligase.

9. The kit of Claim 8, wherein:

the first adaptor further comprises a second strand, comprising a first portion at a 5' end thereof and a second portion at a 3' end thereof, wherein the first portion of the second strand forms a double-stranded duplex with the 5'end of the first strand, and the second portion forms a single-stranded overhang in the first adaptor; and the DNA ligase comprises a bandage strand-facilitated DNA ligase.

10. The kit of Claim 5, wherein the fourth strand further comprises at least one of a second index sequence, or a second barcode sequence, or a sequencing primer sequence at a 5' end of the second primer recognition sequence.

11. The kit of Claim 5, wherein the third strand further comprises, at a 5' end of thereof, at least one of:

a cap structure, comprising a sequence that does not match with a 3'-end sequence of the fourth strand, and configured to avoid concatenation of the second adaptor in a ligation reaction; an overhang sequence, forming a single-stranded segment for the second adaptor; or a functional moiety.

12. The kit of Claim 5, further comprising a pair of primers, configured to amplify the double-stranded DNA molecule corresponding to the each of the plurality of single-stranded DNA molecules therethrough, wherein:

one of the pair of primers comprises a sequence corresponding to at least a portion of a sequence of the first primer in the first strand of the first adaptor; and another of the pair of primers comprises a sequence corresponding to at least a portion of the second primer recognition sequence in the fourth strand of the second adaptor.

**Patentansprüche**

1. Verfahren zum Aufbauen einer DNA-Bibliothek aus einer biologischen Probe, die eine Vielzahl von Nukleinsäuresequenzen enthält, umfassend:

Herstellen einer DNA-Probe aus der biologischen Probe, wobei die DNA-Probe eine Vielzahl von einzelsträngigen DNA-Molekülen umfasst, die jeweils ein dephosphoryliertes 5'-Ende aufweisen;

Ligieren eines ersten Strangs eines ersten Adaptors an ein 3'-Ende jedes der Vielzahl von einzelsträngigen DNA-Molekülen, wobei der erste Strang des ersten Adaptors eine Phosphatgruppe, eine Barcodesequenz und eine erste Primer-Erkennungssequenz entlang einer Richtung von einem 5'-Ende davon zu einem 3'-Ende davon umfasst; und

Synthetisieren eines komplementären Strangs für jedes der Vielzahl von einzelsträngigen DNA-Molekülen, die an den ersten Strang des ersten Adaptors ligiert sind, um ein diesem entsprechendes mit Barcode markiertes doppelsträngiges DNA-Molekül zu erhalten,

wobei der erste Strang des ersten Adaptors ferner einen Immobilisierungsabschnitt an dem 3'-Ende davon umfasst, der konfiguriert ist, um eine stabile Kopplung mit einem festen Träger bilden zu können, wobei:

das Verfahren ferner umfasst, zwischen dem Ligieren eines ersten Strangs eines ersten Adaptors an ein 3'-Ende jedes der Vielzahl von einzelsträngigen DNA-Molekülen und dem Synthetisieren eines komplementären Strangs für jedes der Vielzahl von einzelsträngigen DNA-Molekülen, die an den ersten Strang des ersten Adaptors ligiert sind, um ein diesem entsprechendes mit Barcode markiertes doppelsträngiges DNA-Molekül zu erhalten:

Immobilisieren jedes der Vielzahl von einzelsträngigen DNA-Molekülen, die an den ersten Strang des ersten Adaptors ligiert sind, an dem festen Träger über die stabile Kopplung zwischen dem Immobilisierungsabschnitt und dem festen Träger;

das Verfahren ferner umfasst, nach dem Synthetisieren eines komplementären Strangs für jedes der Vielzahl von einzelsträngigen DNA-Molekülen, die an den ersten Strang des ersten Adaptors ligiert sind, um ein diesem entsprechendes doppelsträngiges mit Barcode markiertes DNA-Molekül zu erhalten:

Ligieren eines zweiten Adaptors an ein freies Ende des doppelsträngigen DNA-Moleküls, das dem jeden der Vielzahl von einzelsträngigen DNA-Molekülen entspricht, die an dem festen Träger an einem immobilisierten Ende davon immobilisiert sind, wobei der zweite Adaptor einen dritten Strang und einen vierten Strang umfasst, wobei:

der vierte Strang umfasst:

eine zweite Primer-Erkennungssequenz, die konfiguriert ist, um eine Primingstelle für eine Amplifikation des doppelsträngigen DNA-Moleküls bereitzustellen, das dem jeden der Vielzahl von einzelsträngigen DNA-Molekülen entspricht; und

eine Phosphatgruppe an einem 5'-Ende davon; und

der dritte Strang eine Sequenz umfasst, die komplementär zu einer 5'-Endsequenz des vierten Strangs ist, und konfiguriert ist, um eine Duplex mit der 5'-Endsequenz des vierten Strangs zu bilden und dadurch eine Stabilität dieser sicherzustellen.

2. Verfahren nach Anspruch 1, wobei die Vielzahl von Nukleinsäuresequenzen in der biologischen Probe eine Vielzahl von DNA-Sequenzen umfasst, und das Herstellen einer DNA-Probe aus der biologischen Probe umfasst:
Durchführen einer Dephosphorylierungsreaktion und einer Dissoziationsreaktion, um eine Vielzahl von einzelsträngigen DNA-Molekülen zu erhalten, die jeweils ein dephosphoryliertes 5'-Ende aufweisen.

3. Verfahren nach Anspruch 1, wobei der erste Adaptor ein einzelsträngiges Segment an dem 5'-Ende des ersten Strangs davon umfasst, und das Ligieren eines ersten Strangs eines ersten Adapters an ein 3'-Ende jedes der Vielzahl von einzelsträngigen DNA-Molekülen umfasst:
Durchführen einer Ligationsreaktion durch eine einzelsträngige DNA-Ligase, so dass das 3'-Ende jedes der Vielzahl von einzelsträngigen DNA-Molekülen an das 5'-Ende des ersten Strangs des ersten Adapters ligiert wird.

4. Verfahren nach Anspruch 1, wobei das Synthetisieren eines komplementären Strangs für jedes der Vielzahl von einzelsträngigen DNA-Molekülen, die an den ersten Strang des ersten Adaptors ligiert sind, um ein diesem entsprechendes mit Barcode markiertes doppelsträngiges DNA-Molekül zu erhalten, umfasst:

Aufschmelzen eines ersten Primers auf jedes der Vielzahl von einzelsträngigen DNA-Molekülen, die an den ersten Strang des ersten Adaptors ligiert sind, wobei der erste Primer eine Sequenz umfasst, die komplementär zu der ersten Primer-Erkennungssequenz in dem ersten Strang des ersten Adaptors ist; und

Durchführen einer Einzelstrangverlängerungsreaktion, um ein doppelsträngiges DNA-Molekül für jedes der Vielzahl von einzelsträngigen DNA-Molekülen zu bilden, die an den ersten Strang des ersten Adaptors ligiert sind.

**5.** Kit zum Aufbauen einer DNA-Bibliothek aus einer biologischen Probe, die eine Vielzahl von Nukleinsäuresequenzen enthält, umfassend:

einen ersten Adaptor, der einen ersten Strang aufweist, umfassend, in einer Richtung von einem 5'-Ende davon zu einem 3'-Ende davon, eine Phosphatgruppe, eine Barcodesequenz und eine erste Primer-Erkennungssequenz, wobei die Barcodesequenz konfiguriert ist, um jedem der Vielzahl von einzelsträngigen DNA-Molekülen, die an den ersten Strang des ersten Adaptors ligiert sind, Barcodeinformationen bereitzustellen;

eine DNA-Ligase, die konfiguriert ist, um eine Ligation zwischen dem 5'-Ende des ersten Strangs des ersten Adaptors und einem 3'-Ende jedes einer Vielzahl von einzelsträngigen DNA-Molekülen zu ermöglichen, wobei jedes der Vielzahl von einzelsträngigen DNA-Molekülen einer der Vielzahl von Nukleinsäuresequenzen in der biologischen Probe entspricht; und

einen ersten Primer, umfassend eine Sequenz, die komplementär zu der ersten Primer-Erkennungssequenz des ersten Adaptors ist und konfiguriert ist, um eine Einzelstrangverlängerungsreaktion zu ermöglichen, um dadurch ein doppelsträngiges DNA-Molekül zu bilden, das jedem der Vielzahl von einzelsträngigen DNA-Molekülen entspricht, die an den ersten Strang des ersten Adaptors ligiert sind,

einen festen Träger, wobei der erste Strang des ersten Adaptors ferner einen Immobilisierungsabschnitt an dem 3'-Ende davon umfasst, der konfiguriert ist, um die Immobilisierung jedes der Vielzahl von einzelsträngigen DNA-Molekülen, die an den ersten Strang des ersten Adaptors an das 5'-Ende davon ligiert sind, an dem festen Träger zu ermöglichen, wobei der Immobilisierungsabschnitt einen ersten Kopplungspartner umfasst, der konfiguriert ist, um eine stabile Kopplung mit einem zweiten Kopplungspartner, der an dem festen Träger angebracht ist, bilden zu können, wobei die stabile Kopplung zwischen dem ersten Kopplungspartner und dem zweiten Kopplungspartner eine nicht kovalente Bindung ist,

einen zweiten Adaptor, der konfiguriert ist, um an ein freies Ende des doppelsträngigen DNA-Moleküls zu ligieren, das jedem der Vielzahl von einzelsträngigen DNA-Molekülen entspricht, die an dem festen Träger an einem immobilisierten Ende davon immobilisiert sind, wobei der zweite Adaptor einen dritten Strang und einen vierten Strang umfasst, wobei:

der vierte Strang umfasst:

eine zweite Primer-Erkennungssequenz, die konfiguriert ist, um eine Primingstelle für eine Amplifikation des doppelsträngigen DNA-Moleküls bereitzustellen, das dem jeden der Vielzahl von einzelsträngigen DNA-Molekülen entspricht; und
eine Phosphatgruppe an einem 5'-Ende davon; und

wobei der dritte Strang eine Sequenz umfasst, die komplementär zu einer 5'-Endsequenz des vierten Strangs ist, und konfiguriert ist, um eine Duplex mit der 5'-Endsequenz des vierten Strangs zu bilden und dadurch eine Stabilität dieser sicherzustellen.

**6.** Kit nach Anspruch 5, wobei der erste Strang des ersten Adaptors ferner mindestens eine von einer Indexsequenz oder einer Trennsequenz umfasst, wobei:

die Indexsequenz zwischen der Phosphatgruppe und der Barcodesequenz oder zwischen der Barcodesequenz und der ersten Primer-Erkennungssequenz liegt und konfiguriert ist, um jedem der Vielzahl von einzelsträngigen DNA-Molekülen, die an den ersten Strang des ersten Adaptors ligiert sind, Indexinformationen bereitzustellen; und
die Trennsequenz zwischen der Phosphatgruppe und der Barcodesequenz angeordnet ist und konfiguriert ist, um als ein Trennungsmarker zwischen der Barcodesequenz und jedem der Vielzahl von einzelsträngigen DNA-Molekülen zu fungieren, die an den ersten Strang des ersten Adaptors ligiert sind.

**7.** Kit nach Anspruch 5, wobei der erste Adaptor einzelsträngig ist und die DNA-Ligase eine einzelsträngige DNA-Ligase umfasst.

**8.** Kit nach Anspruch 5, wobei der erste Adaptor teilweise doppelsträngig ist, wobei:

der erste Adaptor ein einzelsträngiges Segment an dem 5'-Ende des ersten Strangs umfasst; und
die DNA-Ligase eine einzelsträngige DNA-Ligase umfasst.

9. Kit nach Anspruch 8, wobei:

der erste Adaptor ferner einen zweiten Strang umfasst, umfassend einen ersten Abschnitt an einem 5'-Ende davon und einen zweiten Abschnitt an einem 3'-Ende davon, wobei der erste Abschnitt des zweiten Strangs eine doppelsträngige Duplex mit dem 5'-Ende des ersten Strangs bildet, und der zweite Abschnitt einen einzelsträngigen Überhang in dem ersten Adaptor bildet; und
die DNA-Ligase eine bandstrangunterstützte DNA-Ligase umfasst.

10. Kit nach Anspruch 5, wobei der vierte Strang ferner mindestens eine von einer zweiten Indexsequenz oder einer zweiten Barcodesequenz oder einer Sequenzierungsprimersequenz an einem 5'-Ende der zweiten Primer-Erkennungssequenz umfasst.

11. Kit nach Anspruch 5, wobei der dritte Strang ferner an einem 5'-Ende davon mindestens eines umfasst:

eine Kappenstruktur, umfassend eine Sequenz, die nicht mit einer 3'-Endsequenz des vierten Strangs übereinstimmt, und konfiguriert ist, um eine Verkettung des zweiten Adaptors in einer Ligationsreaktion zu vermeiden;
eine Überhangsequenz, die ein einzelsträngiges Segment für den zweiten Adaptor bildet; oder
einen funktionellen Anteil.

12. Kit nach Anspruch 5, ferner umfassend ein Paar von Primern, die konfiguriert sind, um das doppelsträngige DNA-Molekül, das dem jeden der Vielzahl von einzelsträngigen DNA-Molekülen entspricht, dadurch zu amplifizieren, wobei:

einer des Paars von Primern eine Sequenz umfasst, die mindestens einem Abschnitt einer Sequenz des ersten Primers in dem ersten Strang des ersten Adaptors entspricht; und
ein anderer des Paars von Primern eine Sequenz umfasst, die mindestens einem Abschnitt der zweiten Primer-Erkennungssequenz in dem vierten Strang des zweiten Adaptors entspricht.

**Revendications**

1. Procédé de construction d'une bibliothèque d'ADN à partir d'un échantillon biologique contenant une pluralité de séquences d'acide nucléique, comprenant :

la préparation d'un échantillon d'ADN à partir de l'échantillon biologique, dans lequel l'échantillon d'ADN comprend une pluralité de molécules d'ADN simple brin, chacune ayant une extrémité 5' déphosphorylée ;
la ligature d'un premier brin d'un premier adaptateur à une extrémité 3' de chacune de la pluralité de molécules d'ADN simple brin, dans lequel le premier brin du premier adaptateur comprend un groupe phosphate, une séquence de codes-barres et une première séquence de reconnaissance d'amorce dans une direction allant d'une extrémité 5' de celui-ci à une extrémité 3' de celui-ci ; et
la synthèse d'un brin complémentaire pour chacune de la pluralité de molécules d'ADN simple brin ligaturées au premier brin du premier adaptateur pour obtenir une molécule d'ADN double brin à code-barres correspondant à celle-ci,
dans lequel le premier brin du premier adaptateur comprend en outre une partie d'immobilisation à l'extrémité 3' de celui-ci, conçue pour pouvoir former un couplage stable à un support solide, dans lequel :

le procédé comprend en outre, entre la ligature d'un premier brin d'un premier adaptateur à une extrémité 3' de chacune de la pluralité de molécules d'ADN simple brin et la synthèse d'un brin complémentaire pour chacune de la pluralité de molécules d'ADN simple brin ligaturées au premier brin du premier adaptateur pour obtenir une molécule d'ADN double brin à code-barres correspondant à celle-ci :
l'immobilisation de chacune de la pluralité de molécules d'ADN simple brin ligaturées au premier brin du premier adaptateur sur le support solide par l'intermédiaire du couplage stable entre la partie d'immobilisation et le support solide ;
le procédé comprend en outre, après la synthèse d'un brin complémentaire pour chacune de la pluralité de molécules d'ADN simple brin ligaturées au premier brin du premier adaptateur pour obtenir une molécule d'ADN double brin à code-barres correspondant à celle-ci :
la ligature d'un second adaptateur à une extrémité libre de la molécule d'ADN double brin correspondant à chacune de la pluralité de molécules d'ADN simple brin immobilisées sur le support solide à une extrémité immobilisée de celui-ci, dans lequel le second adaptateur comprend un troisième brin et un quatrième brin, dans lequel :
le quatrième brin comprend :

une seconde séquence de reconnaissance d'amorce, conçue pour fournir un site d'amorçage pour l'amplification de la molécule d'ADN double brin correspondant à chacune de la pluralité de molécules d'ADN simple brin ; et

un groupe phosphate à une extrémité 5' de celui-ci ; et

le troisième brin comprend une séquence complémentaire à une séquence d'extrémité 5' du quatrième brin, et est conçu pour former un duplex avec, et ainsi garantir une stabilité de, la séquence d'extrémité 5' du quatrième brin.

**2.** Procédé selon la revendication 1, dans lequel la pluralité de séquences d'acides nucléiques dans l'échantillon biologique comprend une pluralité de séquences d'ADN, et la préparation d'un échantillon d'ADN provenant de l'échantillon biologique comprend :

la réalisation d'une réaction de déphosphorylation et d'une réaction de dissociation pour obtenir une pluralité de molécules d'ADN simple brin, chacune ayant une extrémité 5' déphosphorylée.

**3.** Procédé selon la revendication 1, dans lequel le premier adaptateur comprend un segment simple brin à l'extrémité 5' du premier brin de celui-ci, et la ligature d'un premier brin d'un premier adaptateur à une extrémité 3' de chacune de la pluralité de molécules d'ADN simple brin comprend :

la réalisation d'une réaction de ligature à travers une ADN ligase simple brin de telle sorte que l'extrémité 3' de chacune de la pluralité de molécules d'ADN simple brin est ligaturée à l'extrémité 5' du premier brin du premier adaptateur.

**4.** Procédé selon la revendication 1, dans lequel la synthèse d'un brin complémentaire pour chacune de la pluralité de molécules d'ADN simple brin ligaturées au premier brin du premier adaptateur pour obtenir une molécule d'ADN double brin à code-barres correspondant à celle-ci comprend :

la renaturation d'une première amorce avec chacune de la pluralité de molécules d'ADN simple brin ligaturées au premier brin du premier adaptateur, dans lequel la première amorce comprend une séquence complémentaire à la première séquence de reconnaissance d'amorce dans le premier brin du premier adaptateur ; et

la réalisation d'une réaction d'extension simple brin pour former une molécule d'ADN double brin pour chacune de la pluralité de molécules d'ADN simple brin ligaturées au premier brin du premier adaptateur.

**5.** Kit pour construire une bibliothèque d'ADN à partir d'un échantillon biologique contenant une pluralité de séquences d'acide nucléique, comprenant :

un premier adaptateur, ayant un premier brin comprenant, dans une direction allant d'une extrémité 5' de celui-ci à une extrémité 3' de celui-ci, un groupe phosphate, une séquence de codes-barres et une première séquence de reconnaissance d'amorce, dans lequel la séquence de codes-barres est conçue pour fournir des informations de code-barres à chacune de la pluralité de molécules d'ADN simple brin ligaturées au premier brin du premier adaptateur ;

une ADN ligase, conçue pour permettre une ligature entre l'extrémité 5' du premier brin du premier adaptateur à une extrémité 3' de chacune d'une pluralité de molécules d'ADN simple brin, dans lequel chacune de la pluralité de molécules d'ADN simple brin correspond à l'une de la pluralité de séquences d'acides nucléiques dans l'échantillon biologique ; et

une première amorce, comprenant une séquence complémentaire à la première séquence de reconnaissance d'amorce du premier adaptateur et conçue pour permettre une réaction d'extension simple brin pour ainsi former une molécule d'ADN double brin correspondant à chacune de la pluralité de molécules d'ADN simple brin ligaturées au premier brin du premier adaptateur,

un support solide, dans lequel le premier brin du premier adaptateur comprend en outre une partie d'immobilisation à l'extrémité 3' de celui-ci, conçu pour permettre l'immobilisation de chacune de la pluralité de molécules d'ADN simple brin ligaturées au premier brin du premier adaptateur à l'extrémité 5' de celui-ci au support solide, dans lequel la partie d'immobilisation comprend un premier partenaire de couplage, conçu pour pouvoir former un couplage stable avec un second partenaire de couplage fixé au support solide, dans lequel le couplage stable entre le premier partenaire de couplage et le second partenaire de couplage est une liaison non covalente,

un second adaptateur, conçu pour se ligaturer à une extrémité libre de la molécule d'ADN double brin correspondant à chacune de la pluralité de molécules d'ADN simple brin immobilisées sur le support solide à une extrémité immobilisée de celui-ci, dans lequel le second adaptateur comprend un troisième brin et un quatrième brin, dans lequel :

le quatrième brin comprend :

une seconde séquence de reconnaissance d'amorce, conçue pour fournir un site d'amorçage pour l'amplification de la molécule d'ADN double brin correspondant à chacune de la pluralité de molécules d'ADN simple brin ; et

un groupe phosphate à une extrémité 5' de celui-ci ;

et

le troisième brin comprend une séquence complémentaire à une séquence d'extrémité 5' du quatrième brin, et est conçu pour former un duplex avec, et ainsi garantir une stabilité de, la séquence d'extrémité 5' du quatrième brin.

6. Kit selon la revendication 5, dans lequel le premier brin du premier adaptateur comprend en outre au moins une parmi une séquence d'indice ou une séquence de séparateur, dans lequel :

la séquence d'indice est entre le groupe phosphate et la séquence de codes-barres, ou entre la séquence de codes-barres et la première séquence de reconnaissance d'amorce, et est conçue pour fournir des informations d'indice pour chacune de la pluralité de molécules d'ADN simple brin ligaturées au premier brin du premier adaptateur ; et
la séquence de séparateur est disposée entre le groupe phosphate et la séquence de codes-barres et est conçue pour servir de marqueur de séparation entre la séquence de codes-barres et chacune de la pluralité de molécules d'ADN simple brin ligaturées au premier brin du premier adaptateur.

7. Kit selon la revendication 5, dans lequel le premier adaptateur est simple brin, et l'ADN ligase comprend une ADN ligase simple brin.

8. Kit selon la revendication 5, dans lequel le premier adaptateur est partiellement double brin, dans lequel :

le premier adaptateur comprend un segment simple brin à l'extrémité 5' du premier brin ; et
l'ADN ligase comprend une ADN ligase simple brin.

9. Kit selon la revendication 8, dans lequel :

le premier adaptateur comprend en outre un deuxième brin, comprenant une première partie à une extrémité 5' de celui-ci et une seconde partie à une extrémité 3' de celui-ci, dans lequel la première partie du deuxième brin forme un duplex double brin avec l'extrémité 5' du premier brin, et la seconde partie forme un surplomb simple brin dans le premier adaptateur ; et l'ADN ligase comprend une ADN ligase facilitée par un brin de bande.

10. Kit selon la revendication 5, dans lequel le quatrième brin comprend en outre au moins l'une parmi une seconde séquence d'indice, ou une seconde séquence de code-barres, ou une séquence d'amorces de séquençage à une extrémité 5' de la seconde séquence de reconnaissance d'amorce.

11. Trousse selon la revendication 5, dans laquelle le troisième brin comprend en outre, à une extrémité 5' de celui-ci, au moins l'un parmi :

une structure de coiffe, comprenant une séquence qui ne correspond pas à une séquence d'extrémité 3' du quatrième brin, et conçue pour éviter la concaténation du second adaptateur dans une réaction de ligature ;
une séquence de surplomb, formant un segment simple brin pour le second adaptateur ; ou
une fraction fonctionnelle.

12. Kit selon la revendication 5, comprenant en outre une paire d'amorces, conçue pour amplifier la molécule d'ADN double brin correspondant à chacune de la pluralité de molécules d'ADN simple brin à travers celle-ci, dans lequel :

l'une des paires d'amorces comprend une séquence correspondant à au moins une partie d'une séquence de la première amorce dans le premier brin du premier adaptateur ; et
une autre de la paire d'amorces comprend une séquence correspondant à au moins une partie de la seconde séquence de reconnaissance d'amorce dans le quatrième brin du second adaptateur.

Preparing a DNA sample comprising a plurality of single-stranded DNA molecules, each having a dephosphorylated 5' end ⌐S100

Ligating a first strand of a first adaptor to a 3' end of each of the plurality of single-stranded DNA molecules, wherein the first strand of the first adaptor comprises a barcode sequence and a first primer recognition sequence at a 5' end and a 3' end thereof, respectively ⌐S200

Synthesizing a complementary strand for each of the plurality of single-stranded DNA molecules ligated to the first strand of the first adaptor to obtain a barcoded double-stranded DNA molecule corresponding thereto ⌐S300

**FIG. 1**

S100

Shearing DNA sequences into DNA fragments ⌐S110

Performing a dephosphorylation reaction ⌐S120

Performing a dissociation reaction ⌐S130

**FIG. 2A**

S100

| Shearing DNA sequences into DNA fragments | S110 |

↓

| Performing a dissociation reaction | S130 |

↓

| Performing a dephosphorylation reaction | S120 |

**FIG. 2B**

S100

| Shearing DNA sequences into DNA fragments | S110 |

↓

| Performing a dissociation reaction | S130 |

(x $n$ times)

| Performing a dephosphorylation reaction | S120 |

**FIG. 2C**

S109

| Performing a reverse transcription using an oligo(dT) as a primer to obtain a cDNA sequence corresponding to each RNA molecule | S1091 |

**FIG. 3A**

<u>S109</u>

| Performing a polyadenylation at a 3' end of each RNA molecule | ⊢S1091' |

↓

| Performing a reverse transcription using an oligo(dT) as a primer to obtain a cDNA sequence corresponding to each RNA molecule | ⊢S1092' |

**FIG. 3B**

<u>S109</u>

| Performing a reverse transcription by means of random primers to obtain cDNAs corresponding to each of the plurality of RNA molecules | ⊢S1091'' |

**FIG. 3C**

<u>01</u>

100    200

5' Ⓟ ▓▓▓▓▓▓ ▤▤▤▤▤ 3'

**FIG. 4**

FIG. 5A

FIG. 5B

FIG. 5C

**FIG. 5D (Prior Art)**

**FIG. 6A**

**FIG. 6B**

FIG. 6C

**FIG. 7**

**FIG. 8A**

**FIG. 8B**

FIG. 9A

FIG. 9B

S300

Annealing the first primer with each of the plurality of single-stranded DNA molecule ligated to the first adaptor — S310

Performing a single-strand extension reaction to form the double-stranded DNA molecule for each of the plurality of single-stranded DNA molecules ligated to the single-stranded adaptor — S320

**FIG. 10**

Ligating a second adaptor to a free end of each double-stranded DNA molecule immobilized to the solid support at an immobilized end — S400

Eluting the DNA library from the solid support — S500

Performing a PCR reaction to thereby amplify each double-stranded DNA molecule — S600

**FIG. 11**

02

02a

5′ 3′

3′ (P) 5′

600 02b

**FIG. 12A**

02

700 5′ 600 02a

3′

3′ (P) 5′

02b

**FIG. 12B**

02

800 02a

5′ 3′

3′ (P) 5′

600 02b

**FIG. 12C**

**FIG. 12D**

**FIG. 12E**

**FIG. 13A**

**FIG. 13B**

**FIG. 14A**

FIG. 14B

Product strand dissociation

Amplification with PE primers | DNA Polymerase

NGS Sequencing Applications (WGS, WES, Targeted Sequencing, etc.)

FIG. 14C

FIG. 15

Human Chromosomes

1 2 3 4 5 6 7 8 9 10 11 12 13 14 15 16 17 18 19 20 21 22 X Y

FIG. 16

FIG. 17A

FIG. 17B

## Total number of SNVs detected

FIG. 18A

FIG. 18B

**Read Statistics in RNA probe based DNA double strand capture WES**

FIG. 19A

Statistics of Removed Reads

FIG. 19B

## Coverage efficiency as a function of read numbers

FIG. 19C

## %GC content

FIG. 20A

FIG. 20B

Detection of ultra-rare SNVs

Log10 transformed dilution ratio

FIG. 21

| Gene Symbol | EXON ID | Forward Primer (SEQ ID NO.) | Reverse Primer (SEQ ID NO.) | Amplicon Seq (SEQ ID NO.) | Amplif Eff Constant | GC% of Amplicon |
|---|---|---|---|---|---|---|
| PTEN | 3 | 1 | 305 | 609 | 1.85 | 27.3% |
| FANCL | 3 | 2 | 306 | 610 | 1.99 | 30.1% |
| FANCL | 13 | 3 | 307 | 611 | 1.95 | 35.0% |
| FANCM | 12 | 4 | 308 | 612 | 1.78 | 30.3% |
| NRAS | 6 | 5 | 309 | 613 | 1.90 | 31.7% |
| XPA | 3 | 6 | 310 | 614 | 1.94 | 32.4% |
| XPA | 5 | 7 | 311 | 615 | 1.79 | 34.9% |
| BRCA2 | 9 | 8 | 312 | 616 | 1.85 | 32.8% |
| FANCF | 1 | 9 | 313 | 617 | 1.92 | 32.8% |
| FIP1L1 | 6 | 10 | 314 | 618 | 1.97 | 32.9% |
| BRIP1 | 10 | 11 | 315 | 619 | 1.76 | 33.0% |
| MLH1 | 17 | 12 | 316 | 620 | 1.92 | 33.1% |
| STAG2 | 9 | 13 | 317 | 621 | 1.77 | 33.3% |
| XPO1 | 5 | 14 | 318 | 622 | 1.83 | 33.3% |
| RB1 | 14 | 15 | 319 | 623 | 1.78 | 33.6% |
| NBN | 16 | 16 | 320 | 624 | 1.78 | 33.6% |
| NBN | 10 | 17 | 321 | 625 | 1.94 | 41.7% |
| MDM2 | 4 | 18 | 322 | 626 | 1.78 | 33.8% |
| ATR | 37 | 19 | 323 | 627 | 1.80 | 33.9% |
| FBXO11 | 2 | 20 | 324 | 628 | 1.91 | 34.0% |
| AKAP9 | 20 | 21 | 325 | 629 | 1.80 | 34.1% |
| SDHD | 4 | 22 | 326 | 630 | 1.96 | 34.7% |

**FIG. 22A**

| Gene Symbol | Exon ID | Forward Primer (SEQ ID NO.) | Reverse Primer (SEQ ID NO.) | Amplicon Seq (SEQ ID NO.) | Amplif Eff Constant | GC% of Amplicon |
|---|---|---|---|---|---|---|
| PIK3CA | 17 | 23 | 327 | 631 | 1.89 | 34.9% |
| CDC73 | 12 | 24 | 328 | 632 | 1.92 | 35.2% |
| ACSL3 | 10 | 25 | 329 | 633 | 1.96 | 35.4% |
| WRN | 16 | 26 | 330 | 634 | 1.94 | 35.4% |
| HSP90AA1 | 6 | 27 | 331 | 635 | 1.77 | 36.0% |
| CDK12 | 7 | 28 | 332 | 636 | 1.76 | 36.0% |
| POT1 | 6 | 29 | 333 | 637 | 1.84 | 36.1% |
| KDM6A | 7 | 30 | 334 | 638 | 1.98 | 36.4% |
| KRAS | 2 | 31 | 335 | 639 | 1.90 | 36.5% |
| BLM | 3 | 32 | 336 | 640 | 1.87 | 36.6% |
| GOLGA5 | 9 | 33 | 337 | 641 | 1.91 | 36.6% |
| PRKAR1A | 11 | 34 | 338 | 642 | 1.91 | 36.7% |
| TMPRSS2 | 3 | 35 | 339 | 643 | 1.89 | 36.8% |
| CLTC | 24 | 36 | 340 | 644 | 1.95 | 36.9% |
| FAS | 8 | 37 | 341 | 645 | 1.99 | 36.9% |
| MSH2 | 9 | 38 | 342 | 646 | 1.91 | 37.0% |
| BUB1B | 3 | 39 | 343 | 647 | 1.75 | 37.0% |
| NF1 | 24 | 40 | 344 | 648 | 1.79 | 37.3% |
| TOP1 | 7 | 41 | 345 | 649 | 1.96 | 37.3% |
| NCOR1 | 17 | 42 | 346 | 650 | 1.87 | 37.3% |
| POLH | 8 | 43 | 347 | 651 | 1.75 | 37.3% |
| SMAD2 | 8 | 44 | 348 | 652 | 1.87 | 37.5% |

**FIG. 22B**

| Gene Symbol | Exon ID | Forward Primer (SEQ ID NO.) | Reverse Primer (SEQ ID NO.) | Amplicon Seq (SEQ ID NO.) | Amplif Eff Constant | GC% of Amplicon |
|---|---|---|---|---|---|---|
| FUBP1 | 3 | 45 | 349 | 653 | 1.83 | 37.5% |
| RAF1 | 13 | 46 | 350 | 654 | 1.80 | 37.6% |
| SETD2 | 7 | 47 | 351 | 655 | 1.98 | 37.7% |
| DICER1 | 17 | 48 | 352 | 656 | 1.77 | 37.7% |
| FANCC | 7 | 49 | 353 | 657 | 1.89 | 37.7% |
| GPC3 | 4 | 50 | 354 | 658 | 1.98 | 37.7% |
| DDX5 | 11 | 51 | 355 | 659 | 1.79 | 38.0% |
| FAM46C | 2 | 52 | 356 | 660 | 1.96 | 38.1% |
| KAT6B | 9 | 53 | 357 | 661 | 2.00 | 38.1% |
| RAD51C | 7 | 54 | 358 | 662 | 1.91 | 38.4% |
| PPP6C | 6 | 55 | 359 | 663 | 1.91 | 38.4% |
| IDH1 | 4 | 56 | 360 | 664 | 1.82 | 38.4% |
| PMS2 | 9 | 57 | 361 | 665 | 1.96 | 38.5% |
| ATRX | 4 | 58 | 362 | 666 | 1.86 | 38.7% |
| ASXL1 | 2 | 59 | 363 | 667 | 1.85 | 38.7% |
| ASXL1 | 10 | 60 | 364 | 668 | 1.84 | 54.7% |
| PBRM1 | 8 | 61 | 365 | 669 | 2.00 | 38.8% |
| MET | 16 | 62 | 366 | 670 | 1.96 | 38.9% |
| EWSR1 | 12 | 63 | 367 | 671 | 1.89 | 39.0% |
| ATM | 38 | 64 | 368 | 672 | 1.84 | 39.3% |
| PALB2 | 6 | 65 | 369 | 673 | 1.79 | 39.3% |
| SF3B1 | 5 | 66 | 370 | 674 | 1.93 | 39.3% |

**FIG. 22C**

| Gene Symbol | Exon ID | Forward Primer (SEQ ID NO.) | Reverse Primer (SEQ ID NO.) | Amplicon Seq (SEQ ID NO.) | Amplif Eff Constant | GC% of Amplicon |
|---|---|---|---|---|---|---|
| FANCI | 16 | 67 | 371 | 675 | 1.98 | 39.7% |
| TET2 | 5 | 68 | 372 | 676 | 1.92 | 39.8% |
| TSHR | 6 | 69 | 373 | 677 | 1.97 | 40.0% |
| WHSC1L1 | 19 | 70 | 374 | 678 | 1.76 | 40.2% |
| NFE2L2 | 4 | 71 | 375 | 679 | 2.00 | 40.3% |
| BCOR | 4 | 72 | 376 | 680 | 1.91 | 40.4% |
| ETV5 | 10 | 73 | 377 | 681 | 1.77 | 40.5% |
| CREBBP | 23 | 74 | 378 | 682 | 1.82 | 40.6% |
| SDHAF2 | 3 | 75 | 379 | 683 | 1.82 | 40.7% |
| NPM1 | 11 | 76 | 380 | 684 | 1.86 | 40.7% |
| SPOP | 10 | 77 | 381 | 685 | 1.93 | 40.7% |
| TCF12 | 20 | 78 | 382 | 686 | 1.97 | 40.7% |
| ARID1B | 3 | 79 | 383 | 687 | 1.96 | 40.8% |
| BRAF | 14 | 80 | 384 | 688 | 1.89 | 41.0% |
| FH | 2 | 81 | 385 | 689 | 1.85 | 41.0% |
| ERCC5 | 3 | 82 | 386 | 690 | 1.78 | 41.2% |
| FANCD2 | 8 | 83 | 387 | 691 | 1.78 | 41.3% |
| MTOR | 52 | 84 | 388 | 692 | 1.81 | 41.3% |
| CASP8 | 7 | 85 | 389 | 693 | 1.76 | 41.5% |
| NUP98 | 3 | 86 | 390 | 694 | 1.90 | 41.7% |
| MDM4 | 10 | 87 | 391 | 695 | 1.99 | 41.7% |
| ACVR1 | 7 | 88 | 392 | 696 | 1.83 | 41.8% |

FIG. 22D

| Gene Symbol | Exon ID | Forward Primer (SEQ ID NO.) | Reverse Primer (SEQ ID NO.) | Amplicon Seq (SEQ ID NO.) | Amplif Eff Constant | GC% of Amplicon |
|---|---|---|---|---|---|---|
| ARID2 | 18 | 89 | 393 | 697 | 1.95 | 41.8% |
| MECOM | 6 | 90 | 394 | 698 | 1.76 | 42.0% |
| RET | 16 | 91 | 395 | 699 | 2.00 | 42.0% |
| BCL2 | 1 | 92 | 396 | 700 | 1.99 | 42.0% |
| FUS | 9 | 93 | 397 | 701 | 1.94 | 42.1% |
| TCF7L2 | 4 | 94 | 398 | 702 | 1.94 | 42.4% |
| ATIC | 5 | 95 | 399 | 703 | 1.80 | 42.6% |
| PMS1 | 2 | 96 | 400 | 704 | 1.79 | 42.6% |
| CHD4 | 34 | 97 | 401 | 705 | 1.77 | 42.7% |
| PTPN11 | 8 | 98 | 402 | 706 | 1.88 | 42.8% |
| ABL2 | 3 | 99 | 403 | 707 | 1.78 | 42.9% |
| MAP2K4 | 9 | 100 | 404 | 708 | 1.91 | 42.9% |
| CRTC3 | 5 | 101 | 405 | 709 | 1.96 | 43.0% |
| BRCA1 | 3 | 102 | 406 | 710 | 1.82 | 43.2% |
| LCP1 | 2 | 103 | 407 | 711 | 1.87 | 43.3% |
| FCRL4 | 10 | 104 | 408 | 712 | 1.83 | 43.3% |
| RAC1 | 4 | 105 | 409 | 713 | 1.77 | 43.5% |
| SMARCA4 | 14 | 106 | 410 | 714 | 1.76 | 43.5% |
| HSP90AB1 | 6 | 107 | 411 | 715 | 2.00 | 43.5% |
| TBL1XR1 | 3 | 108 | 412 | 716 | 1.88 | 43.6% |
| VHL | 2 | 109 | 413 | 717 | 1.93 | 43.7% |
| NOTCH2 | 12 | 110 | 414 | 718 | 1.94 | 43.7% |

**FIG. 22E**

| Gene Symbol | Exon ID | Forward Primer (SEQ ID NO.) | Reverse Primer (SEQ ID NO.) | Amplicon Seq (SEQ ID NO.) | Amplif Eff Constant | GC% of Amplicon |
|---|---|---|---|---|---|---|
| CTNNB1 | 15 | 111 | 415 | 719 | 1.86 | 43.7% |
| POLE | 3 | 112 | 416 | 720 | 1.95 | 43.8% |
| SDHC | 3 | 113 | 417 | 721 | 1.97 | 43.9% |
| KIT | 20 | 114 | 418 | 722 | 1.76 | 44.1% |
| ETV6 | 7 | 115 | 419 | 723 | 1.85 | 44.1% |
| CTCF | 6 | 116 | 420 | 724 | 1.98 | 44.1% |
| MAP2K1 | 9 | 117 | 421 | 725 | 1.97 | 44.2% |
| PSIP1 | 16 | 118 | 422 | 726 | 1.80 | 44.3% |
| RAD21 | 13 | 119 | 423 | 727 | 1.85 | 44.6% |
| KMT2A | 31 | 120 | 424 | 728 | 1.88 | 44.7% |
| CBL | 14 | 121 | 425 | 729 | 1.90 | 44.7% |
| MYB | 4 | 122 | 426 | 730 | 1.87 | 44.7% |
| SYK | 8 | 123 | 427 | 731 | 1.91 | 44.7% |
| ETV1 | 14 | 124 | 428 | 732 | 1.92 | 44.7% |
| BMPR1A | 12 | 125 | 429 | 733 | 1.96 | 44.7% |
| TBX3 | 4 | 126 | 430 | 734 | 1.96 | 44.9% |
| CBFB | 3 | 127 | 431 | 735 | 1.90 | 44.9% |
| DDR2 | 11 | 128 | 432 | 736 | 1.79 | 45.0% |
| NF2 | 5 | 129 | 433 | 737 | 2.00 | 45.0% |
| MSH6 | 7 | 130 | 434 | 738 | 1.88 | 45.1% |
| TSC1 | 2 | 131 | 435 | 739 | 1.86 | 45.3% |
| AFF4 | 12 | 132 | 436 | 740 | 1.91 | 45.3% |

**FIG. 22F**

| Gene Symbol | Exon ID | Forward Primer (SEQ ID NO.) | Reverse Primer (SEQ ID NO.) | Amplicon Seq (SEQ ID NO.) | Amplif Eff Constant | GC% of Amplicon |
|---|---|---|---|---|---|---|
| FOXP1 | 24 | 133 | 437 | 741 | 1.75 | 45.3% |
| TNFAIP3 | 4 | 134 | 438 | 742 | 1.96 | 45.6% |
| EP300 | 19 | 135 | 439 | 743 | 1.96 | 45.8% |
| MAP3K1 | 18 | 136 | 440 | 744 | 1.80 | 46.0% |
| U2AF1 | 4 | 137 | 441 | 745 | 1.98 | 46.0% |
| ERCC3 | 4 | 138 | 442 | 746 | 1.82 | 46.0% |
| CYLD | 2 | 139 | 443 | 747 | 1.87 | 46.1% |
| IKZF1 | 4 | 140 | 444 | 748 | 1.96 | 46.1% |
| STK11 | 2 | 141 | 445 | 749 | 1.83 | 46.2% |
| PHOX2B | 1 | 142 | 446 | 750 | 1.97 | 46.3% |
| SBDS | 2 | 143 | 447 | 751 | 1.88 | 46.3% |
| SFPQ | 7 | 144 | 448 | 752 | 1.86 | 46.6% |
| WHSC1 | 10 | 145 | 449 | 753 | 1.85 | 46.6% |
| PRRX1 | 4 | 146 | 450 | 754 | 1.80 | 46.7% |
| GNAQ | 4 | 147 | 451 | 755 | 1.76 | 46.8% |
| ERCC4 | 10 | 148 | 452 | 756 | 1.91 | 47.2% |
| FLT3 | 16 | 149 | 453 | 757 | 1.75 | 47.2% |
| MAX | 3 | 150 | 454 | 758 | 1.92 | 47.5% |
| EGFR | 5 | 151 | 455 | 759 | 1.80 | 47.7% |
| RPL22 | 3 | 152 | 456 | 760 | 1.80 | 47.7% |
| SMAD4 | 7 | 153 | 457 | 761 | 1.94 | 48.0% |
| KDR | 20 | 154 | 458 | 762 | 1.82 | 48.0% |

FIG. 22G

| Gene Symbol | Exon ID | Forward Primer (SEQ ID NO.) | Reverse Primer (SEQ ID NO.) | Amplicon Seq (SEQ ID NO.) | Amplif Eff Constant | GC% of Amplicon |
|---|---|---|---|---|---|---|
| CDKN1B | 2 | 155 | 459 | 763 | 1.76 | 48.1% |
| MKL1 | 6 | 156 | 460 | 764 | 1.99 | 48.1% |
| BCL11A | 3 | 157 | 461 | 765 | 1.77 | 48.4% |
| JAK2 | 2 | 158 | 462 | 766 | 1.86 | 48.6% |
| ERBB4 | 5 | 159 | 463 | 767 | 1.87 | 48.7% |
| ERBB3 | 24 | 160 | 464 | 768 | 1.85 | 48.7% |
| PDGFRA | 2 | 161 | 465 | 769 | 1.77 | 48.7% |
| ABL1 | 6 | 162 | 466 | 770 | 1.96 | 48.8% |
| TGFBR2 | 6 | 163 | 467 | 771 | 1.92 | 49.0% |
| EZH2 | 13 | 164 | 468 | 772 | 1.88 | 49.0% |
| ATF1 | 3 | 165 | 469 | 773 | 1.99 | 49.1% |
| XPC | 8 | 166 | 470 | 774 | 1.96 | 49.3% |
| DNAJB1 | 2 | 167 | 471 | 775 | 1.80 | 49.4% |
| USP6 | 2 | 168 | 472 | 776 | 1.79 | 49.4% |
| FGFR2 | 5 | 169 | 473 | 777 | 1.98 | 49.4% |
| RHOA | 4 | 170 | 474 | 778 | 1.96 | 49.5% |
| EXT2 | 9 | 171 | 475 | 779 | 1.89 | 49.7% |
| CDK4 | 5 | 172 | 476 | 780 | 1.92 | 49.7% |
| BTK | 12 | 173 | 477 | 781 | 1.77 | 50.0% |
| PIK3R1 | 3 | 174 | 478 | 782 | 1.83 | 50.0% |
| CUX1 | 8 | 175 | 479 | 783 | 1.77 | 50.0% |
| BCR | 6 | 176 | 480 | 784 | 1.89 | 50.0% |

**FIG. 22H**

| Gene Symbol | Exon ID | Forward Primer (SEQ ID NO.) | Reverse Primer (SEQ ID NO.) | Amplicon Seq (SEQ ID NO.) | Amplif Eff Constant | GC% of Amplicon |
|---|---|---|---|---|---|---|
| DNMT3A | 10 | 177 | 481 | 785 | 1.94 | 50.0% |
| COL1A1 | 6 | 178 | 482 | 786 | 1.94 | 50.3% |
| ESR1 | 10 | 179 | 483 | 787 | 1.80 | 50.5% |
| ALK | 26 | 180 | 484 | 788 | 1.76 | 51.0% |
| ARHGAP26 | 3 | 181 | 485 | 789 | 1.95 | 51.4% |
| ERCC1 | 6 | 182 | 486 | 790 | 1.94 | 51.6% |
| PTCH1 | 25 | 183 | 487 | 791 | 1.80 | 51.7% |
| AR | 7 | 184 | 488 | 792 | 1.90 | 51.7% |
| NCOA3 | 16 | 185 | 489 | 793 | 1.84 | 51.7% |
| IGF2R | 20 | 186 | 490 | 794 | 1.87 | 52.0% |
| IGF1R | 15 | 187 | 491 | 795 | 1.92 | 52.2% |
| TERT | 10 | 188 | 492 | 796 | 1.76 | 52.2% |
| ROS1 | 4 | 189 | 493 | 797 | 1.95 | 52.3% |
| RUNX1 | 6 | 190 | 494 | 798 | 1.87 | 52.5% |
| XRCC1 | 16 | 191 | 495 | 799 | 1.79 | 52.6% |
| MLLT4 | 11 | 192 | 496 | 800 | 1.85 | 53.2% |
| ARID1A | 17 | 193 | 497 | 801 | 1.96 | 53.2% |
| SDHB | 6 | 194 | 498 | 802 | 1.87 | 53.2% |
| KLF6 | 3 | 195 | 499 | 803 | 1.82 | 53.3% |
| SMAD3 | 7 | 196 | 500 | 804 | 1.96 | 53.3% |
| PAX5 | 7 | 197 | 501 | 805 | 1.85 | 53.3% |
| SMARCB1 | 3 | 198 | 502 | 806 | 1.99 | 53.4% |

**FIG. 22I**

| Gene Symbol | Exon ID | Forward Primer (SEQ ID NO.) | Reverse Primer (SEQ ID NO.) | Amplicon Seq (SEQ ID NO.) | Amplif Eff Constant | GC% of Amplicon |
|---|---|---|---|---|---|---|
| PRSS1 | 1 | 199 | 503 | 807 | 1.80 | 53.8% |
| ELF4 | 7 | 200 | 504 | 808 | 1.86 | 54.0% |
| FANCA | 13 | 201 | 505 | 809 | 1.90 | 54.0% |
| CALR | 4 | 202 | 506 | 810 | 1.94 | 54.1% |
| EXT1 | 8 | 203 | 507 | 811 | 1.98 | 54.1% |
| TP53 | 6 | 204 | 508 | 812 | 1.93 | 54.3% |
| PDGFRB | 21 | 205 | 509 | 813 | 1.83 | 54.3% |
| FAT1 | 20 | 206 | 510 | 814 | 1.79 | 54.6% |
| KDM5C | 2 | 207 | 511 | 815 | 1.83 | 54.6% |
| KDM5C | 24 | 208 | 512 | 816 | 1.80 | 55.6% |
| WT1 | 7 | 209 | 513 | 817 | 1.76 | 54.6% |
| IDH2 | 3 | 210 | 514 | 818 | 1.88 | 54.7% |
| WAS | 8 | 211 | 515 | 819 | 1.95 | 54.7% |
| MUTYH | 18 | 212 | 516 | 820 | 1.79 | 54.8% |
| MYD88 | 4 | 213 | 517 | 821 | 1.80 | 54.9% |
| PLCG1 | 20 | 214 | 518 | 822 | 1.87 | 54.9% |
| AURKA | 3 | 215 | 519 | 823 | 1.79 | 54.9% |
| MAML1 | 4 | 216 | 520 | 824 | 1.86 | 55.1% |
| SH2B3 | 6 | 217 | 521 | 825 | 1.91 | 55.2% |
| FGFR1 | 5 | 218 | 522 | 826 | 1.92 | 55.5% |
| PPP2R1A | 2 | 219 | 523 | 827 | 1.93 | 55.6% |
| MED12 | 44 | 220 | 524 | 828 | 1.84 | 56.0% |

FIG. 22J

| Gene Symbol | Exon ID | Forward Primer (SEQ ID NO.) | Reverse Primer (SEQ ID NO.) | Amplicon Seq (SEQ ID NO.) | Amplif Eff Constant | GC% of Amplicon |
|---|---|---|---|---|---|---|
| NDRG1 | 2 | 221 | 525 | 829 | 1.84 | 56.0% |
| FANCE | 8 | 222 | 526 | 830 | 1.91 | 56.0% |
| DDB2 | 5 | 223 | 527 | 831 | 1.75 | 56.1% |
| ERCC2 | 16 | 224 | 528 | 832 | 1.98 | 56.1% |
| SRC | 6 | 225 | 529 | 833 | 1.81 | 56.2% |
| AXIN2 | 9 | 226 | 530 | 834 | 1.80 | 56.2% |
| NOTCH4 | 26 | 227 | 531 | 835 | 1.93 | 56.4% |
| GATA3 | 5 | 228 | 532 | 836 | 1.98 | 56.4% |
| EIF4A2 | 1 | 229 | 533 | 837 | 1.97 | 56.5% |
| PRKACA | 2 | 230 | 534 | 838 | 1.83 | 56.7% |
| JAK1 | 3 | 231 | 535 | 839 | 1.89 | 57.1% |
| MYH9 | 5 | 232 | 536 | 840 | 1.85 | 57.1% |
| CHEK2 | 1 | 233 | 537 | 841 | 2.00 | 57.3% |
| FANCG | 9 | 234 | 538 | 842 | 2.00 | 57.3% |
| SUFU | 5 | 235 | 539 | 843 | 1.89 | 57.6% |
| CEBPA | 1 | 236 | 540 | 844 | 1.97 | 57.8% |
| SRGAP3 | 19 | 237 | 541 | 845 | 1.81 | 58.0% |
| NCOR2 | 9 | 238 | 542 | 846 | 1.99 | 58.1% |
| FLCN | 3 | 239 | 543 | 847 | 1.98 | 58.2% |
| CIITA | 10 | 240 | 544 | 848 | 1.85 | 58.7% |
| RBM10 | 5 | 241 | 545 | 849 | 1.99 | 58.7% |
| ETV4 | 2 | 242 | 546 | 850 | 1.98 | 58.7% |

**FIG. 22K**

| Gene Symbol | Exon ID | Forward Primer (SEQ ID NO.) | Reverse Primer (SEQ ID NO.) | Amplicon Seq (SEQ ID NO.) | Amplif Eff Constant | GC% of Amplicon |
|---|---|---|---|---|---|---|
| CCND1 | 4 | 243 | 547 | 851 | 1.87 | 58.8% |
| ERBB2 | 10 | 244 | 548 | 852 | 1.82 | 58.8% |
| MEN1 | 9 | 245 | 549 | 853 | 1.98 | 58.9% |
| CCNE1 | 4 | 246 | 550 | 854 | 1.84 | 59.1% |
| CDK6 | 2 | 247 | 551 | 855 | 1.77 | 59.3% |
| MYH11 | 1 | 248 | 552 | 856 | 1.97 | 59.4% |
| HLA-A | 5 | 249 | 553 | 857 | 1.99 | 59.4% |
| RNF43 | 5 | 250 | 554 | 858 | 1.87 | 59.7% |
| HLF | 2 | 251 | 555 | 859 | 1.76 | 60.0% |
| AXIN1 | 4 | 252 | 556 | 860 | 1.92 | 60.0% |
| FLT4 | 22 | 253 | 557 | 861 | 1.79 | 60.0% |
| SMO | 7 | 254 | 558 | 862 | 1.98 | 60.2% |
| ACSL6 | 10 | 255 | 559 | 863 | 1.99 | 60.7% |
| AKT1 | 13 | 256 | 560 | 864 | 1.94 | 60.7% |
| H3F3B | 3 | 257 | 561 | 865 | 1.80 | 60.8% |
| RARA | 1 | 258 | 562 | 866 | 1.83 | 60.9% |
| FBXW7 | 2 | 259 | 563 | 867 | 1.79 | 61.2% |
| TACC3 | 5 | 260 | 564 | 868 | 1.87 | 61.3% |
| FGFR3 | 4 | 261 | 565 | 869 | 1.98 | 61.7% |
| TSC2 | 2 | 262 | 566 | 870 | 1.96 | 62.0% |
| MYCN | 1 | 263 | 567 | 871 | 1.84 | 62.0% |
| CSF1R | 17 | 264 | 568 | 872 | 1.92 | 62.0% |

**FIG. 22L**

| Gene Symbol | Exon ID | Forward Primer (SEQ ID NO.) | Reverse Primer (SEQ ID NO.) | Amplicon Seq (SEQ ID NO.) | Amplif Eff Constant | GC% of Amplicon |
|---|---|---|---|---|---|---|
| APC | 2 | 265 | 569 | 873 | 1.88 | 62.2% |
| NTRK1 | 5 | 266 | 570 | 874 | 1.91 | 62.3% |
| BAP1 | 8 | 267 | 571 | 875 | 1.94 | 62.4% |
| AKT2 | 7 | 268 | 572 | 876 | 1.78 | 62.7% |
| POLD1 | 14 | 269 | 573 | 877 | 1.84 | 62.7% |
| RECQL4 | 10 | 270 | 574 | 878 | 1.86 | 62.9% |
| GNAS | 9 | 271 | 575 | 879 | 1.85 | 62.9% |
| DAXX | 1 | 272 | 576 | 880 | 1.79 | 62.9% |
| GATA2 | 6 | 273 | 577 | 881 | 1.77 | 63.1% |
| PHF6 | 1 | 274 | 578 | 882 | 1.82 | 63.2% |
| MPL | 11 | 275 | 579 | 883 | 1.96 | 63.3% |
| MYC | 2 | 276 | 580 | 884 | 1.93 | 63.3% |
| CNOT3 | 14 | 277 | 581 | 885 | 1.81 | 63.6% |
| CIC | 14 | 278 | 582 | 886 | 1.93 | 63.9% |
| FGFR4 | 14 | 279 | 583 | 887 | 1.90 | 64.2% |
| NOTCH1 | 2 | 280 | 584 | 888 | 1.86 | 64.9% |
| FOXL2 | 1 | 281 | 585 | 889 | 1.78 | 65.3% |
| GNA11 | 4 | 282 | 586 | 890 | 1.98 | 65.3% |
| GATA1 | 1 | 283 | 587 | 891 | 1.95 | 65.4% |
| KLF4 | 2 | 284 | 588 | 892 | 1.95 | 65.5% |
| FANCB | 1 | 285 | 589 | 893 | 1.86 | 65.7% |
| THRAP3 | 1 | 286 | 590 | 894 | 1.80 | 65.7% |

**FIG. 22M**

| Gene Symbol | Exon ID | Forward Primer (SEQ ID NO.) | Reverse Primer (SEQ ID NO.) | Amplicon Seq (SEQ ID NO.) | Amplif Eff Constant | GC% of Amplicon |
|---|---|---|---|---|---|---|
| RPL5 | 1 | 287 | 591 | 895 | 1.90 | 65.8% |
| JAK3 | 1 | 288 | 592 | 896 | 1.87 | 66.7% |
| NOTCH3 | 2 | 289 | 593 | 897 | 1.91 | 66.7% |
| CDKN2A | 1 | 290 | 594 | 898 | 1.90 | 66.8% |
| H3F3A | 1 | 291 | 595 | 899 | 1.84 | 66.9% |
| HNF1A | 8 | 292 | 596 | 900 | 1.96 | 67.4% |
| NKX2-1 | 1 | 293 | 597 | 901 | 1.88 | 67.5% |
| FOXA1 | 2 | 294 | 598 | 902 | 1.82 | 68.4% |
| CDH1 | 2 | 295 | 599 | 903 | 1.93 | 68.7% |
| CDKN2B | 1 | 296 | 600 | 904 | 1.77 | 68.7% |
| ZFHX3 | 1 | 297 | 601 | 905 | 1.79 | 70.0% |
| HRAS | 5 | 298 | 602 | 906 | 1.79 | 70.0% |
| MYCL1 | 2 | 299 | 603 | 907 | 1.91 | 70.2% |
| PER1 | 1 | 300 | 604 | 908 | 1.80 | 71.1% |
| PER1 | 3 | 301 | 605 | 909 | 1.83 | 60.1% |
| SUZ12 | 1 | 302 | 606 | 910 | 1.79 | 71.6% |
| CDKN2C | 1 | 303 | 607 | 911 | 1.77 | 72.8% |
| NSD1 | 2 | 304 | 608 | 912 | 1.93 | 74.1% |

**FIG. 22N**

|  | Normal Tissue | Tumor Tissue |
|---|---|---|
| Initial mapped reads | 30.7 billion | 25.7 billion |
| Average raw coverage | 240.5 X | 173.9 X |
| Unique read family (URF) | 625 million | 577 million |
| Super read duplexes | 272 million | 231 million |
| Initial mapped reads per super read family | 49.1 | 44.5 |
| Initial mapped reads per super read duplex | 112.9 | 111.3 |
| Super reads per super read duplex | 2.3 | 2.5 |

**FIG. 23**

| Gene Symbol | Transcript Accession | Nucleotide (genomic) | Amino Acid (protein) | Mutant Allele Fraction | Mutant Allele Fraction in 1 to 1000 spiked normal sample | Mutation Type | Consequence | Confirmed | Detected again in 1:1,000 dilution |
|---|---|---|---|---|---|---|---|---|---|
| FOCAD | CCDS34993 | chr9:20865973 A->G | p.K2104E | 29.41% | 0.029% | Substitution | Non-synonymous | YES | YES |
| KRAS | CCDS8703.1 | chr12:25289551C->A | p.G12V | 27.69% | 0.028% | Substitution | Non-synonymous | YES | YES |
| TRIM59 | CCDS3190 | chr3:160156368 ->T | NA | 23.08% | 0.023% | Insertion | Frameshift | YES | NO |
| GOLGA6L2 | ENST00000312015 | chr15:23685010 C->T | p.E1633K | 23.08% | 0.023% | Substitution | Non-synonymous | YES | YES |
| CAMKK2 | CCDS53837 | chr12:121678328 ->T | NA | 22.58% | 0.023% | Insertion | Frameshift | YES | NO |
| TTC39A | ENST00000262676 | chr1:51767913 C-> | NA | 21.74% | 0.022% | Deletion | Frameshift | YES | NO |
| VEGFC | CCDS43285 | chr4:177605084 A->C | p.M1256R | 21.43% | 0.021% | Substitution | Non-synonymous | YES | NO |
| NBPF15 | CCDS932 | chr1:148594474 C->T | p.S1847L | 19.54% | 0.020% | Substitution | Non-synonymous | YES | YES |
| ALG9 | ENST00000428306 | chr11:111742145 C->G | NA | 16.96% | 0.017% | NA | Splicesite donor | YES | YES |
| OAS1 | ENST00000377508 | chr12:113369718 C->A | NA | 16.95% | 0.017% | NA | Splicesite acceptor | YES | NO |
| NFE2L2 | CCDS42782 | chr2:178097188 C->T | p.D526N | 16.46% | 0.016% | Substitution | Non-synonymous | YES | YES |
| OAS1 | ENST00000377508 | chr12:113369716 A->C | NA | 15.87% | 0.016% | NA | Splicesite acceptor | YES | NO |
| E2F8 | ENST00000396159 | chr11:19263371 T-> | NA | 15.63% | 0.016% | NA | Splicesite donor | YES | NO |
| FUZ | ENST00000421740 | chr19:50314458 G->A | p.S650F | 15.15% | 0.015% | Substitution | Non-synonymous | YES | NO |
| FUZ | ENST00000421740 | chr19:50314459 A->G | p.S649P | 15.07% | 0.015% | Substitution | Non-synonymous | YES | NO |
| ADAM21 | CCDS9804 | chr14:70924632 G->A | p.S416N | 14.67% | 0.015% | Substitution | Non-synonymous | YES | YES |
| COL27A1 | CCDS6802 | chr9:116918267 GCG-> | NA | 14.04% | 0.014% | Deletion | Frameshift | YES | NO |

**FIG. 24A**

| USP19 | CCDS43090 | chr3:49155556 A-> | NA | 14.04% | 0.014% | NA | Splicesite acceptor | YES | NO |
|---|---|---|---|---|---|---|---|---|---|
| FAM182A | ENST00000246000 | chr20:26061967 G->T | p.A319S | 13.72% | 0.014% | Substitution | Non-synonymous | YES | YES |
| VLDLR | ENST00000397921 | chr9:2622173 C->G | p.T155S | 12.28% | 0.012% | Substitution | Non-synonymous | YES | NO |
| MMEL1 | CCDS30569 | chr1:2560819 CAG-> | NA | 11.94% | 0.012% | Deletion | Frameshift | YES | NO |
| LSM12 | ENST00000411445 | chr17:42141311 ->A | NA | 11.32% | 0.011% | Insertion | Frameshift | YES | YES |
| MFSD4B | CCDS5090 | chr6:111587361 T-> | NA | 10.94% | 0.011% | Deletion | Frameshift | YES | YES |
| NADK | CCDS30565 | chr1:1684348 ->CCT | NA | 10.85% | 0.011% | Insertion | Frameshift | YES | YES |
| PTPRG | CCDS2895 | chr3:62267290 A->G | p.E3818G | 10.71% | 0.011% | Substitution | Non-synonymous | YES | NO |
| CTNNB1 | CCDS2694.1 | chr3:41241445C->A | NA | 10.53% | 0.011% | NA | Splicesite acceptor | YES | YES |
| ZMYND8 | ENST00000372023 | chr20:45867410 A-> | NA | 10.09% | 0.010% | Deletion | Frameshift | YES | NO |
| TP53 | CCDS11118.1 | chr17:7520075A->C | p.F113V | 9.39% | 0.009% | Substitution | Non-synonymous | YES | YES |
| DCD | ENST00000419084 | chr12:55039821 G->A | p.P211S | 8.46% | 0.008% | Substitution | Non-synonymous | YES | NO |
| KCNB2 | CCDS6209 | chr8:73480397 ->A | NA | 8.34% | 0.008% | Insertion | Frameshift | YES | YES |
| ALG9 | ENST00000428306 | chr11:111742141 G->C | p.P761R | 7.98% | 0.008% | Substitution | Non-synonymous | YES | NO |
| PRRT2 | CCDS10654 | chr16:29825016 C-> | NA | 7.80% | 0.008% | Deletion | Frameshift | NO | YES |
| PYGM | CCDS8079 | chr11:64526120 C->A | p.M300I | 7.55% | 0.008% | Substitution | Non-synonymous | YES | YES |
| TP53 | CCDS11118.1 | chr17:7518898A->G | NA | 6.97% | 0.007% | NA | Splice site donor | YES | YES |
| MYO9B | CCDS46010 | chr19:17294680 ->A | NA | 6.56% | 0.007% | Substitution | Splice site donor | YES | YES |
| TUBBP5 | ENST00000290377 | chr9:141070139 C->T | p.L37F | 6.49% | 0.006% | Substitution | Non-synonymous | YES | NO |
| FOXC1 | CCDS4473 | chr6:1612018 CGG-> | NA | 5.96% | 0.006% | Deletion | Frameshift | NO | NO |

FIG. 24B

| Gene | Transcript ID | Position | Protein | % | % | Type | Consequence | | |
|---|---|---|---|---|---|---|---|---|---|
| GALNT9 | ENST00000424720 | chr12:132824394 C->A | p.M1146I | 5.44% | 0.005% | Substitution | Non-synonymous | YES | YES |
| RP11-830F9.6 | ENST00000378347 | chr16:89017605 A->G | p.D1079G | 5.29% | 0.005% | Substitution | Non-synonymous | YES | YES |
| SRD5A2 | ENST00000233139 | chr2:31805883 C->G | p.K67N | 5.06% | 0.005% | Substitution | Non-synonymous | YES | YES |
| SMG7 | CCDS1355 | chr1:183515267 ->A | NA | 4.98% | 0.005% | Insertion | Frameshift | N/A | N/A |
| LZTS3 | CCDS13049 | chr20:3146451 G->A | p.R1015W | 4.86% | 0.005% | Substitution | Non-synonymous | N/A | N/A |
| TNFSF9 | CCDS12169 | chr19:6531149 GCT-> | NA | 4.74% | 0.005% | Deletion | Frameshift | N/A | N/A |
| NOP9 | CCDS9624 | chr14:24769850 ->GAG | NA | 4.63% | 0.005% | Insertion | Frameshift | N/A | N/A |
| ABCA2 | CCDS43909 | chr9:139917492 G->A | p.A176V | 4.28% | 0.004% | Substitution | Non-synonymous | N/A | N/A |
| SHC1 | CCDS44233 | chr1:154938890 ->C | NA | 4.14% | 0.004% | Insertion | Frameshift | N/A | N/A |
| RNF43 | CCDS11607.1 | chr17:53792509 C->A | p.E318X | 3.99% | 0.004% | Substitution | Nonsense | N/A | N/A |
| RNF43 | CCDS11607.1 | chr17:53790631 C->A | p.S502I | 3.66% | 0.004% | Substitution | Non-synonymous | N/A | N/A |
| SMAD4 | CCDS11950.1 | chr18:46835279 C->A | p.Y195X | 3.45% | 0.003% | Substitution | Nonsense | N/A | N/A |
| IGSF3 | CCDS30814 | chr1:117158745 A->C | p.D378E | 3.42% | 0.003% | Substitution | Non-synonymous | N/A | N/A |
| FOLR3 | ENST00000456237 | chr11:71850159 A->T | p.E449V | 3.27% | 0.003% | Substitution | Non-synonymous | N/A | N/A |
| CTRC | CCDS156 | chr1:15771154 C->T | p.H547Y | 3.20% | 0.003% | Substitution | Non-synonymous | N/A | N/A |
| NRAP | CCDS7579 | chr10:115348730 T->G | NA | 3.08% | 0.003% | Substitution | Non-synonymous | N/A | N/A |
| RNF43 | CCDS11607.1 | chr17:53790810 G->T | p.D442E | 3.07% | 0.003% | Substitution | Non-synonymous | N/A | N/A |
| SH3BP5 | CCDS2625.2 | chr3:15373633 TCC-> | NA | 3.04% | 0.003% | Deletion | Frameshift | N/A | N/A |
| WIZ | ENST00000389282 | chr19:15549933 C->A | p.E1728D | 2.96% | 0.003% | Substitution | Non-synonymous | N/A | N/A |
| HDC | CCDS10134 | chr15:50557790 C->A | p.G31W | 2.85% | 0.003% | Substitution | Non-synonymous | N/A | N/A |

**FIG. 24C**

| | | | | | | | | | |
|---|---|---|---|---|---|---|---|---|---|
| UBQLNL | CCDS31385 | chr11:5536683 C->T | p.R989Q | 2.82% | 0.003% | Substitution | Non-synonymous | N/A | N/A |
| CDKN2A | CCDS6511.1 | chr9:21961019C->T | p.A169T | 2.69% | 0.003% | Substitution | Non-synonymous | N/A | N/A |
| AHDC1 | CCDS30652 | chr1:27877505 G-> | NA | 2.40% | 0.002% | Deletion | Frameshift | N/A | N/A |
| PTPRS | CCDS45930 | chr19:5208036 G->A | p.T5675M | 2.34% | 0.002% | Substitution | Non-synonymous | N/A | N/A |
| TMEM63A | CCDS31042.1 | chr1:226034840 CTG-> | NA | 2.13% | 0.002% | Deletion | Frameshift | N/A | N/A |
| NOTCH4 | CCDS34420.1 | chr6:32163554 ->C | NA | 2.02% | 0.002% | Insertion | Frameshift | N/A | N/A |
| AZGP1 | CCDS5680.1 | chr7:99573573 T->C | p.Q71R | 1.94% | 0.002% | Substitution | Non-synonymous | N/A | N/A |
| TBC1D29 | CCDS32606.1 | chr17:28890361 G->A | p.R371Q | 1.88% | 0.002% | Substitution | Non-synonymous | N/A | N/A |
| GIGYF2 | CCDS46542.1 | chr2:233697764 GCA-> | NA | 1.84% | 0.002% | Deletion | Frameshift | N/A | N/A |
| KIF7 | CCDS32325.2 | chr15:90171904 G-> | NA | 1.81% | 0.002% | Deletion | Frameshift | N/A | N/A |
| TP53I13 | CCDS42289.1 | chr17:27899242 C->T | p.S596F | 1.79% | 0.002% | Substitution | Non-synonymous | N/A | N/A |
| PCNX3 | CCDS44650.1 | chr11:65385491 C->A | p.L658M | 1.72% | 0.002% | Substitution | Non-synonymous | N/A | N/A |
| EIF3J | CCDS10111.1 | chr15:44829395 GGC-> | NA | 1.71% | 0.002% | Deletion | Frameshift | N/A | N/A |
| SFSWAP | CCDS9273.1 | chr12:132281734 AGA-> | NA | 1.60% | 0.002% | Deletion | Frameshift | N/A | N/A |
| TMC4 | CCDS12882.1 | chr19:54675747 TCC-> | NA | 1.55% | 0.002% | Deletion | Frameshift | N/A | N/A |
| IQSEC2 | CCDS48130.1 | chrX:53264131 TGG-> | NA | 1.43% | 0.001% | Deletion | Frameshift | N/A | N/A |
| C6orf132 | CCDS47428.1 | chr6:42072710 CTC-> | NA | 1.36% | 0.001% | Deletion | Frameshift | N/A | N/A |
| ARX | CCDS14215.1 | chrX:25025356 GGC-> | NA | 1.34% | 0.001% | Deletion | Frameshift | N/A | N/A |
| NHS | ENST00000380057 | chrX:17705855 C->T | p.L22F | 1.24% | 0.001% | Substitution | Non-synonymous | N/A | N/A |
| RAB40C | CCDS10413.1 | chr16:677580 ->C | NA | 1.22% | 0.001% | Insertion | Frameshift | N/A | N/A |

**FIG. 24D**

| | | | | | | | | | |
|---|---|---|---|---|---|---|---|---|---|---|
| C16orf91 | CCDS32360.1 | chr16:1476278 C->A | p.Q345H | 1.06% | 0.001% | Substitution | Non-synonymous | N/A | N/A |
| EME2 | CCDS32364.1 | chr16:1824298 TGC-> | NA | 1.04% | 0.001% | Deletion | Frameshift | N/A | N/A |
| ATP13A2 | CCDS175.1 | chr1:17318887 G-> | NA | 0.94% | 0.001% | Deletion | Frameshift | N/A | N/A |
| ZIC3 | CCDS14663.1 | chrX:136648985 CGC-> | NA | 0.74% | 0.001% | Deletion | Frameshift | N/A | N/A |
| NAA30 | CCDS32088.1 | chr14:57858199 AGG-> | NA | 0.61% | 0.001% | Deletion | Frameshift | N/A | N/A |
| NDUFS3 | CCDS7941.1 | chr11:47600646 GGC-> | NA | 0.58% | 0.001% | Deletion | Frameshift | N/A | N/A |
| CCKBR | CCDS7761.1 | chr11:6292451 ->T | NA | 0.49% | 0.000% | Insertion | Frameshift | N/A | N/A |
| RUSC2 | CCDS35008.1 | chr9:35560102 GCT-> | NA | 0.46% | 0.000% | Deletion | Frameshift | N/A | N/A |
| NOS2 | CCDS11223.1 | chr17:26099407 G->A | p.A1631V | 0.42% | 0.000% | Substitution | Non-synonymous | N/A | N/A |
| WASL | CCDS34743.1 | chr7:123388755 GCG-> | NA | 0.38% | 0.000% | Deletion | Frameshift | N/A | N/A |
| SNRNP35 | CCDS45005.1 | chr12:123950763 GA-> | NA | 0.31% | 0.000% | Deletion | Frameshift | N/A | N/A |
| NBPF14 | CCDS30836.1 | chr1:148009468 C->G | p.E1839D | 0.29% | 0.000% | Substitution | Non-synonymous | N/A | N/A |
| FAM104B | CCDS35305.1 | chrX:55172647 G->A | p.P221L | 0.27% | 0.000% | Substitution | Non-synonymous | N/A | N/A |
| TFEB | CCDS4858.1 | chr6:41658830 TGC-> | NA | 0.25% | 0.000% | Deletion | Frameshift | N/A | N/A |
| SEPT9 | CCDS45790.1 | chr17:75478417 G->A | p.G913R | 0.22% | 0.000% | Substitution | Non-synonymous | N/A | N/A |
| ROR2 | CCDS6691.1 | chr9:94486026 TCC-> | NA | 0.15% | 0.000% | Deletion | Frameshift | N/A | N/A |
| PKD2 | CCDS3627.1 | chr4:88929174 GAG-> | NA | 0.12% | 0.000% | Deletion | Frameshift | N/A | N/A |
| GLIS2 | CCDS10511.1 | chr16:4384871 G-> | NA | 0.08% | 0.000% | Deletion | Frameshift | N/A | N/A |
| HECTD4 | CCDS44978.1 | chr12:112622808 T-> | NA | 0.02% | 0.000% | Deletion | Frameshift | N/A | N/A |
| MUC4 | NM_018406 | chr3:195508284 C->A | p.Q10167H | 0.01% | 0.000% | Substitution | Non-synonymous | N/A | N/A |

**FIG. 24E**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CIBULSKIS, K. et al.** Sensitive detection of somatic point mutations in impure and heterogeneous cancer samples. *Nat Biotechnol,* 2013, vol. 31 (3), 213-219
- **KERICK, M. et al.** Targeted high throughput sequencing in clinical cancer settings: formaldehyde fixed-paraffin embedded (FFPE) tumor tissues, input amount and tumor heterogeneity. *BMC Med Genomics,* 2011, vol. 4, 68

- **MCKENNA, A. et al.** The Genome Analysis Toolkit: a MapReduce framework for analyzing next-generation DNA sequencing data. *Genome Res,* 2010, vol. 20 (9), 1297-1303
- **TSIATIS, A. C. et al.** Comparison of Sanger sequencing, pyrosequencing, and melting curve analysis for the detection of KRAS mutations: diagnostic and clinical implications. *J Mol Diagn,* 2010, vol. 12 (4), 425-432
- **UNTERGASSER, A. et al.** Primer3--new capabilities and interfaces. *Nucleic Acids Res,* 2012, vol. 40 (15), e115